# EUROPEAN PATENT APPLICATION

(11) **EP 4 032 971 A1**
(43) Date of publication of application: **27.07.2022**
(21) Application number: 20865108.3
(22) Date of filing: 18.09.2020
(51) Int. Cl.: C12N 5/077, C12N 5/10, C12N 15/09, C12Q 1/02, C12Q 1/686

(54) **LBM, CPC, OPC, PRODUCTION AND QUALITY CONTROL METHODS THEREFOR, KIT, GRAFT MATERIAL, AND DISEASE MODEL**

(30) Priority: 18.09.2019 JP 2019169278; 31.03.2020 JP 2020062441
(71) Applicant: National University Corporation Okayama University, Kita-ku, Okayama-shi, Okayama 700-8530 (JP)
(72) Inventor: TAKARADA, Takeshi, Okayama-shi, Okayama 700-8530 (JP); YAMADA, Daisuke, Okayama-shi, Okayama 700-8530 (JP); TAKAO, Tomoka, Okayama-shi, Okayama 700-8530 (JP); TOGUCHIDA, Junya, Kyoto-shi, Kyoto 606-8501 (JP); YOSHITOMI, Hiroyuki, Kyoto-shi, Kyoto 606-8501 (JP)
(74) Representative: Müller-Boré & Partner Patentanwälte PartG mbB
(86) International application number: PCT/JP2020/035517
(87) International publication number: WO 2021/054449

(57) **Abstract**

The present invention provides a limb bud mesenchymal cell population, which is derived from mammalian lateral plate mesoderm cells, and is PRRX1 protein-positive.

SEQUENCE LISTING

<110>  NATIONAL UNIVERSITY CORPORATION OKAYAMA UNIVERSITY
       Kyoto University

<120>  LBM\201ACPC\201AOPC\201APRODUCTION AND QUALITY CONTROL METHOD
THEREOF, KIT, GRAFT MATERIAL AND DISEASE MODEL

<130>  P20-181WO

<150>  JP 2019-169278
<151>  2019-09-18

<150>  JP 2020-062441
<151>  2020-03-31

<160>  4

<170>  PatentIn version 3.5

<210>  1
<211>  28
<212>  RNA
<213>  Artificial Sequence

<220>
<223>  RUNX2 forward primer

<400>  1
tcaacgatct gagatttgtg gg                                              28

<210>  2
<211>  28
<212>  RNA
<213>  Artificial Sequence

<220>
<223>  RUNX2 reverse primer

<400>  2
ggggaggatt tgtgaagacg g                                               28

<210>  3
<211>  28
<212>  RNA
<213>  Artificial Sequence

<220>
<223>  ACTB forward primer

<400>  3
agaaaatctg gcaccacacc                                                 28

<210>  4
<211>  28
<212>  RNA
<213>  Artificial Sequence

<220>
<223>  ACTB reverse primer

<400> 4
agaggcgtac agggatagca 28

## Description

### Technical Field

### [Cross-reference to Related Applications]

This application claims priority from Japanese Patent Application No. 2019-169278, filed on September 18, 2019, and Japanese Patent Application No. 2020-62441, filed on March 31, 2020, the entire disclosures of which are incorporated herein by reference.

The present invention relates to a limb bud mesenchymal cell population, a chondrocyte progenitor cell population, an osteoblast progenitor cell population, a preparation method and quality control method therefor, a transplantation material, and a disease model.

Herein, the term "CPC" is used to mean a chondrocyte progenitor cell or a chondrocyte progenitor cell population, and the term "LBM" is used to mean a limb bud mesenchymal cell or a limb bud mesenchymal cell population. The term "OPC" is used to mean an osteoblast progenitor cell or an osteoblast progenitor cell population. In addition, "PRRX1-positive" is sometimes described as PRRX1^{high}, and "PRRX1-negative" is sometimes described as PRRX1^{low}.

### Background Art

Mesenchymal stem cells, which are a type of somatic stem cells, are cells having an ability to differentiate into cells such as osteoblasts and chondrocytes. Their application to regenerative medicine is being considered, but involves difficulties of low differentiation induction efficiency and limited self-growth ability. In addition, a related-art differentiation induction method involving using pluripotent stem cells has a focus on direct induction into tissue cells of interest, but low induction efficiency and low quality thereof are perceived as problems (Non-patent Literatures 1 and 2).

In addition, when quality of osteoblasts or chondrocytes is poor, a bone tissue or cartilage tissue to be obtained is degraded in quality, and hence cannot be transplanted into a living body.

### Citation List

### Non-patent Literature

NPL 1: Stem Cell Reports. 2015 Mar 10; 4(3): 404-18.
NPL 2: Nat Biotechnol. 2015 Jun; 33(6): 638-45.

### Summary of Invention

### Technical Problem

An object of the present invention is to provide a limb bud mesenchymal cell population, osteoblast progenitor cell population, or chondrocyte progenitor cell population capable of producing a bone tissue or cartilage tissue of interest with high efficiency and high purity, a preparation method therefor, and a quality control method therefor.

Another object of the present invention is to provide a kit for obtaining, from mammalian pluripotent stem cells, the limb bud mesenchymal cell population, the osteoblast progenitor cell population, the chondrocyte progenitor cell population, or chondrocytes or a cartilage tissue that are or is inducible therefrom.

Still another object of the present invention is to provide a transplantation material for treating a bone- or cartilage-related disease.

Yet still another object of the present invention is to provide a bone- or cartilage-related disease model.

### Solution to Problem

The present invention provides a limb bud mesenchymal cell population, a chondrocyte progenitor cell population, an osteoblast progenitor cell population, a preparation method and quality control method therefor, a kit, a transplantation material, and a disease model described below.
Item 1. A limb bud mesenchymal cell population, which is derived from mammalian lateral plate mesoderm cells, and is PRRX1 protein-positive.
Item 2. The limb bud mesenchymal cell population according to Item 1, wherein the limb bud mesenchymal cell population satisfies at least one kind of condition selected from the group consisting of CD44 positivity, CD140B positivity, and CD49f negativity.
Item 3. A method of preparing the limb bud mesenchymal cell population of Item 1 or 2, including the steps of:
   inducing pluripotent stem cells to differentiate into lateral plate mesoderm cells; and
   culturing the lateral plate mesoderm cells obtained in the differentiation induction step under Wnt signal-activating and non-FGF signal-activating conditions.
Item 4. A method of preparing an osteoblast progenitor cell population, including a step of culturing the limb bud mesenchymal cell population of Item 1 or 2 under a Wnt signal activator-free environment.
Item 5. A method of preparing a mammalian chondrocyte progenitor cell population, including a step of culturing the limb bud mesenchymal cell population of Item 1 or 2 under a Wnt signal-activating environment.
Item 6. The method of preparing a mammalian chondrocyte progenitor cell population according to Item 5, wherein the method includes a step of culturing the limb bud mesenchymal cell population of Item 1 or 2 under a Wnt signal-activating environment and under an FGF signal-activating condition.
Item 7. A quality control method for a mammalian limb bud mesenchymal cell population, including a step of determining whether mammalian limb bud mesenchymal cells satisfy at least one kind of condition selected from the group consisting of CD44 positivity, CD140B positivity, and CD49f negativity.
Item 8. A quality control method for a mammalian chondrocyte progenitor cell population, including a step of determining whether mammalian chondrocyte progenitor cells satisfy at least one kind of condition selected from the group consisting of CD90 positivity and CD140B positivity.
Item 9. A mammalian chondrocyte progenitor cell population, which satisfies at least one kind of condition selected from the group consisting of CD90 positivity and CD140B positivity.
Item 10. The mammalian chondrocyte progenitor cell population according to Item 9, wherein the mammalian chondrocyte progenitor cell population is cryopreserved.
Item 11. A mammalian osteoblast progenitor cell population, which has a RUNX2-positive rate of 95% or more.
Item 12. The mammalian osteoblast progenitor cell population according to Item 11, wherein the mammalian osteoblast progenitor cell population is cryopreserved.
Item 13. A kit for inducing mammalian pluripotent stem cells into limb bud mesenchymal cells (LBMs), including the following items (i) to (iii):
   (i) a medium for inducing pluripotent stem cells into primitive streak cells;
   (ii) a medium for inducing primitive streak cells into lateral plate mesoderm cells; and
   (iii) a medium for inducing lateral plate mesoderm cells into limb bud mesenchymal cells.
Item 14. A kit for inducing mammalian pluripotent stem cells into chondrocyte progenitor cells, including the following items (i) to (iv):
   (i) a medium for inducing pluripotent stem cells into primitive streak cells;
   (ii) a medium for inducing primitive streak cells into lateral plate mesoderm cells;
   (iii) a medium for inducing lateral plate mesoderm cells into limb bud mesenchymal cells; and
   (iv) a medium for inducing limb bud mesenchymal cells into chondrocyte progenitor cells.
Item 15. A kit for inducing mammalian pluripotent stem cells into chondrocytes, including the following items (i) to (v) :
   (i) a medium for inducing pluripotent stem cells into primitive streak cells;
   (ii) a medium for inducing primitive streak cells into lateral plate mesoderm cells;
   (iii) a medium for inducing lateral plate mesoderm cells into limb bud mesenchymal cells;
   (iv) a medium for inducing limb bud mesenchymal cells into chondrocyte progenitor cells; and
   (v) a medium for inducing chondrocyte progenitor cells into chondrocytes.
Item 16. A kit for inducing mammalian pluripotent stem cells into RUNX2-positive osteoblast progenitor cells, including the following items (i) to (iv) :
   (i) a medium for inducing pluripotent stem cells into primitive streak cells;
   (ii) a medium for inducing primitive streak cells into lateral plate mesoderm cells;
   (iii) a medium for inducing lateral plate mesoderm cells into limb bud mesenchymal cells; and
   (iv) a medium for inducing limb bud mesenchymal cells into RUNX2-positive osteoblast progenitor cells.
Item 17. A transplantation material, including:
   the mammalian chondrocyte progenitor cell population of Item 9 or 10; or
   chondrocytes or a cartilage tissue obtained by differentiation induction from the chondrocyte progenitor cell population.
Item 18. A transplantation material, including:
   the mammalian osteoblast progenitor cell population of Item 11 or 12; or
   osteoblasts or a bone tissue obtained by differentiation induction from the osteoblast progenitor cell population.
Item 19. A cartilage-related disease model, including:
   a chondrocyte progenitor cell population, which is induced from cartilage-related disease patient-derived iPS cells, and satisfies at least one kind of condition selected from the group consisting of CD90 positivity and CD140B positivity; or
   chondrocytes induced from the chondrocyte progenitor cell population.
Item 20. A bone-related disease model, including:
   an osteoblast progenitor cell population, which is induced from bone-related disease patient-derived iPS cells, and has a RUNX2-positive rate of 95% or more; or
   osteoblasts induced from the osteoblast progenitor cell population.

### Advantageous Effects of Invention

According to the present invention, a transplantation material for regenerating a high-quality cartilage tissue and cartilage can be obtained through quality control of the limb bud mesenchymal cell population or the chondrocyte progenitor cell population.

The limb bud mesenchymal cell population obtained in the present invention and the osteoblast progenitor cell population or chondrocyte progenitor cell population inducible therefrom, or the chondrocytes or cartilage tissue induced from the chondrocyte progenitor cell population can be used as a transplantation material for treating a bone- or cartilage-related disease. It has been recognized by the inventors that the transplantation material of the present invention is a safe transplantation material that does not cause a benign or malignant tumor.

A high-quality bone tissue or cartilage tissue can be obtained through use of the osteoblast progenitor cell population or the chondrocyte progenitor cell population of the present invention.

Through use of the kit of the present invention, a limb bud mesenchymal cell population, an osteoblast progenitor cell population, a chondrocyte progenitor cell population, or chondrocytes or a cartilage tissue that are or is inducible therefrom can be easily obtained from pluripotent stem cells, such as iPS cells.

When the limb bud mesenchymal cell population, the chondrocyte progenitor cell population, or the osteoblast progenitor cell population of the present invention is induced from iPS cells of a bone- or cartilage-related disease patient, the cell population is useful for the generation of a bone disease model or a cartilage disease model, and further, for the generation of a bone-related disease or cartilage-related disease model.

Through use of the disease model of the present invention, the pathology of a bone- or cartilage-related disease can be investigated, and the development of a novel therapeutic drug can be supported.

### Brief Description of Drawings

Fig. 1 is an illustration of an overview of a differentiation induction protocol of the present invention.
Fig. 2 is a schematic illustration of a targeting vector for generating a PRRX1 reporter.
Fig. 3 is an illustration of a protocol for differentiation induction into PRRX1-positive cells via a lateral plate mesoderm lineage.
Fig. 4 shows the results of immunostaining for HAND1 (lateral plate mesoderm marker).
Fig. 5 shows the results of immunostaining for CDX2 (paraxial mesoderm marker).
Fig. 6 is an illustration of a protocol for differentiation induction into PRRX1-positive cells via lateral plate mesoderm lineage.
Fig. 7 shows activators and/or suppressors for various signals used (16 combinations).
Fig. 8 shows fluorescence images of tdTomato.
Fig. 9 shows measurement results of quantitative RT-PCR (qPCR) for PRRX1.
Fig. 10 shows measurement results of quantitative RT-PCR (qPCR) for HAND1 (lateral plate mesoderm marker).
Fig. 11 shows measurement results of quantitative RT-PCR (qPCR) for ISL-1 (lateral plate mesoderm marker).
Fig. 12 shows measurement results of quantitative RT-PCR (qPCR) for FOXF1 (lateral plate mesoderm marker).
Fig. 13 is an illustration of a preferred protocol for inducing PRRX1-positive cells from lateral plate mesoderm cells (day 2).
Fig. 14 shows the results of flow cytometry.
Fig. 15 shows the results of immunostaining using a PRRX1 antibody.
Fig. 16 shows an expression profile (1) of CD antigens and the like.
Fig. 17 shows an expression profile (2) of CD antigens and the like.
Fig. 18 shows an expression profile (3) of CD antigens and the like.
Fig. 19 is an illustration of a differentiation induction protocol.
Fig. 20 shows phase-contrast micrographs at different time points.
Fig. 21 shows PRRX1 mRNA expression amounts at different time points.
Fig. 22 shows the mRNA expression amounts of lateral plate mesoderm markers (HAND1, ISL1, and FOXF1) at different time points.
Fig. 23 shows the results of immunostaining using a PRRX1 antibody.
Fig. 24 shows phase-contrast micrographs at different time points.
Fig. 25 shows the mRNA expression amounts of PRRX1 and mesoderm markers (HAND1, ISL1, and FOXF1) at different time points.
Fig. 26 shows the results of immunostaining using a PRRX1 antibody.
Fig. 27 is an illustration of a protocol for differentiation induction into PRRX1-positive cells via a paraxial mesoderm lineage.
Fig. 28 shows the results of immunostaining for HAND1 (lateral plate mesoderm marker).
Fig. 29 shows the results of immunostaining for CDX2 (paraxial mesoderm marker).
Fig. 30 shows activators and/or suppressors for various signals used (6 combinations).
Fig. 31 shows fluorescence images of tdTomato.
Fig. 32 shows the results of flow cytometry.
Fig. 33 shows measurement results of quantitative RT-PCR (qPCR) for PRRX1, a paraxial mesoderm marker (CDX2), and somite markers (MEOX1 and PARAXIS).
Fig. 34 shows activators and/or suppressors for various signals used (16 combinations).
Fig. 35 shows measurement results of quantitative RT-PCR (qPCR) for RUNX2.
Fig. 36 is an illustration of a differentiation induction protocol.
Fig. 37 shows measurement results of quantitative RT-PCR (qPCR).
Fig. 38 shows measurement results of quantitative RT-PCR (qPCR) for RUNX2.
Fig. 39 shows the results of immunostaining for RUNX2.
Fig. 40 shows the results of flow cytometry.
Fig. 41 shows the results of staining with alizarin red.
Fig. 42 is an illustration of a differentiation induction protocol.
Fig. 43 shows phase-contrast images under different conditions.
Fig. 44 shows the results of an investigation of coating. (A) Seeding into a culture dish coated with each coating agent with use of Stem Medium 3. (B) Coating with iMatrix-511, which is the same coating agent as that used at the time of differentiation induction and is of a clinical grade, is selected.
Fig. 45 shows phase-contrast micrographs at different passage numbers during passage culture. PN: passage number.
Fig. 46 shows flow cytometry analysis results and a growth curve. PN: passage number.
Fig. 47 shows a comparison of the growth abilities of hCPCs induced from various pluripotent stem cells.
Figs. 48: the development of a quality control method for LBM cells. Figs. 48 (a): the chromosomal karyotype of a 414C2 iPSC line, Figs. 48 (b): the karyotype of a human CPC (PN3) established from the 414C2 iPSC line.
Figs. 49 show phase-contrast images of cells before and after liquid nitrogen preservation. Fig. 49(a): before liquid nitrogen preservation, Fig. 49(b): after liquid nitrogen preservation.
Fig. 50 shows RUNX2 expressions of cells (derived from hCPCs and derived from LBMs) obtained by inducing hCPCs and human LBMs (day 4) in a RUNX2-positive osteoblast progenitor cell differentiation-inducing medium. It was revealed that an increase in expression of RUNX2 was not found in the cells derived from hCPCs.
Figs. 51: the expressions of chondrocyte markers (SOX5, SOX6, SOX9, COL2A1, and ACAN) for respective hCPCs. Fig. 51(a): SOX9 expressions of hCPCs derived from various iPS cells, Fig. 51(b): a protocol for treating hCPCs with Step 1 medium and Step 2 medium, Fig. 51(c): the results of assay of cells with cytokines (complete) or without cytokines (w/o cytokines), Fig. 51(d): the results of mRNA expression of the chondrocyte markers (SOX5, SOX6, SOX9, COL2A1, and ACAN).
Figs. 52: the generation and staining of a hyaline cartilage-like tissue body. Fig. 52(a): a protocol for obtaining hyaline cartilage from hCPCs, Fig. 52(b): hyaline cartilage tissue bodies on the 42nd day of the treatment of step 3, Fig. 52 (c) : the results of HE staining, Alcian Blue staining, Safranin O staining, and immunostaining of chondrocyte markers (SOX9 and COL2), a hypertrophic chondrocyte marker (COLX), and a fibrocartilage marker (COL1).
Fig. 53 shows the results of staining of cartilage tissues induced from human LBMs (day 4) and hCPCs with Safranin O. It was revealed that the hCPCs had a higher cartilage tissue-forming ability than the human LBMs.
Figs. 54: subcutaneous transplantation of a hyaline cartilage-like tissue body into a NOD-SCID mouse. Fig. 54(a): a protocol for inducing a cartilage tissue body from hCPCs, followed by subcutaneous transplantation, Fig. 54(b): the generation of sections of a hyaline cartilage-like tissue body removed from under the skin, Fig. 54(c): SafO staining results of hyaline cartilage-like tissue bodies derived from respective iPS cells (414C2 and Reporter) or ES cells (SEES6).
Figs. 55: an experiment of transplantation of a human-derived cartilage tissue mass into a cartilage defective site of a SCID rat. Fig. 55(a): the generation of a cartilage tissue mass and transplantation thereof into a cartilage defective site, Fig. 55(b): the recognition of engraftment of the transplanted tissue after 2 weeks from transplantation.
Figs. 56: subcutaneous transplantation of a hyaline cartilage-like tissue body into a NOD-SCID mouse. Fig. 56(a): a protocol for subrenal capsule transplantation of hCPCs into a mouse, Fig. 56(b): the results of immunostaining of a hyaline cartilage tissue body obtained in the 8th week after the transplantation, Fig. 56(c): a photograph taken immediately after the subrenal capsule transplantation.
Figs. 57: the generation of a cartilage plate. Fig. 57(a): a protocol for obtaining a cartilage plate from hCPCs, Fig. 57(b): photographs taken immediately after loading of hCPC spheroids into a mold, after 15 days of culture, and after 30 days of culture.
Figs. 58 show the results of Safranin O staining of a cartilage plate tissue formed in the mold. Fig. 58(a): the results of Safranin O staining of the cartilage plate tissue formed in the mold. Fig. 58(b): subcutaneous transplantation of the cartilage plate tissue formed in the mold into a NOD-SCID mouse, and the result of Safranin O staining after 3 weeks from the transplantation.
Fig. 59: An overview of a quality control technology for chondrocyte progenitor cells (CPCs). A method for the quality control of CPCs (=chondrocyte/cartilage tissue-forming ability) was developed. In order to stably supply chondrocytes/cartilage tissue from CPCs, it is important to subject hCPCs to expansion culture in a state of satisfying at least one kind of condition selected from the group consisting of CD90 positivity and CD140B positivity.

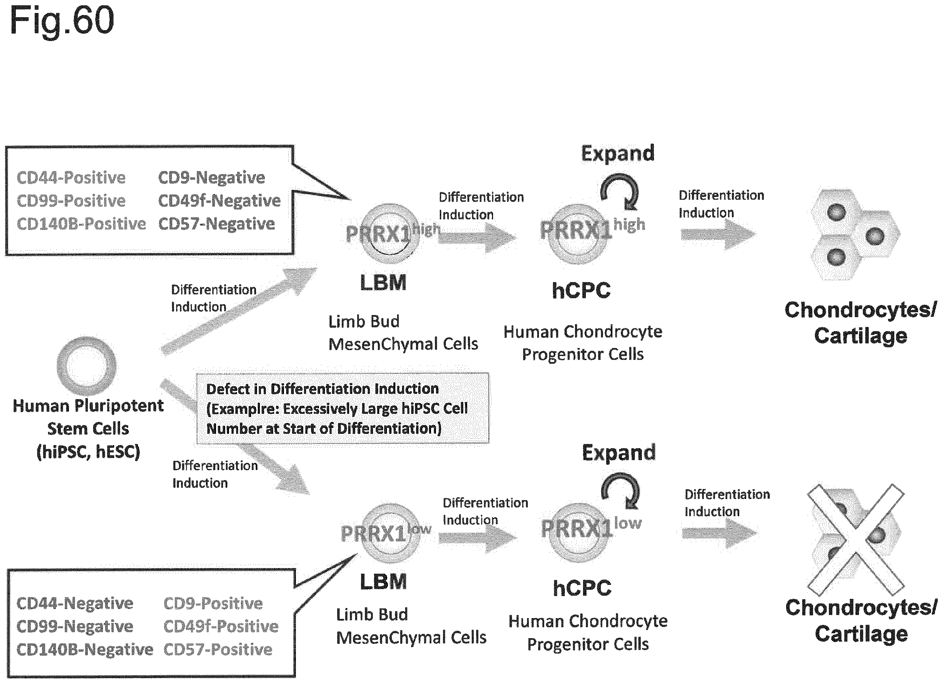

Fig. 60 is an illustration of an overview of a quality control technology for limb bud mesenchymal cells (LBMs). A method for the quality control of LBMs (=PRRX1-positive CPC-forming ability) was developed. In order to supply CPCs having a high cartilage tissue-forming ability from LBMs, it is important to induce CPCs from "LBMs in a state of satisfying at least one kind of condition selected from the group consisting of CD44 positivity, CD99 positivity, CD140B positivity, CD9 negativity, CD49f negativity, and CD57 negativity," and in particular, it is important to induce CPCs from "LBMs in a state of satisfying at least one kind of condition selected from the group consisting of CD44 positivity, CD140B positivity, and CD49f negativity."

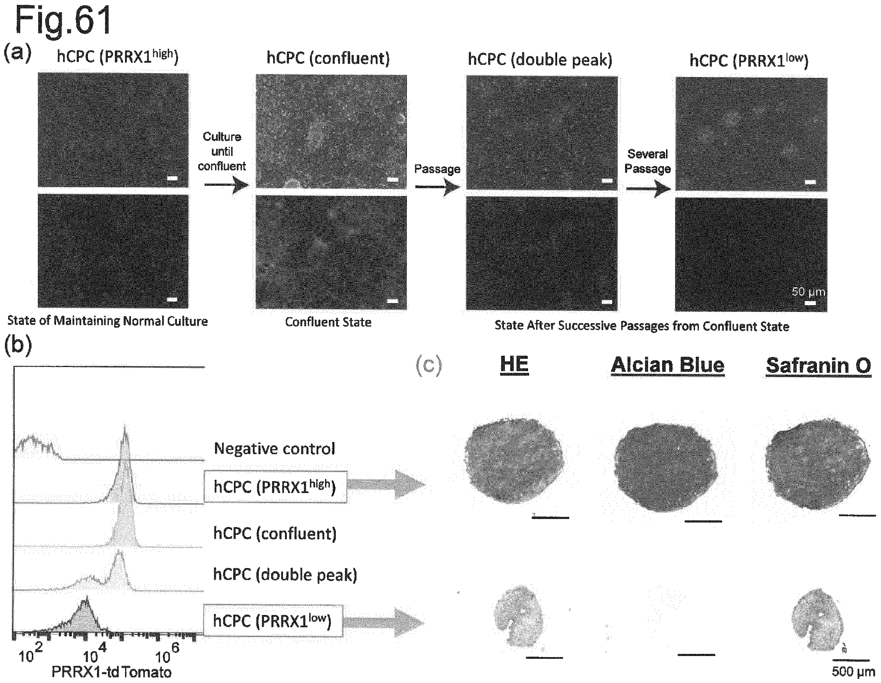

Figs. 61(a) and Fig. 61(b): PRRX1 expression patterns of PRRX1-positive human chondrocyte progenitor cells (hCPCs). Fig. 61(c): staining results of cartilage tissues produced therefrom. hCPCs in each PRRX1-expressing state were collected from their culture dish, and were subjected to differentiation induction. Tissues formed after cartilage differentiation induction were fixed and subjected to HE staining, Alcian blue staining, and Safranin O staining, and as a result, it was found that hCPCs maintained in a state of having a high expression amount of PRRX1 had a high chondrocyte/cartilage tissue-forming ability, but hCPCs maintained in a state of having a low expression amount of PRRX1 had a low chondrocyte/cartilage tissue-forming ability. That is, it is found that there is a positive correlation between the expression amount of PRRX1 and the final cartilage tissue-forming ability thereof.
Fig. 62: a difference in stainability with Alcian blue at the time of plane cartilage differentiation induction due to a difference in expression amount of PRRX1.
   hCPCs having a high expression amount of PRRX1 (PRRX1-positive) or hCPCs having a low expression amount thereof (PRRX1-negative) were seeded into a culture dish, and were subjected to differentiation induction in accordance with the illustrated procedure. After cartilage differentiation induction, the cells were fixed and subjected to Alcian blue staining, and as a result, only the hCPCs having a high expression amount of PRRX1 formed an Alcian blue-positive nodule.

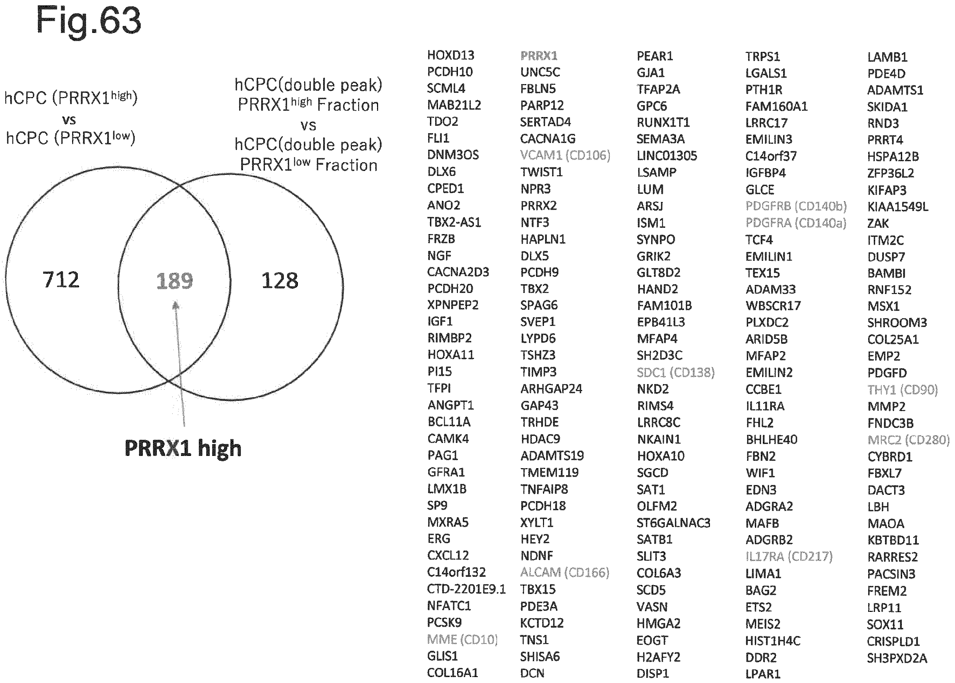

Fig. 63: RNAseq was performed in each of the following two kinds of combinations, and genes upregulated in a sample having a high PRRX1 expression were extracted. 901 genes were extracted from the combination (1), and 317 genes were extracted from the combination (2).
   189 genes common to the two combinations were able to be identified (=good-quality cell marker molecules).
   Combination (1): hCPCs (PRRX1-positive peak) vs hCPCs (PRRX1-negative peak),
   Combination (2): PRRX1-positive fraction vs PRRX1-negative fraction in hCPCs (double peak).

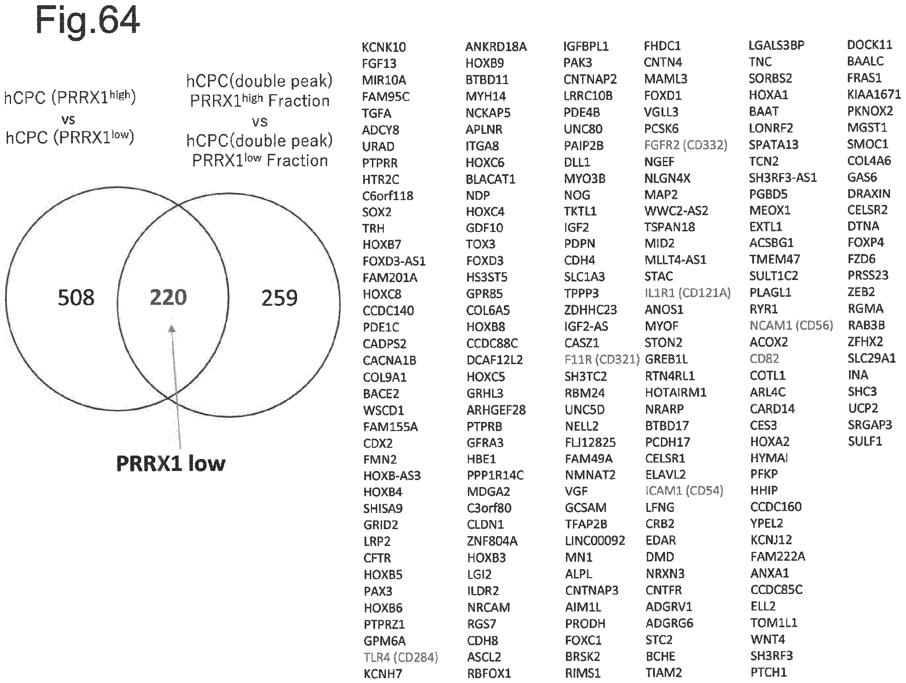

Fig. 64: RNAseq was performed in each of the following two kinds of combinations, and genes upregulated in a sample having a low PRRX1 expression were extracted. 728 genes were extracted from the combination (3), and 479 genes were extracted from the combination (4).
   220 genes common to the two combinations were able to be identified (=poor-quality cell marker molecules)
   Combination (3): hCPCs (PRRX1-positive peak) vs hCPCs (PRRX1-negative peak),
   Combination (4): PRRX1-positive fraction vs PRRX1-negative fraction in hCPCs (double peak).

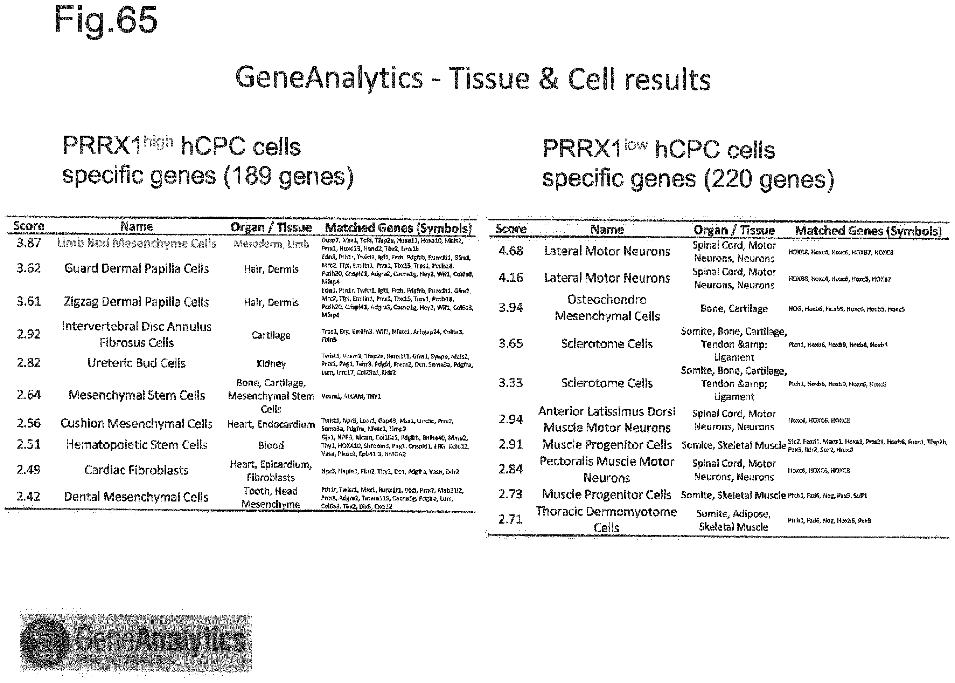

Fig. 65: genes specific to PRRX1-positive hCPCs The good-quality marker molecules (189 genes) and the poor-quality marker molecules (220 genes) were analyzed with Gene analytics software, and as a result, the good-quality marker molecules (189 genes) were found to be genes defining hCPCs.

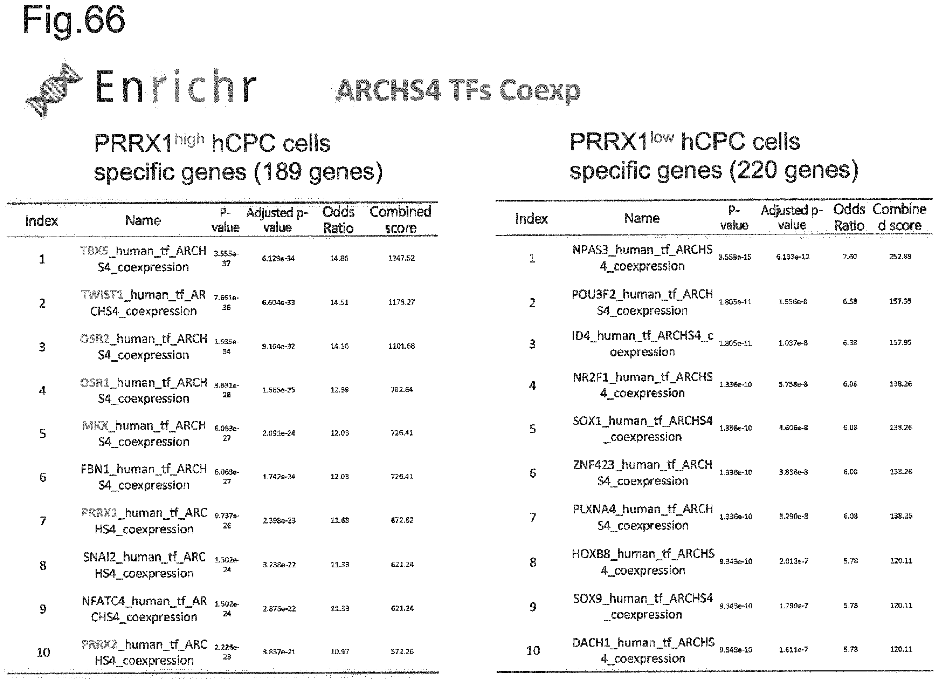

Fig. 66: The good-quality marker molecules (189 genes) and the poor-quality marker molecules (220 genes) were analyzed with Enricher software, and as a result, the good-quality marker molecules (189 genes) were found to be similar to the expression pattern of transcriptional regulators characterizing hCPCs.

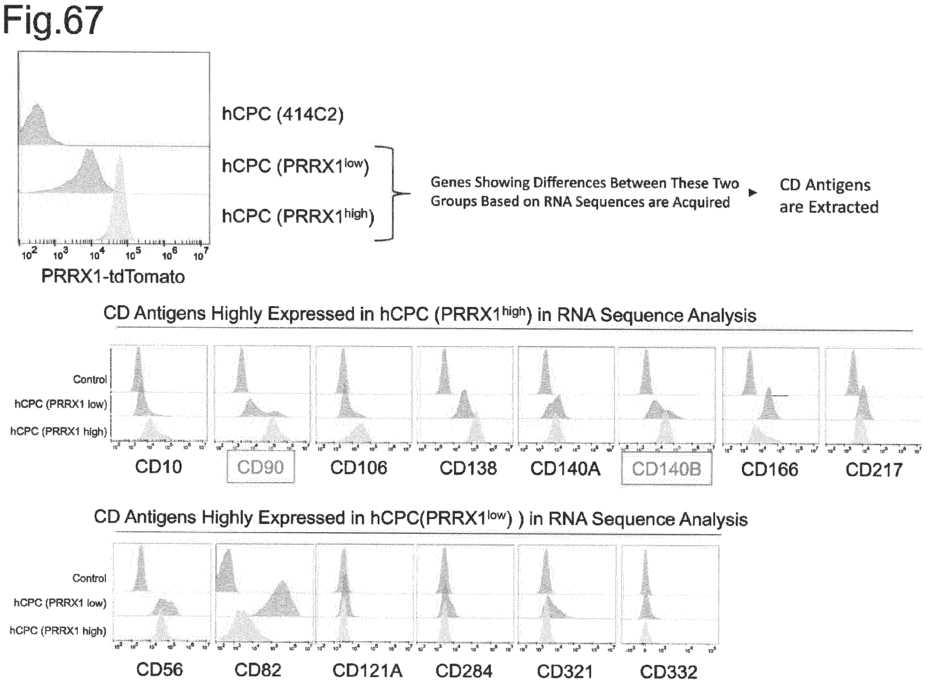

Fig. 67: candidate genes for selecting PRRX1-negative or -positive hCPCs The results of flow cytometry of each surface antigen identified by RNAseq as having a correlation with the expression amount of PRRX1. hCPCs having a low expression amount of PRRX1 (PRRX1-negative) or hCPCs having a high expression amount of PRRX1 (PRRX1-positive) were stained with antibodies against the respective surface antigens, and expression amounts were compared by flow cytometry. As a result, it was found that CD90 and CD140B were highly expressed in the PRRX1-positive cells.

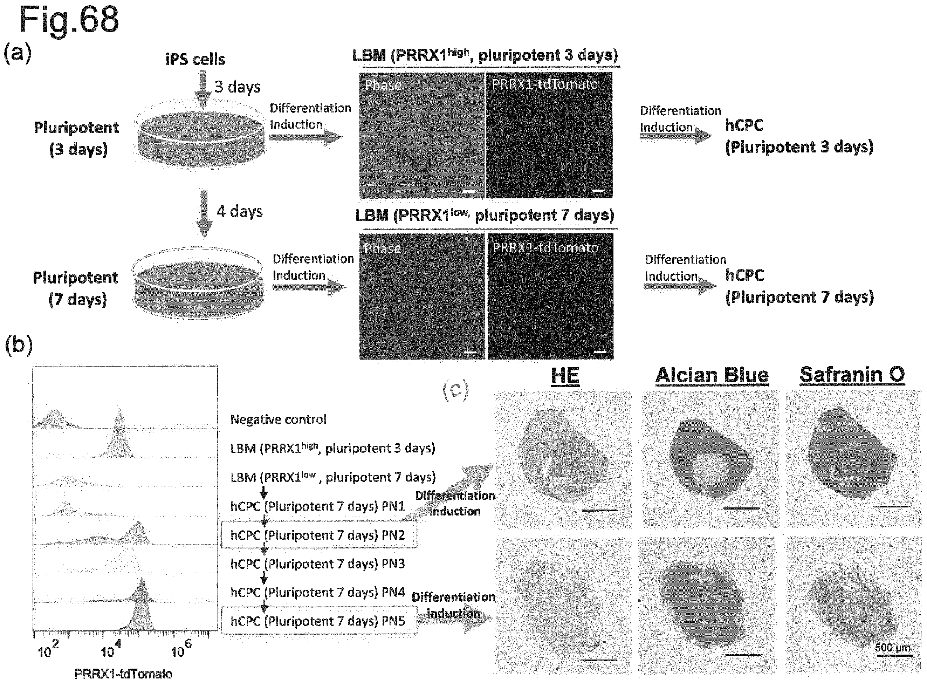

Fig. 68(a) and Fig. 68(b): the PRRX1 expression pattern of PRRX1-positive human limb bud mesenchymal cells (LBMs) and hCPCs produced therefrom. Fig. 68(c): cartilage tissues produced from respective hCPCs. hCPCs in each PRRX1-expressing state were collected from their culture dish, and were subjected to differentiation induction in accordance with the illustrated procedure. Tissues formed after cartilage differentiation induction were fixed and subjected to HE staining, Alcian blue staining, and Safranin O staining, and as a result, it was found that hCPCs maintained in a state of having a high expression amount of PRRX1 had a high chondrocyte/cartilage tissue-forming ability, but hCPCs maintained in a state of having a low expression amount of PRRX1 had a low chondrocyte/cartilage tissue-forming ability. That is, it is found that there is a positive correlation between the expression amount of PRRX1 and the final cartilage tissue-forming ability thereof.

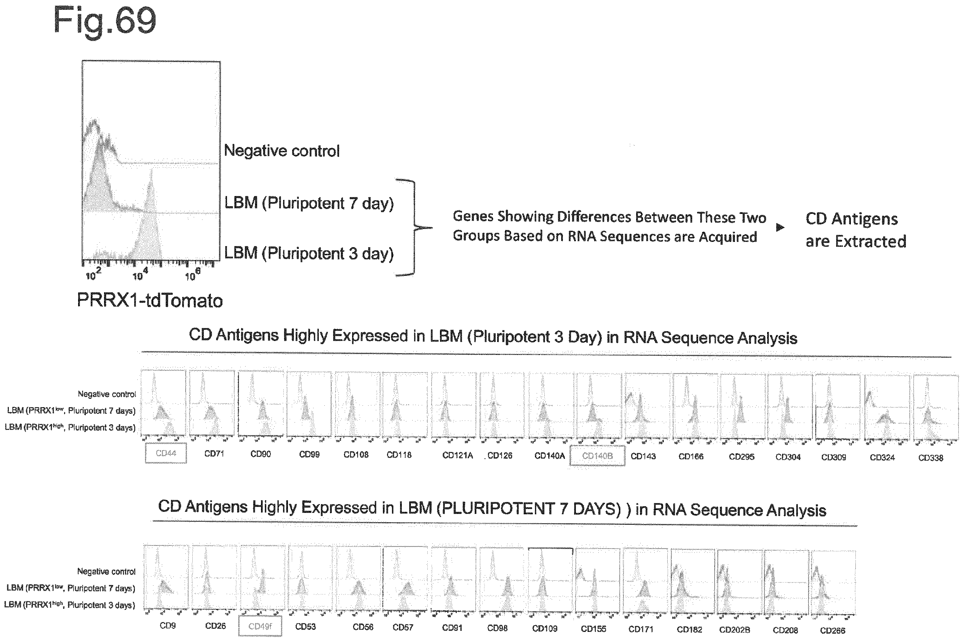

Fig. 69: candidate genes for selecting PRRX1-negative or -positive LBMs The results of flow cytometry of each surface antigen identified by RNAseq as having a correlation with the expression amount of PRRX1. LBMs having a low expression amount of PRRX1 (PRRX1-negative) or LBMs having a high expression amount of PRRX1 (PRRX1-positive) were stained with antibodies against the respective surface antigens, and expression amounts were compared by flow cytometry. As a result, it was found that CD44, CD99, and CD140B were highly expressed in the PRRX1-positive cells, and CD9, CD49f, and CD57 were highly expressed in the PRRX1-negative cells.

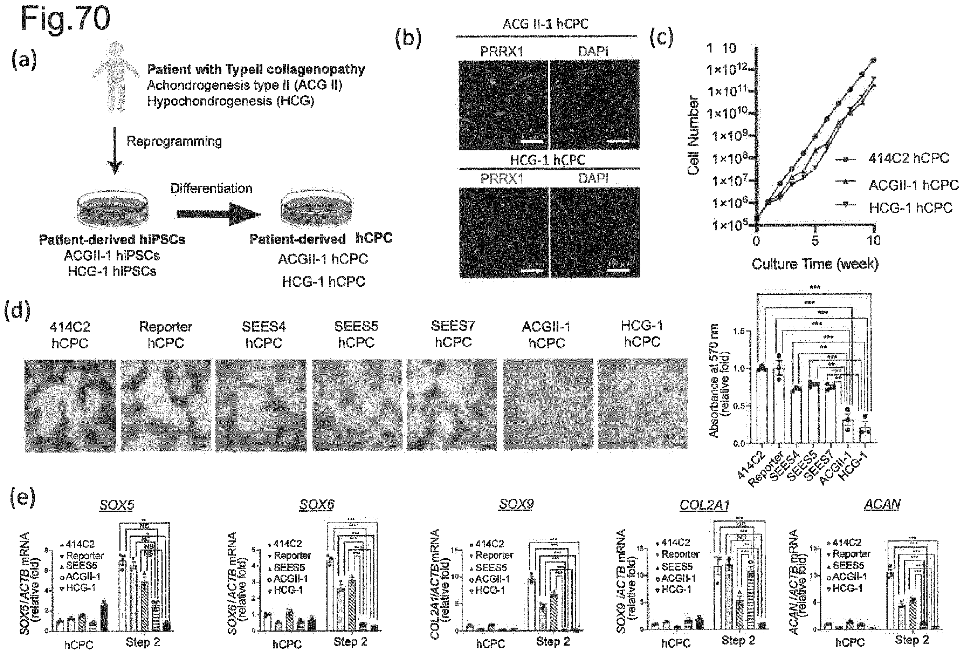

Figs. 70: a type II collagenopathy disease model 1. Fig. 70(a): a protocol for generating hCPCs from each of type II collagenopathy-related disease patient-derived iPSC lines (ACGII-1 and HCG-1), Fig. 70(b): immunostaining of PRRX1 expressions for respective hCPCs, Fig. 70(c): growth curves, Fig. 70(d): the results of cartilage matrix staining by Alcian blue staining, Fig. 70(e): measurement (qPCR) of the expressions of various cartilage differentiation marker genes.

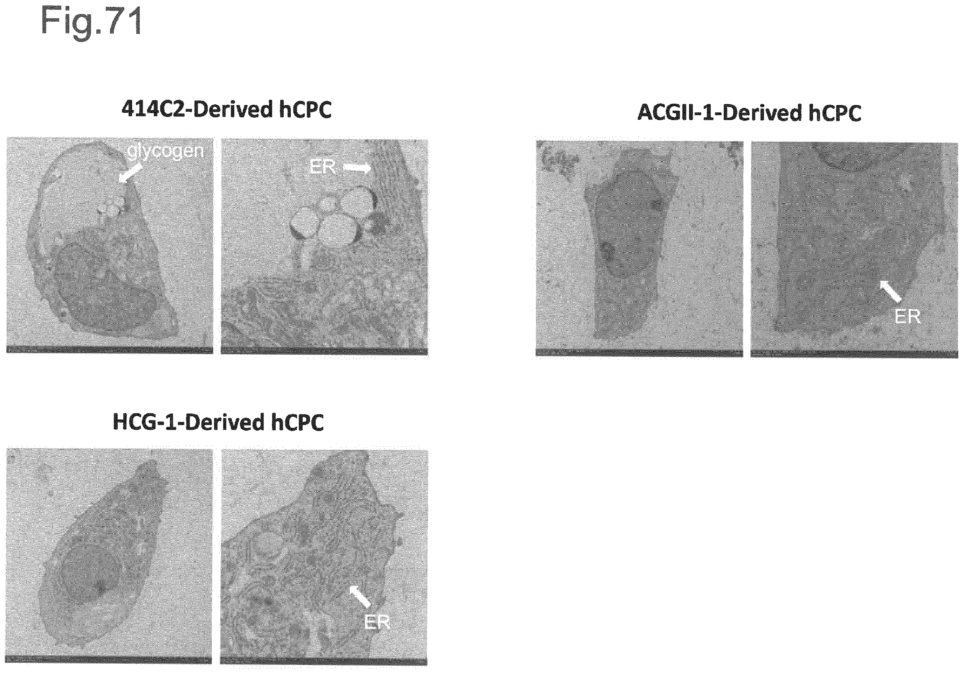

Fig. 71: a type II collagenopathy disease model 2. In type II collagenopathy-related disease patient-derived CPCs (ACGII-1 and HCG-1), reductions in glycogen amount in the chondrocytes were observed as compared to healthy individual-derived cells (414C2). In addition, in the patient-derived cells, normal aligned arrangement of ER found in normal cells is not found.

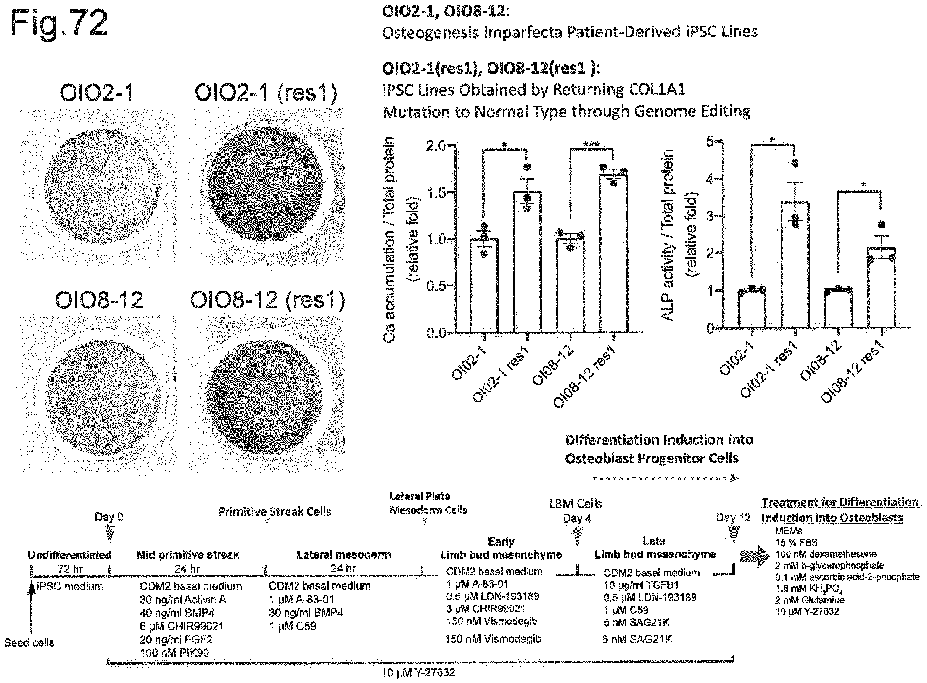

Fig. 72: an osteogenesis imperfecta disease model.

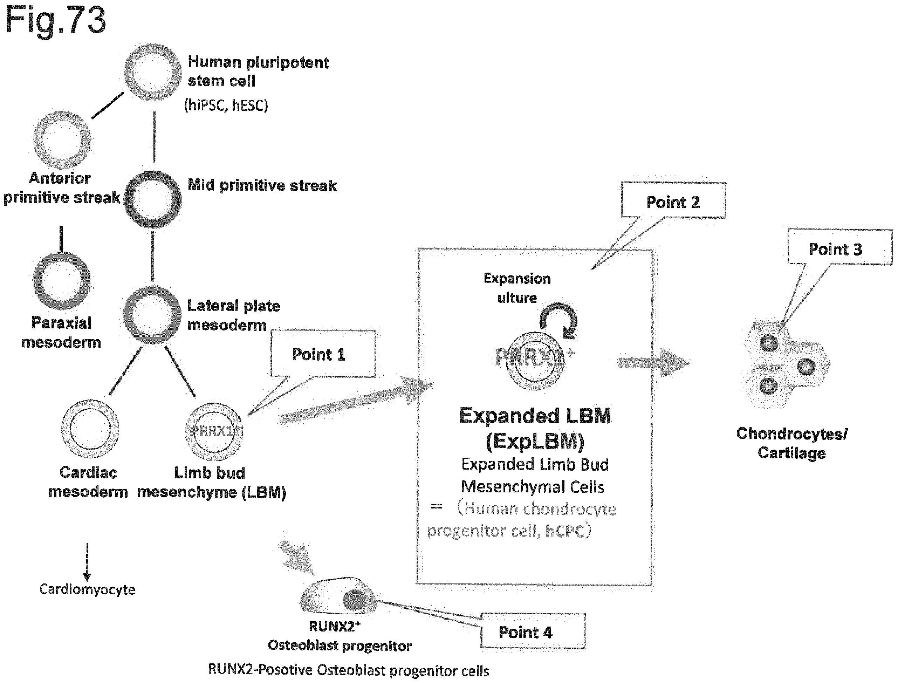

Fig. 73 is an illustration of an overview of the present invention.

### Description of Embodiments

### 1. Limb Bud Mesenchymal Cells, Chondrocyte Progenitor Cells, and RUNX2-positive Osteoblast Progenitor Cells

In the present invention, mammalian limb bud mesenchymal cells (LBMs) are pluripotent cells capable of differentiating into both chondrocyte progenitor cells and RUNX2-positive osteoblast progenitor cells. Meanwhile, the chondrocyte progenitor cells are cells different from the LBMs in that the chondrocyte progenitor cells do not change into osteoblasts. More satisfactory chondrocytes/cartilage tissue is obtained through induction from LBMs subjected to quality control in the present invention, or CPCs subjected to quality control in the present invention. High-quality LBMs or CPCs are obtained by a quality control method of the present invention, and osteoblast progenitor cells induced from the LBMs, osteoblasts or a bone tissue induced from the osteoblast progenitor cells, and chondrocytes or a cartilage tissue induced from the CPCs are of high quality applicable to transplantation.

Limb bud mesenchymal cells (LBMs) or chondrocyte progenitor cells and RUNX2-positive osteoblast progenitor cells (hereinafter sometimes abbreviated as "LBMs or differentiated cells thereof") of the present invention are derived from pluripotent stem cells. The LBMs and the chondrocyte progenitor cells are PRRX1 protein-positive, and the RUNX2-positive osteoblast progenitor cells are PRRX1 protein-negative. In addition, the LBMs of the present invention have growth properties, and for example, differentiation induction from the LBMs into a chondrocyte progenitor cell population may be performed under a Wnt signal-activating environment, and the differentiation induction and growth may be simultaneously performed. When the CPCs are passage-cultured in the presence of an FGF signal activator, the cell number thereof can be greatly increased. The inventors have recognized that the passage culture of the CPCs does not cause a chromosomal abnormality. In addition, the inventors have recognized that the CPCs having a greatly increased cell number as a result of the passage culture cause neither a benign tumor nor a malignant tumor when transplanted into a living body.

Herein, the term "pluripotent stem cells" means cells having both of an ability to self-renew (self-renewal ability) and an ability to differentiate into a plurality of other lineages of cells (pluripotency). That cells are pluripotent stem cells may be recognized on the basis of the expression of a stem cell marker, such as Oct3/4 or Klf-4, instead of by recognizing that the cells have a self-renewal ability and pluripotency. The term "progenitor cells" means cells in the course of differentiating from stem cells into functional cells serving as a final differentiation destination. The RUNX2-positive osteoblast progenitor cells are cells capable of being induced to differentiate into osteoblasts. Through differentiation induction from the LBMs into an osteoblast progenitor cell population, there can be obtained a RUNX2-positive osteoblast progenitor cell population having a RUNX2-positive rate of 95% or more, 96% or more, 97% or more, 98% or more, 99% or more, particularly 99.5% or more, most preferably 100%. Through use of an osteoblast progenitor cell population having a high RUNX2-positive rate, a higher-quality bone tissue can be produced. In addition, when chondrocytes are prepared from the chondrocyte progenitor cells of the present invention, a higher-quality cartilage tissue can be produced as compared to a case in which chondrocytes are directly obtained from pluripotent stem cells, such as iPS cells or ES cells. In particular, chondrocyte progenitor cells subjected to quality control in accordance with the quality control method of the present invention can be used to stably regenerate a high-quality cartilage tissue.

An organism from which the LBMs or the differentiated cells thereof of the present invention are derived is a mammal, such as a human, a mouse, a rat, a bovine, a horse, a pig, a rabbit, a dog, a cat, a goat, a monkey, or a chimpanzee. Of those, a human is preferred.

The "paired related homeobox 1 (PRRX1) protein" is a transcription factor having a homeodomain, and is known to be specifically expressed in a lateral plate mesoderm-derived limb bud in a developmental process.

The base sequence of cDNA of a human (*Homo sapiens*) PRRX1 gene and the amino acid sequence of the PRRX1 protein thereof are registered with the following accession numbers in GenBank provided by the National Center for Biotechnology Information (NCBI), U.S. (it is understood that, when a plurality of revisions are registered, the latest revision is referred to.) :
Human PRRX1 gene: NM_006902 (NM_006902.5), NM_022716 (NM_022716.4)
Human PRRX1 protein: NP_008833 (NP_008833.1), NP_073207 (NP_073207.1).

The PRRX1 protein positivity of cells may be detected by a known technique. Examples thereof include: detection with a reporter gene whose expression is controlled by a transcription promoter sequence of the PRRX1 gene; and detection by immunostaining using a specific antibody against the PRRX1 protein.

The LBMs or the differentiated cells thereof of the present invention are derived from pluripotent stem cells. Being derived from pluripotent stem cells means being cells obtained by differentiation induction using pluripotent stem cells as raw material cells. For example, the LBMs or the differentiated cells thereof derived from pluripotent stem cells are LBMs or differentiated cells thereof obtained through a differentiation induction process at least part of which has been performed outside a living body.

The pluripotent stem cells are preferably cells having complete pluripotency (ability to differentiate into all somatic cells and germline cells), such as embryonic stem cells (ES cells) or induced pluripotent stem cells (iPS cells).

The LBM cells according to a preferred embodiment of the present invention have a feature of at least one antigen marker expression positivity or negativity selected from the group consisting of CD9 negativity, CD24 positivity, CD29 positivity, CD44 positivity, CD46 positivity, CD49f negativity, CD55 positivity, CD57 negativity, CD58 positivity, CD90 positivity, CD99 positivity, CD140A positivity, CD140B positivity, SSEA1 negativity, SSEA3 negativity, SSEA4 positivity, TRA-1-60 negativity, TRA-1-81 negativity, CD73 negativity, CD105 negativity, and CD166 negativity.

The LBMs are PRRX1-positive, and have growth properties under an FGF signal-activating environment. In one embodiment of the present invention, surface antigens of the LBMs preferably satisfy at least one kind, at least two kinds, at least three kinds, at least four kinds, at least five kinds, or six kinds of conditions selected from the group consisting of CD44 positivity, CD99 positivity, CD140B positivity, CD9 negativity, CD49f negativity, and CD57 negativity (Fig. 60), and particularly preferably satisfy at least one kind, at least two kinds, or three kinds of conditions selected from the group consisting of CD44 positivity, CD140B positivity, and CD49f negativity. In a preferred embodiment of the present invention, when at least one kind, at least two kinds, or three kinds of conditions selected from the group consisting of CD44 positivity, CD140B positivity, and CD49f negativity are satisfied, one kind, two kinds, or three kinds of conditions selected from the group consisting of CD99 positivity, CD9 negativity, and CD57 negativity may be further satisfied. When the LBMs satisfy at least one kind, at least two kinds, at least three kinds, at least four kinds, at least five kinds, or six kinds of conditions selected from the group consisting of CD44 positivity, CD99 positivity, CD140B positivity, CD9 negativity, CD49f negativity, and CD57 negativity, the LBMs are preferred as a raw material to be induced to differentiate into chondrocyte progenitor cells or osteoblast progenitor cells. Accordingly, quality control of the LBMs as a raw material for chondrocyte progenitor cells or osteoblast progenitor cells may be performed by recognizing whether the LBMs satisfy at least one kind, at least two kinds, at least three kinds, at least four kinds, at least five kinds, or six kinds of conditions selected from the group consisting of CD44 positivity, CD99 positivity, CD140B positivity, CD9 negativity, CD49f negativity, and CD57 negativity.

In one preferred embodiment of the present invention, a mammalian chondrocyte progenitor cell population that satisfies at least one kind or two kinds of conditions selected from the group consisting of CD90 positivity and CD140B positivity can produce or regenerate a high-quality cartilage tissue. Accordingly, quality control of the chondrocyte progenitor cell population may be performed by investigating whether the chondrocyte progenitor cell population satisfies at least one kind or two kinds of conditions selected from the group consisting of CD90 positivity and CD140B positivity.

In the quality control method of the present invention, the presence or absence of the expression of at least one kind of surface antigen selected from the group consisting of CD44, CD99, CD140B, CD9, CD49f, and CD57 is detected in LBMs, and the presence or absence of the expression of at least one kind selected from the group consisting of CD90 and CD140B is detected in CPCs. The CPCs are preferably positive for CD90 and CD140B. The LBMs are preferably positive for CD44, CD99, and CD140B out of those six surface antigens, and preferably negative for CD9, CD49f, and CD57. Herein, the "positivity" and "negativity" for the expression of the CD antigens are judged by setting numerical values (negative histogram) obtained from a control (sample without antibodies).

PRRX1 positivity/negativity may be judged, for example, by using, as standards, numerical values of flow cytometry using PRRX1-tdTomato reporter cells. Specifically, a case in which the peak of measured values for tdTomato appears at a value of 4×10⁴ or more may be judged to be PRRX1-positive, and a case in which the peak appears at a value of 2×10⁴ or less may be judged to be PRRX1-negative. tdTomato is an example of a fluorescent label, and when any other label is used, PRRX1 positivity/negativity may be judged with similar standards.

The osteoblast progenitor cells or the chondrocyte progenitor cells may be produced in a large amount, cryopreserved, and used for the production or regeneration of bone or cartilage as needed.

That the cryopreserved chondrocyte progenitor cells are preferred as a transplantation material may be recognized by the chondrocyte progenitor cells satisfying at least one kind, at least two kinds, or three kinds of conditions selected from the group consisting of CD90 positivity and CD140B positivity. Accordingly, quality control of the chondrocyte progenitor cells may be performed by recognizing whether the chondrocyte progenitor cells satisfy the conditions of CD90 positivity and CD140B positivity.

When the chondrocyte progenitor cells are obtained by differentiation induction from iPS cells of a cartilage-related disease patient, the patient-derived chondrocyte progenitor cells serve as a disease model for the cartilage-related disease. The disease model may be used for screening for a therapeutic drug for the cartilage-related disease, and is preferably cryopreserved.

Similarly, when the osteoblast progenitor cells are obtained by differentiation induction from iPS cells of a bone-related disease patient, the patient-derived osteoblast progenitor cells serve as a disease model for the bone-related disease. The disease model may be used for screening for a therapeutic drug for the bone-related disease, and is similarly preferably cryopreserved.

Examples of the cartilage-related disease include type II collagenopathy, relapsing polychondritis, osteoarthritis, rheumatoid arthritis, and achondroplasia.

Examples of the bone-related disease include osteoarthritis, osteoporosis, osteomalacia, rheumatoid arthritis, femoral head necrosis, osteochondritis dissecans, and osteogenesis imperfecta.

When conditions under which the LBMs are obtained by differentiation induction from pluripotent stem cells, such as iPS cells or ES cells, are inappropriate, the quality of the LBMs is impaired. Accordingly, quality control is desirably performed by recognizing whether, before being induced into a chondrocyte progenitor cell population or an osteoblast progenitor cell population, the LBMs satisfy at least one kind, at least two kinds, at least three kinds, at least four kinds, at least five kinds, or six kinds of conditions selected from the group consisting of CD44 positivity, CD99 positivity, CD140B positivity, CD9 negativity, CD49f negativity, and CD57 negativity. Of the six kinds, i.e., CD44 positivity, CD99 positivity, CD140B positivity, CD9 negativity, CD49f negativity, and CD57 negativity, the three kinds of CD44 positivity, CD140B positivity, and CD49f negativity are more important. As long as at least one kind out of those three kinds is satisfied, at least one kind out of the other three kinds (CD99 positivity, CD9 negativity, and CD57 negativity) may be further satisfied. Further, for the chondrocyte progenitor cells, it is preferred that quality control based on the surface antigens of the LBMs and quality control based on the surface antigens of the chondrocyte progenitor cells be doubly performed.

In the preparation of the LBMs, first, pluripotent stem cells may be induced to differentiate into lateral plate mesoderm cells.

Known means may be used as means for inducing mammalian, particularly human, pluripotent stem cells to differentiate into lateral plate mesoderm cells. For example, a method in conformity with a method described in the literature: Loh et al, 2016, Cell, 451-467 may be adopted. Specifically, the mammal, particularly human, pluripotent stem cells are first induced to differentiate into primitive streak cells (mid-primitive streak), and then the primitive streak cells are induced to differentiate into lateral plate mesoderm cells.

That the differentiation induction into lateral plate mesoderm cells has been performed may be recognized by, for example, detecting the expression of a HAND1 protein serving as a specific marker for lateral plate mesoderm cells.

Then, the lateral plate mesoderm cells are cultured under a Wnt signal-activating environment to be induced to differentiate into LBMs, and the LBMs are further induced to differentiate. Thus, CPCs may be obtained. The CPCs subjected to quality control according to the present invention may be cryopreserved to be induced to become chondrocytes or a cartilage tissue as needed. hCPCs can be induced to become human chondrocytes or a human cartilage tissue, and hence are particularly preferred as a transplantation material for humans.

A transplantation material of the present invention may be used for treating a bone- or cartilage-related disease, and is also suitably used in other applications, such as plastic surgery or cosmetic surgery. For example, the shape of a nose is determined by major alar cartilage, septal nasal cartilage, lateral nasal cartilage, and the like, and the shape of an ear is determined by auricular cartilage. When the nose or the ear has, for example, a trauma from a traffic accident or the like, congenital nasal bone defect, or microtia, there arises a need to reshape or reconstruct the nose or the ear. The need can be met by generating and transplanting the LBMs or the CPCs of the present invention, or the chondrocytes or cartilage tissue induced therefrom with a desired shape. In the case of the reconstruction or cosmetic surgery of the nose or the ear, silicone, patient-derived costal cartilage, or the like is used. In this regard, silicone is a foreign body, and hence may cause a foreign body reaction, such as inflammation or an immunoreaction, and the use of the patient's costal cartilage is invasive. However, cartilage obtained according to the present invention can suppress such foreign body reaction and is non-invasive. Cartilage having a desired shape can be expected to have a cosmetic effect also when implanted at a site other than the nose or the ear, where there is originally no cartilage. For example, a cartilage plate or a cartilage block may be prepared and cut into a desired shape, to thereby generate a cartilage transplantation material having the desired shape.

The following description takes, as an example, the human, which is a particularly preferred mammal.

When hLBMs are induced into hCPCs under such inappropriate conditions as described below, a CPC population having a peak of cells that do not satisfy all of the conditions of CD90 positivity and CD140B positivity and a peak of cells that satisfy all thereof is obtained (double peak), or a CPC population formed of a single peak of cells that do not satisfy all of the conditions of CD90 positivity and CD140B positivity is obtained (Figs. 61). When such CPC population is induced to differentiate into chondrocytes or a cartilage tissue, high-quality chondrocytes or cartilage tissue that can be used for transplantation is not obtained.

In order to induce human limb bud mesenchymal cells (hLBMs) into hCPCs, when culture is performed under a confluent condition even once in the course of passage culture, a peak of cells that do not satisfy all of the conditions of CD90 positivity and CD140B positivity appears, resulting in hCPCs that are not suited for transplantation.

In order to obtain hCPCs that satisfy all of the conditions of CD90 positivity and CD140B positivity, it is preferred that the cells be passaged before becoming confluent.

As shown in Figs. 61, a hCPC population (PRRX1-negative) formed of a single peak of cells that do not satisfy all of at least one kind of condition selected from the group consisting of CD90 positivity and CD140B positivity is inappropriate for being induced into chondrocytes or a cartilage tissue, but when there is obtained a double peak of cells (PRRX1-positive) that satisfy all of at least one kind of condition selected from the group consisting of CD90 positivity and CD140B positivity and cells (PRRX1-negative) that do not satisfy all thereof, as passage culture is continued thereafter, all the cells converge into a cell population formed of cells that do not satisfy all of the conditions of CD90 positivity and CD140B positivity, and hence the culture may be stopped at a time point when a peak of cells that do not satisfy all of at least one kind of condition selected from the group consisting of CD90 positivity and CD140B positivity becomes able to be recognized. The following hCPCs can be induced into such satisfactory chondrocytes or cartilage tissue as to be usable for transplantation: at the end of culture, the hCPCs are such that a peak made up of cells that do not satisfy all of the conditions of CD90 positivity and CD140B positivity cannot be recognized; the ratio of cells that do not satisfy all of at least one kind of condition selected from the group consisting of CD90 positivity and CD140B positivity is preferably 5% or less, 4% or less, 3% or less, 2% or less, or 1% or less; and it is particularly preferred that the hCPCs be substantially free of such inappropriate cells. In addition, such satisfactory hCPCs are themselves useful as a transplantation material for treating cartilage-related diseases including chondrodystrophy and osteoarthritis.

As shown in Fig. 68(b), LBMs (PRRX1-negative) formed of a single peak of cells that do not satisfy all of the conditions of CD44 positivity, CD99 positivity, CD140B positivity, CD9 negativity, CD49f negativity, and CD57 negativity are inappropriate for being induced into PRRX1-positive hCPCs (=hCPCs that satisfy all of the conditions of CD90 positivity and CD140B positivity) or osteoblast progenitor cells. The following LBMs can induce hCPCs enough to be induced into such satisfactory chondrocytes or cartilage tissue as to be usable for transplantation, and osteoblast progenitor cells: at the end of culture, the LBMs are such that a peak made up of cells that do not satisfy all of the conditions of CD44 positivity, CD99 positivity, CD140B positivity, CD9 negativity, CD49f negativity, and CD57 negativity, in particular, CD44 positivity, CD140B positivity, and CD49f negativity cannot be recognized; the ratio of cells that do not satisfy all of the conditions of CD44 positivity, CD99 positivity, CD140B positivity, CD9 negativity, CD49f negativity, and CD57 negativity, in particular, the conditions of CD44 positivity, CD140B positivity, and CD49f negativity is preferably 5% or less, 4% or less, 3% or less, 2% or less, or 1% or less; and it is particularly preferred that the LBMs be substantially free of such inappropriate cells.

According to one particularly preferred embodiment of the present invention, there is provided a two-stage quality control method including: preparing CPCs using LBMs shown to be of satisfactory quality by the quality control method of the present invention; and subjecting the CPCs to the quality control method of the present invention. When the two-stage quality control method for LBMs and CPCs is performed, the CPC, and further, the chondrocytes or cartilage tissue to be finally obtained can be further improved in quality.

CPCs not only can themselves be used as a transplantation material, but also can be induced into chondrocytes or a cartilage tissue to serve as a transplantation material, and hence quality control of CPCs alone, or two-stage quality control of LBMs and CPCs is important for preparing a transplantation material.

The CPCs may be any one of a two-dimensional culture product or a massed three-dimensional culture product. When the CPC cell population is directly injected as a cell suspension (transplantation material) into an affected site, a plane culture product is suitably used, and massed CPCs can provide a massed cartilage tissue through differentiation induction. The cell suspension of the CPCs may be, for example, injected into an affected site having cartilage, such as a painful knee or meniscus, as with a hyaluronic acid injection solution, and can remove the cause of pain by regenerating cartilage unlike hyaluronic acid.

As shown in Fig. 67, when the hCPCs are a uniform cell population of a single peak within the ranges of predetermined standards for three surface antigens, satisfactory chondrocytes/cartilage tissue is induced, and when chondrocytes/cartilage tissue is induced from hCPCs (double peak) of two or more peaks including a peak that satisfies at least one kind selected from the group consisting of CD90 positivity and CD140B positivity and a peak that does not, the chondrocytes/cartilage tissue has a defect, thereby being inappropriate as a transplantation material. Further, in the case of hCPCs of a single peak outside the ranges of predetermined standards for the three surface antigens, chondrocytes/cartilage tissue is substantially not obtained.

As shown in Fig. 69, when the LBMs are a uniform cell population of a single peak within the ranges of predetermined standards for six surface antigens, satisfactory hCPCs are induced, and when hCPCs are induced from LBMs (double peak) of two or more peaks including a peak that satisfies at least one kind selected from the group consisting of CD44 positivity, CD99 positivity, CD140B positivity, CD9 negativity, CD49f negativity, and CD57 negativity, in particular, at least one kind selected from the group consisting of CD44 positivity, CD140B positivity, and CD49f negativity, and a peak that does not, chondrocytes/cartilage tissue induced therefrom has a defect, thereby being inappropriate as a transplantation material. Further, in the case of LBMs of a single peak outside the ranges of predetermined standards for the six surface antigens, chondrocytes/cartilage tissue is substantially not obtained.

In one embodiment of the present invention, the CPCs may be differentiated into a cartilage-like tissue to be used as a transplantation material to be transplanted into a patient in need of cartilage transplantation.

In a method for differentiation induction from CPCs selected by the quality control method of the present invention into chondrocytes, a period for which culture under a Wnt signal-activating environment is performed is not particularly limited as long as the effects of the present invention are not impaired. The period may be set to, for example, from about 24 hours to about 12 days, or from about 4 days to about 8 days. As required, medium exchange may be performed. Culture conditions are preferably in conformity with a conventional method.

In the culture, passage may be performed as required. When passage is performed, the cells are collected before or immediately after reaching a confluent state, and the cells are seeded into a fresh medium. In addition, in the culture of the present invention, the medium may be appropriately exchanged.

The medium to be used in the present invention is not particularly limited. In a preferred embodiment, a known cartilage-inducing medium may be used. As the cartilage-inducing medium, there is given a known composition containing L-ascorbic acid, ITS, GDF5, BMP4, and the like. As required, the medium may be supplemented with components such as antibiotics, such as streptomycin and penicillin, Non-Essential Amino Acids (NEAA), and a ROCK inhibitor (e.g., Y-27362) .

Thus, chondrocytes or a cartilage tissue is produced.

That the chondrocytes have been obtained may be recognized on the basis of, for example, positivity for Safranin O staining or positivity for Alcian Blue staining.

The LBMs of the present invention may be used as, for example, a raw material for differentiation induction into functional cells and tissues into which PRRX1-positive cells (e.g., limb bud) differentiate in a living body. Examples of such functional cells and tissues serving as differentiation destinations include bone, fibroblasts (e.g., dermal fibroblasts), joints, cartilage, and tendons and ligaments.

With regard to the LBMs or the differentiated cells thereof according to a preferred embodiment of the present invention, the LBMs, or the differentiated cells thereof, i.e., the chondrocyte progenitor cells or the RUNX2-positive osteoblast progenitor cells may be cryopreserved. The cryopreserved LBMs, or chondrocyte progenitor cells or RUNX2-positive osteoblast progenitor cells can be grown by passage culture after being thawed.

### 2. Method of Inducing Pluripotent Stem Cells to Differentiate into LBMs

The LBMs of the present invention described above may be produced by a combination of: a step of inducing pluripotent stem cells to differentiate into lateral plate mesoderm cells; and a step of culturing the cells obtained in the differentiation induction step under a Wnt signal-activating environment.

### Method for Differentiation Induction via Lateral Plate Mesoderm Cells

### Pluripotent Stem Cells

In the method of producing the LBMs of the present invention, the pluripotent stem cells to be used may be those described above.

### Lateral Plate Mesoderm Cells

In the method of producing the LBMs of the present invention, first, the pluripotent stem cells are induced to differentiate into lateral plate mesoderm cells.

The differentiation induction from the pluripotent stem cells into the lateral plate mesoderm cells may be performed as described above (see Fig. 73).

That the differentiation induction into lateral plate mesoderm cells has been performed may be recognized by, for example, detecting the expression of a HAND1 protein serving as a specific marker for lateral plate mesoderm cells.

### Differentiation Induction from Lateral Plate Mesoderm Cells into PRRX1-positive Limb Bud Mesenchymal Cell Population of the Present Invention

Then, the differentiation-induced lateral plate mesoderm cells are cultured under a Wnt signal-activating environment to be induced to differentiate into the PRRX1-positive LBMs of the present invention. In order to reduce the influence of the environment of the previous culture, it is preferred that the culture under a Wnt signal-activating environment be performed after washing with a PBS buffer or the like has been appropriately performed. The differentiation induction from the lateral plate mesoderm cells into the LBMs is preferably performed in the absence of any FGF signal activator, such as FGF2.

When the lateral plate mesoderm cells are cultured in the presence of an FGF signal activator, such as FGF2, and BMP, the cells differentiate into a cardiac mesoderm. Accordingly, when the lateral plate mesoderm is induced into the LBMs, a Wnt signal-activating condition is used. However, it is desired to avoid the addition of an effective amount of an FGF signal activator, such as FGF2, into the medium. The lateral plate mesoderm cells are induced to differentiate into the LBMs in an FGF signal activator-free medium, and hence the cell number is not greatly increased at the time of the induction into the LBMs.

The present invention has a feature in that the LBMs are cultured under a Wnt signal-activating environment, preferably further in the presence of an FGF signal activator, such as FGF2, to thereby obtain chondrocyte progenitor cells while increasing the cell number. Hitherto, it has been considered that the FGF signal activator cannot be used for LBMs because, when allowed to act on a lateral plate mesoderm, the FGF signal activator promotes differentiation into cells that are not of interest, such as a cardiac mesoderm. However, the inventors have found that high-quality chondrocyte progenitor cells are obtained by allowing the Wnt signal activator and the FGF signal activator to act after the induction into LBMs has been performed.

### Culture under Wnt Signal-activating Environment

The lateral plate mesoderm cells may be cultured under a Wnt signal-activating environment by being, for example, cultured in the presence of an effective amount of a Wnt signal activator. As described above, at the time of this culture, it is preferred that the content of the FGF signal activator be less than an effective amount for a cell growth-promoting action, and it is more preferred that the environment be free of any FGF2 signal activator.

The Wnt signal activator enhances Wnt-mediated signal transduction (in particular, a canonical Wnt pathway). Examples of the Wnt signal activator include a GSK3β inhibitor and a Wnt family protein.

Examples of the GSK3β inhibitor include CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile), XAV939, and LiCl.

When CHIR99021 is used as the Wnt signal activator, its addition amount may be set to, for example, from about 0.1 µM to about 20 µM, preferably from 1 µM to 10 µM.

### Culture under BMP Signal-suppressing Environment

In a preferred embodiment of the present invention, the step of culturing the differentiation-induced lateral plate mesoderm cells under a Wnt signal-activating environment may be performed under a BMP signal-suppressing environment.

The culture under a BMP signal-suppressing environment may be performed by, for example, culturing the cells in the presence of an effective amount of a BMP signal suppressor.

The BMP signal suppressor suppresses (inhibits) BMP-mediated signal transduction. Examples of the BMP signal suppressor include LDN193189 (4-[6-[4-(1-piperazinyl)phenyl]pyrazolo[1,5-a]pyrimidin-3-yl]quinoline) or a salt (e.g., hydrochloride) thereof, and DMH-1.

When LDN193189 is used as the BMP signal suppressor, its addition amount may be set to, for example, from about 0.1 µM to about 10 µM, preferably from 0.2 µM to 5 µM.

### Culture under TGFβ Signal-suppressing Environment

In a preferred embodiment of the present invention, the step of culturing the differentiation-induced lateral plate mesoderm cells under a Wnt signal-activating environment may be performed further under a TGFβ signal-suppressing environment.

The culture under a TGFβ signal-suppressing environment may be performed by, for example, culturing the cells in the presence of an effective amount of a TGFβ signal suppressor.

The TGFβ signal suppressor suppresses (inhibits) TGFβ-mediated signal transduction. Examples of the TGFβ signal suppressor include A-83-01 (3-(6-methyl-2-pyridinyl)-N-phenyl-4-(4-quinolinyl)-1H-pyrazole-1-carbothioamide) and SB431542.

When A-83-01 is used as the TGFβ signal suppressor, its addition amount may be set to, for example, from about 0.1 µM to about 10 µM, preferably from 0.2 µM to 5 µM.

### Culture under Hedgehog Signal-suppressing Environment

In a preferred embodiment of the present invention, the step of culturing the differentiation-induced lateral plate mesoderm cells under a Wnt signal-activating environment is preferably performed further under a hedgehog (HH) signal-suppressing environment.

The culture under a hedgehog signal-suppressing environment may be performed by, for example, culturing the cells in the presence of an effective amount of a hedgehog signal suppressor.

The hedgehog signal suppressor suppresses (inhibits) hedgehog-mediated signal transduction. Examples of the hedgehog signal suppressor include vismodegib, cyclopamine, and sonidegib.

When vismodegib is used as the hedgehog signal suppressor, its addition amount may be set to, for example, from about 10 nM to about 1 µM, preferably from about 50 nM to about 500 nM.

In method (A) of the present invention, from the viewpoint of the efficiency of induction into the LBMs or the differentiated cells thereof of the present invention of interest, it is preferred to perform culture under a Wnt signal-activating environment and under a BMP signal-suppressing environment, it is more preferred to perform culture under a Wnt signal-activating environment and under a BMP signal-suppressing environment, and under a TGFβ signal-suppressing environment and/or under a hedgehog signal-suppressing environment, and it is still more preferred to perform culture under a Wnt signal-activating environment, under a BMP signal-suppressing environment, under a TGFβ signal-suppressing environment, and under a hedgehog signal-suppressing environment.

The differentiation induction of the lateral plate mesoderm cells into the LBMs is performed under a Wnt signal-activating environment, and may be performed further in the presence of one kind, two kinds, or three kinds selected from the group consisting of a TGFβ signal suppressor, a BMP signal suppressor, and a hedgehog signal suppressor, more preferably in the presence of a TGFβ signal suppressor, a BMP signal suppressor, and a hedgehog signal suppressor.

### Culture

The culture may be performed in an appropriate vessel for accommodating cells and a medium. A technique for performing suitable culture is exemplified by a technique involving performing culture under the conditions of about 37°C and a carbon dioxide concentration of about 5%, but is not limited thereto. The culture under the above-mentioned conditions may be performed, for example, while the temperature and the CO₂ concentration are controlled using a known CO₂ incubator.

The culture may be performed by two-dimensional cell culture (plane culture). The two-dimensional cell culture may be performed with a vessel subjected to coating treatment for promoting adhesion of the cells as required.

A period for which the culture under a Wnt signal-activating environment is performed is not particularly limited as long as the effects of the present invention are not impaired. The period may be set to, for example, from about 6 hours to about 4 days, preferably from about 1 day to about 3 days, more preferably about 2 days (about 48 hours). As required, medium exchange may be performed. Culture conditions are preferably in conformity with a conventional method.

In the culture, passage may be performed as required. When passage is performed, the cells are collected before or immediately after reaching a confluent state, and the cells are seeded into a fresh medium. In addition, in the culture of the present invention, the medium may be appropriately exchanged. When the culture is continued after reaching a confluent state, the LBMs are degraded in quality, thereby being highly liable to fail to satisfy the conditions in quality control.

### Medium

A serum-free medium, such as CDM2 basal medium, IMDM medium, or F12 medium, is preferably used as the medium. Differentiation induction and maintenance culture may be performed under a state of being free of any animal-derived component. In addition, the medium may be supplemented with components such as antibiotics, such as streptomycin and penicillin, Non-Essential Amino Acids (NEAA), and a ROCK inhibitor (e.g., Y-27632).

Thus, the LBMs of the present invention, which are derived from pluripotent stem cells and are PRRX1 protein-positive, are produced.

It is conceived that the PRRX1 protein-positive LBM cells produced according to the present invention individually have properties comparable to those of limb bud cells differentiated from lateral plate mesoderm cells in a living body. The LBMs of the present invention are a homogeneous cell population, but LBM cells in a living body are in a state of coexisting with various cells, and for example, exist as a population including cells that do not satisfy one or both of: the condition of PRRX1 protein positivity; and the conditions of CD44 positivity, CD99 positivity, CD140B positivity, CD9 negativity, CD49f negativity, and CD57 negativity (in particular, CD44 positivity, CD140B positivity, and CD49f negativity), and hence the limb bud mesenchymal cell population (LBM) of the present invention is novel.

The LBMs according to the present invention are homogeneous and of high quality, and can be induced into chondrocyte progenitor cells and osteoblast progenitor cells. The chondrocyte progenitor cells and the osteoblast progenitor cells themselves, or osteoblasts, chondrocytes, bone tissue, and cartilage tissue further obtained therefrom are excellent as transplantation materials.

### 3. Differentiation Induction into Osteoblast Progenitor Cells

The LBMs of the present invention may be used as, for example, raw material cells to be induced to differentiate into RUNX2-positive osteoblast progenitor cells (hereinafter sometimes referred to as "RUNX2-positive cells").

The RUNX2-positive cells mean cells capable of differentiating and developing into osteoblasts, and further into a bone tissue. The RUNX2-positive cells and the osteoblasts induced therefrom may each be used as a transplantation material to be transplanted into a patient in need of bone regeneration.

The RUNX2-positive cells may be obtained by a method including a step of culturing the LBMs of the present invention under a non-Wnt signal-activating environment.

### Culture under Non-Wnt Signal-activating Environment

Specifically, the culture under a non-Wnt signal-activating environment may be performed by culture using a medium substantially free of any Wnt signal activator. When a medium containing a Wnt signal activator is used immediately before the step of performing the culture under a Wnt signal activator-free environment, it is preferred that the culture under a Wnt signal activator-free environment be performed after washing with a PBS buffer or the like has been appropriately performed to remove the Wnt signal activator. The case where such washing is performed and the like are encompassed in "under a non-Wnt signal-activating environment" because the Wnt signal is not activated even when the medium contains a minute amount of the Wnt signal activator.

Examples of the Wnt signal activator include the above-mentioned ones.

### Culture under BMP Signal-suppressing Environment

In the differentiation induction into the RUNX2-positive cells in a preferred embodiment of the present invention, the LBMs are cultured under a BMP signal-suppressing environment in a serum-free medium.

The culture under a BMP signal-suppressing environment may be performed by, for example, culturing the cells in the presence of an effective amount of a BMP signal suppressor.

Examples of the BMP signal suppressor include the above-mentioned ones.

When LDN193189 is used as the BMP signal suppressor, its addition amount may be set to, for example, from about 0.1 µM to about 1 µM, preferably from 0.2 µM to 5 µM.

### Culture under TGFβ Signal-activating Environment

In the differentiation induction into the RUNX2-positive cells in a preferred embodiment of the present invention, the step of performing culture in a serum-free medium may be performed further under a TGFβ signal-activating environment. environment.

The culture under a TGFβ signal-activating environment may be performed by, for example, culturing the cells in the presence of an effective amount of a TGFβ signal activator.

The TGFβ signal activator enhances TGFβ-mediated signal transduction. An example of the TGFβ signal activator is a TGFβ1 protein.

When the TGFβ1 protein is used as the TGFβ signal activator, its addition amount may be set to, for example, from about 0.1 µM to about 10 µM, preferably from 0.2 µM to 5 µM.

### Culture under Hedgehog Signal-activating Environment

In the differentiation induction into the RUNX2-positive cells in a preferred embodiment of the present invention, the step of performing culture in a serum-free medium is preferably performed further under a hedgehog (HH) signal-activating environment.

The culture under a hedgehog signal-activating environment may be performed by, for example, culturing the cells in the presence of an effective amount of a hedgehog signal activator.

The hedgehog signal activator enhances hedgehog-mediated signal transduction. An example of the hedgehog signal activator is a hedgehog agonist, such as 21K.

When 21K is used as the hedgehog signal activator, its addition amount may be set to, for example, from about 1 nM to about 100 nM, preferably from about 1 nM to about 10 nM.

### Culture

The culture may be performed in an appropriate vessel for accommodating cells and a medium. A technique for performing suitable culture is exemplified by a technique involving performing culture under the conditions of about 37°C and a carbon dioxide concentration of about 5%, but is not limited thereto. The culture under the above-mentioned conditions may be performed, for example, while the temperature and the CO₂ concentration are controlled using a known CO₂ incubator.

In the method of performing differentiation induction into the RUNX2-positive cells, the culture may be performed by two-dimensional cell culture (plane culture). The two-dimensional cell culture may be performed with a culture instrument subjected to coating treatment for promoting adhesion of the cells as required.

In the method of performing differentiation induction into the RUNX2-positive cells, a period for which the culture under a non-Wnt signal-activating environment is performed is not particularly limited as long as the effects of the present invention are not impaired. The period may be set to, for example, from about 1 day to about 20 days, preferably from about 3 days to about 15 days, more preferably about 5 days to about 10 days. As required, medium exchange may be performed. Culture conditions are preferably in conformity with a conventional method.

In the culture, passage may be performed as required. When passage is performed, the cells are collected before or immediately after reaching a confluent state, and the cells are seeded into a fresh medium. In addition, in the culture of the present invention, the medium may be appropriately exchanged.

### Medium

In the present invention, a serum-free medium, such as CDM2 basal medium, IMDM medium, or F12 medium, is preferably used. Differentiation induction and maintenance culture may be performed under a state of being free of any animal-derived component. In addition, the medium may be supplemented with components such as antibiotics, such as streptomycin and penicillin, Non-Essential Amino Acids (NEAA), and a ROCK inhibitor (e.g., Y-27362).

Thus, the RUNX2-positive cells are produced.

That the RUNX2-positive osteoblast progenitor cells have been obtained may be recognized on the basis of, for example, positivity for alizarin red staining after treatment of the RUNX2-positive osteoblast progenitor cells in an osteoblast differentiation-inducing medium.

### 4. Differentiation Induction of LBMs into Chondrocyte Progenitor Cells

The LBMs of the present invention may be used as, for example, raw material cells to be induced to differentiate into a chondrocyte progenitor cell population.

The chondrocyte progenitor cell population and the chondrocyte population may each be used as a transplantation material to be transplanted into a patient in need of cartilage transplantation.

The chondrocyte progenitor cell population may be obtained by a method including a step of culturing the LBMs according to the present invention under a Wnt signal-activating environment, preferably in the presence of an FGF signal activator, such as FGF2.

### Culture under Wnt Signal-activating Environment

The culture under a Wnt signal-activating environment may be performed by, for example, culturing the cells in the presence of an effective amount of a Wnt signal activator.

The Wnt signal activator enhances Wnt-mediated signal transduction (in particular, a canonical Wnt pathway). Examples of the Wnt signal activator include a GSK3β inhibitor and a Wnt family protein.

Examples of the GSK3β inhibitor include the above-mentioned ones.

When CHIR99021 is used as the Wnt signal activator, its addition amount may be set to, for example, from about 0.1 µM to about 20 µM, preferably from 1 µM to 10 µM.

### Culture in Presence of FGF Signal Activator

The differentiation induction of the LBMs into chondrocyte progenitor cells requires an FGF signal activator.

The FGF signal activator contributes to an increase in cell number as well as differentiation induction into chondrocyte progenitor cells, thereby enabling the chondrocyte progenitor cells to be obtained in a large amount.

An example of the FGF signal activator is FGF2, and FGF2 is preferred.

The differentiation induction from the chondrocyte progenitor cells into chondrocytes is preferably performed in a plurality of stages. Specifically, the differentiation induction is preferably performed in the following three stages:
(i) a step of performing culture under a Wnt signal-activating environment and under an FGF signal-activating environment;
(ii) a step of performing culture in the presence of an FGF signal activator (preferably in the absence of any Wnt signal activator); and
(iii) a step of performing culture preferably in the absence of any Wnt signal activator and in the absence of any FGF signal activator.

When the differentiation induction in the above-mentioned three stages is performed, the step (i) and the step (ii) may each be performed by two-dimensional cell culture (plane culture) or three-dimensional culture. The two-dimensional cell culture may be performed with a culture instrument subjected to coating treatment for promoting adhesion of the cells as required. The step (iii) is preferably performed by three-dimensional culture. Between the cultures of the respective stages, in order to reduce the influence of the environment of the previous culture, it is preferred that the culture be performed after washing with a PBS buffer or the like has been appropriately performed.

### Culture

The culture may be performed in an appropriate vessel for accommodating cells and a medium. A technique for performing suitable culture is exemplified by a technique involving performing culture under the conditions of about 37°C and a carbon dioxide concentration of about 5%, but is not limited thereto. The culture under the above-mentioned conditions may be performed, for example, while the temperature and the CO₂ concentration are controlled using a known CO₂ incubator.

In a method for differentiation induction from chondrocyte progenitor cells of the present invention, (i) a period for which culture under a Wnt signal-activating environment is performed is not particularly limited as long as the effects of the present invention are not impaired. The period may be set to, for example, from about 24 hours to about 12 days, or from about 4 days to about 8 days. As required, medium exchange may be performed, (ii) A period for which the culture in the presence of an FGF signal activator is performed is not particularly limited as long as the effects of the present invention are not impaired.
(iii) a period for which the culture preferably in the absence of any Wnt signal activator and in the absence of any FGF signal activator is performed is not particularly limited as long as the effects of the present invention are not impaired. In the culture of each of the steps (i) to (iii), medium exchange may be performed as required. Culture conditions are preferably in conformity with a conventional method.

In the culture, passage may be performed as required. When passage is performed, the cells are collected before or immediately after reaching a confluent state, and the cells are seeded into a fresh medium. In addition, in the culture of the present invention, the medium may be appropriately exchanged.

### Medium

The medium to be used in the method of the present invention is not particularly limited. In a preferred embodiment, a known cartilage-inducing medium may be used. As the cartilage-inducing medium, there is given a known composition containing L-ascorbic acid, ITS, GDF5, BMP4, and the like. As required, the medium may be supplemented with components such as antibiotics, such as streptomycin and penicillin, Non-Essential Amino Acids (NEAA), and a ROCK inhibitor (e.g., Y-27362).

Thus, chondrocytes are produced.

That the chondrocytes have been obtained may be recognized on the basis of, for example, positivity for Safranin O staining or positivity for Alcian Blue staining.

Each medium to be used in a kit of the present invention is described below.
(i) Medium for inducing pluripotent stem cells into primitive streak cells A known medium is given, and for example, a medium described in the literature: Loh et al, 2016, Cell, 451-467 may be used.
(ii) Medium for inducing primitive streak cells into lateral plate mesoderm cells A known medium is given, and for example, a medium described in the literature: Loh et al, 2016, Cell, 451-467 may be used.
(iii) Medium for inducing lateral plate mesoderm cells into limb bud mesenchymal cells A medium obtained by supplementing the above-mentioned serum-free medium with a Wnt signal activator is given. This medium is free of any FGF signal activator, such as FGF2.
(iv) Medium for inducing limb bud mesenchymal cells into chondrocyte progenitor cells A medium obtained by incorporating an FGF signal activator, such as FGF2, and a Wnt signal activator into the above-mentioned serum-free medium may be used.
(v) Medium for inducing chondrocyte progenitor cells into chondrocytes

In differentiation induction from chondrocyte progenitor cells into chondrocytes, at least one of the following media (a) to (c) is preferably used. It is more preferred that the kit include all of the media (a) to (c), or include the media (a) and (c).
(a) A medium obtained by supplementing the above-mentioned serum-free medium with a Wnt signal activator and an FGF signal activator. The presence of the Wnt signal activator allows induction into massed chondrocyte progenitor cells. At the stage of having been cultured in this medium, the cells have the properties of chondrocyte progenitor cells.
(b) A medium obtained by supplementing the above-mentioned serum-free medium with an FGF signal activator. This medium is preferably free of any Wnt signal activator. Through culture in this medium, chondrocyte progenitor cells can be induced to differentiate into chondrocytes.
(c) A medium that is the above-mentioned serum-free medium not supplemented with a Wnt signal activator and an FGF signal activator may be used. This medium is preferably supplemented with BMP4.

The kit may include only one or both of the above-mentioned media (b) and (c).
(vi) Medium for inducing limb bud mesenchymal cells into RUNX2-positive osteoblast progenitor cells

The above-mentioned serum-free medium free of any Wnt signal activator is given.

### Examples

Now, the present invention is described in more detail by way of Examples.

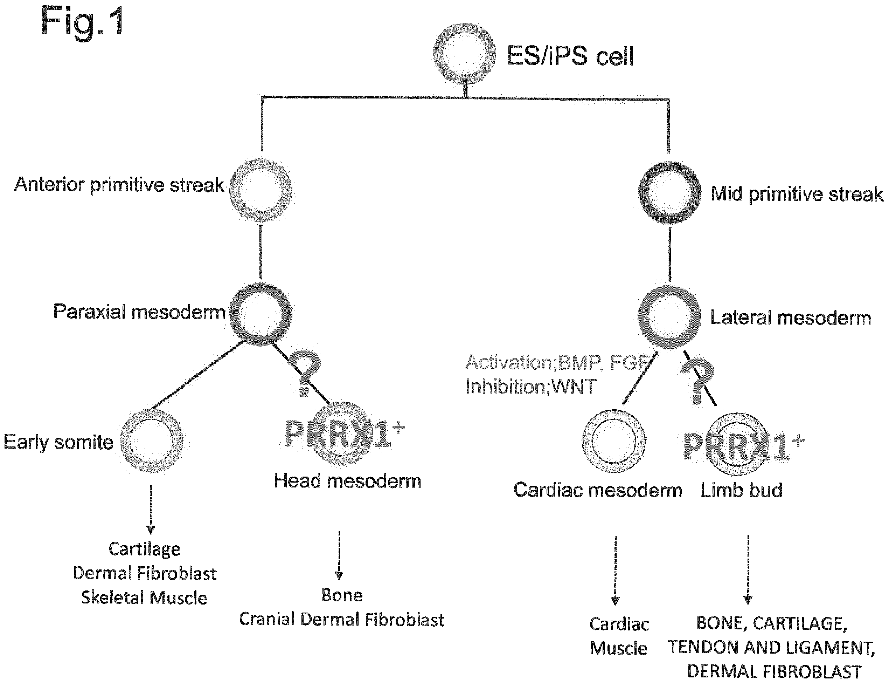

In Fig. 1, an overview of a differentiation induction protocol according to the present invention is illustrated.

A limb bud mesenchymal cell population can be induced to differentiate, via chondrocyte progenitor cells and RUNX2-positive osteoblast progenitor cells, further into mainly, for example, osteoblasts, chondrocytes, tendon and ligament cells, and dermal fibroblasts of osteoarticular tissues forming limbs.

### Reference Example 1: Generation of PRRX1 Reporter Human iPS Cells

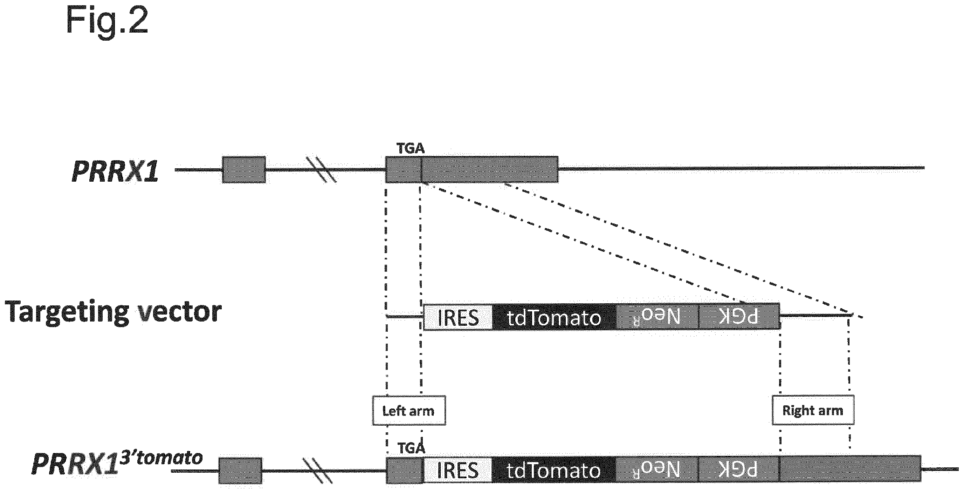

With use of a targeting vector illustrated in Fig. 2 and a CRISPR/Cas9 system, 414C2 cells serving as an iPS cell line were used as a parental line, and a fluorescent protein tdTomato was knocked into the PRRX1 gene locus. Thus, PRRX1 reporter iPS cells capable of visualizing PRRX1 expression in the iPS cells with the tdTomato fluorescent protein were generated.

### Example 1: Differentiation Induction into PRRX1-positive Cells via Lateral Plate Mesoderm Lineage

### (Induction of Lateral Plate Mesoderm from iPS Cells via Primitive Streak)

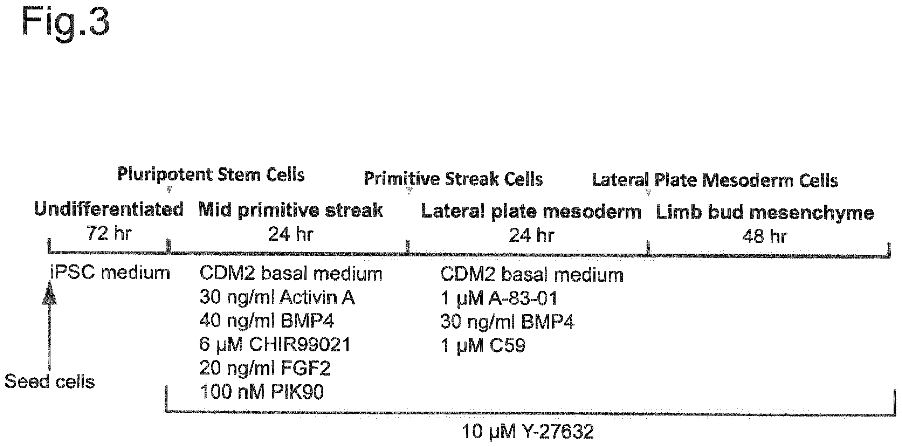

In accordance with a protocol illustrated in Fig. 3, iPS cell lines (414C2 and the PRRX1 reporter cell line) were each induced to differentiate into a primitive streak (mid-primitive streak, day 1), and then into a lateral plate mesoderm (day 2).

First, the undifferentiated PRRX1 reporter iPS cells obtained in Reference Example 1 were seeded and cultured in a general medium for iPS cells (iPSC medium) for 72 hours (hr) (day 0) .

Then, after being washed with PBS once, the cells were cultured in a medium having the following composition for 24 hours to be induced to differentiate into a primitive streak.

### • Medium for Primitive Streak Differentiation Induction CDM2 basal medium

30 ng/ml Activin A
40 ng/ml BMP4
6 µM CHIR99021
20 ng/ml FGF2
100 nM PIK90
10 µM Y-27632

Then, after being washed with PBS once, the cells were cultured in a medium having the following composition for 24 hours to be induced to differentiate into a lateral plate mesoderm.

### • Medium for Primitive Streak Differentiation Induction CDM2 basal medium

1 µM A-83-01
30 ng/ml BMP4
1 µM C59
10 µM Y-27632

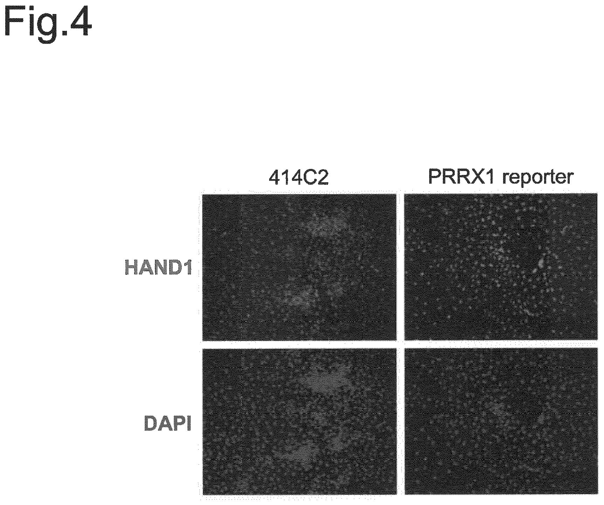

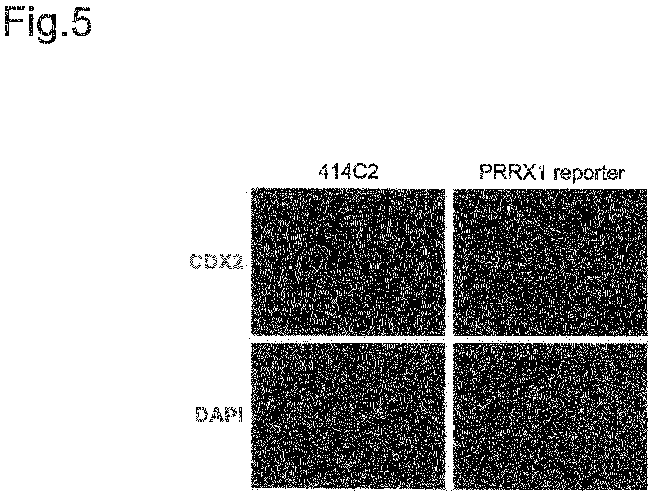

The induced cells were immunostained for HAND1 (lateral plate mesoderm marker) and CDX2 (paraxial mesoderm marker). The results are shown in Fig. 4 and Fig. 5. It was able to be recognized that the induced lateral plate mesoderm cells were HAND1-positive and CDX2-negative.

### (Investigation of Differentiation Induction Conditions for PRRX1-positive Cells)

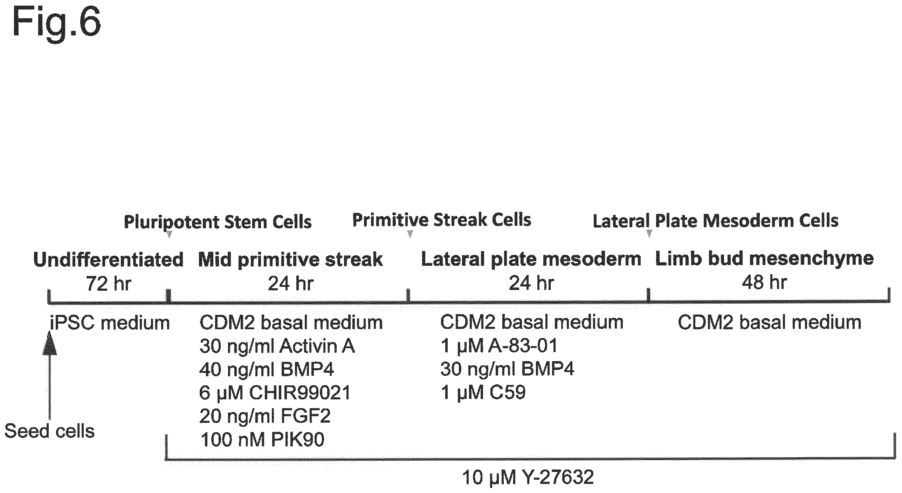

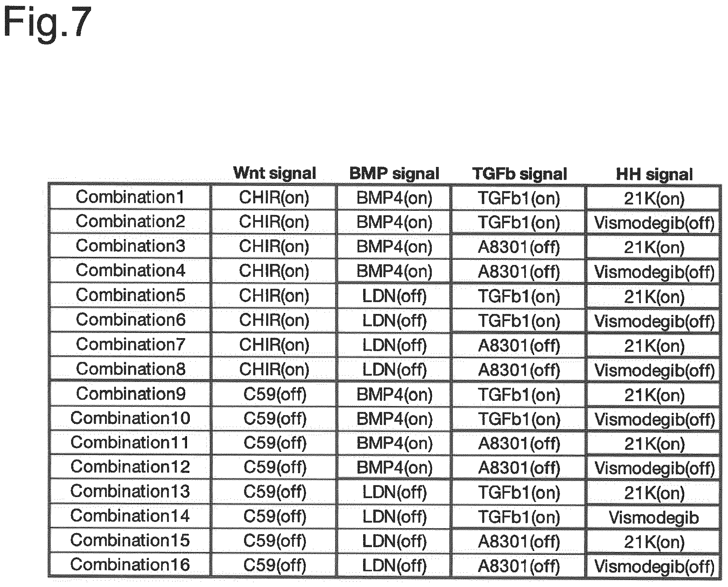

The above-mentioned induced lateral plate mesoderm cells (day 2) having the PRRX1 reporter were washed with PBS once, and were then cultured in CDM2 basal media containing activators and/or suppressors for various signals (16 combinations) shown in Fig. 7 for 48 hours (Fig. 6).

Reagents used are as described below.
CHIR (CHIR99021, GSK3β inhibitor)
C59 (Wnt-C59, PORCN inhibitor)
BMP4 (Bone morphogenetic Protein 4)
LDN (LDN193189, ALK2/ALK3 inhibitor)
TGFβ1 (Tumor growth factor β1)
A8301 (A83-01, ALK5 inhibitor)
21K (Hedgehog agonist)
Vismodegib (Hedgehog inhibitor)

The expression of PRRX1 in the cells after the 48 hours of culture (day 4) was evaluated through observation based on the expression of tdTomato serving as a reporter under a fluorescence microscope.

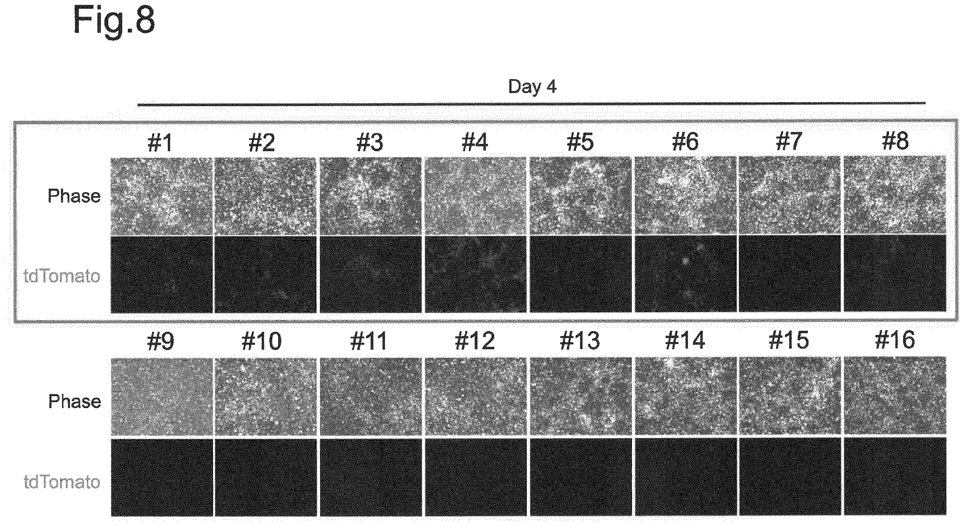

The results are shown in Fig. 8. Particularly high expressions of tdTomato were found in Combinations 1, 2, 3, 4, 5, 6, 7, and 8.

RNA was collected from the cells after the 48 hours of culture (day 4), and the expression of PRRX1 was evaluated by quantitative RT-PCR (qPCR).

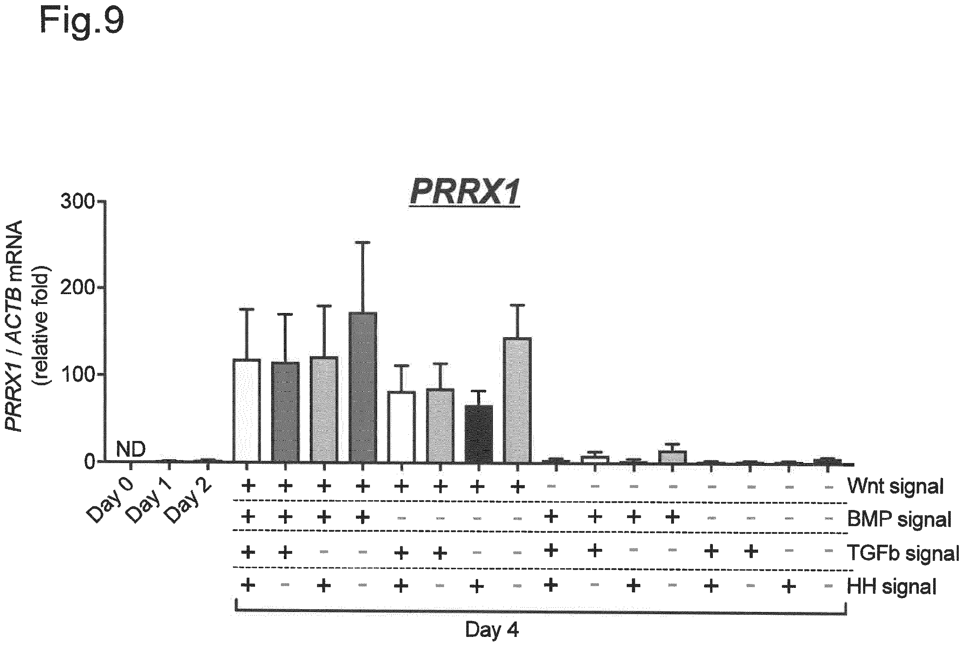

The results are shown in Fig. 9. Particularly high expressions of PRRX1 were found in Combinations 1, 2, 3, 4, 5, 6, 7, and 8.

In addition, RNA was collected from the cells after the 48 hours of culture (day 4), and the expression of HAND1 serving as a lateral plate mesoderm marker was measured by quantitative RT-PCR (qPCR).

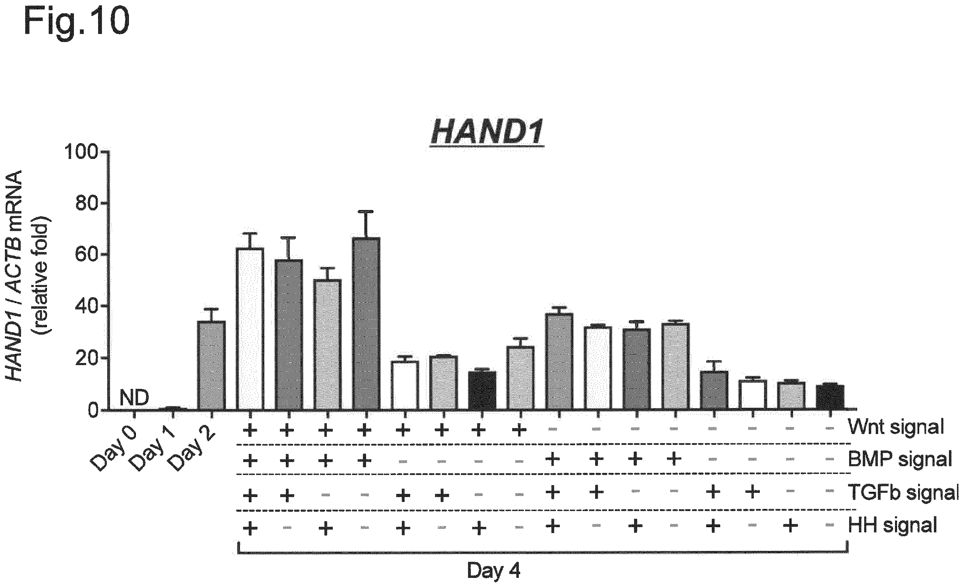

The results are shown in Fig. 10. In each of Combinations 1, 2, 3, and 4, the expression of HAND1 was increased as compared to that on day 2, while in each of Combinations 5, 6, 7, and 8, the expression of HAND1 was decreased.

In addition, RNA was collected from the cells after the 48 hours of culture (day 4), and the expression of ISL-1 serving as a lateral plate mesoderm marker was measured by quantitative RT-PCR (qPCR).

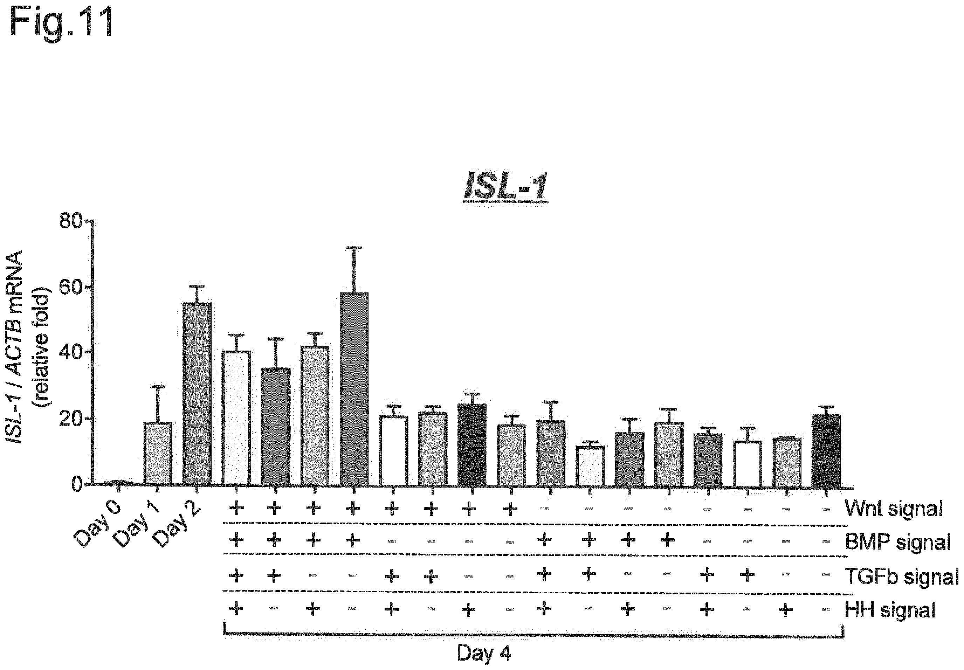

The results are shown in Fig. 11. In each of Combinations 5, 6, 7, and 8, the expression of ISL1 was decreased as compared to that on day 2.

In addition, RNA was collected from the cells after the 48 hours of culture (day 4), and the expression of FOXF1 serving as a lateral plate mesoderm marker was measured by quantitative RT-PCR (qPCR).

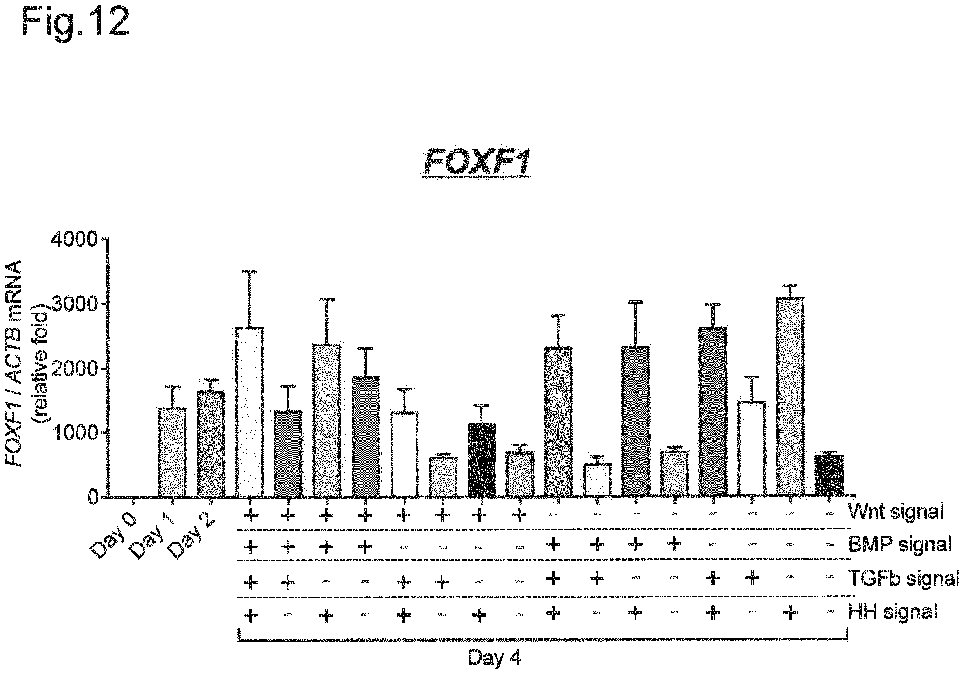

The results are shown in Fig. 12. In each of Combinations 5, 6, 7, and 8, the expression of FOXF1 was decreased as compared to that on day 2.

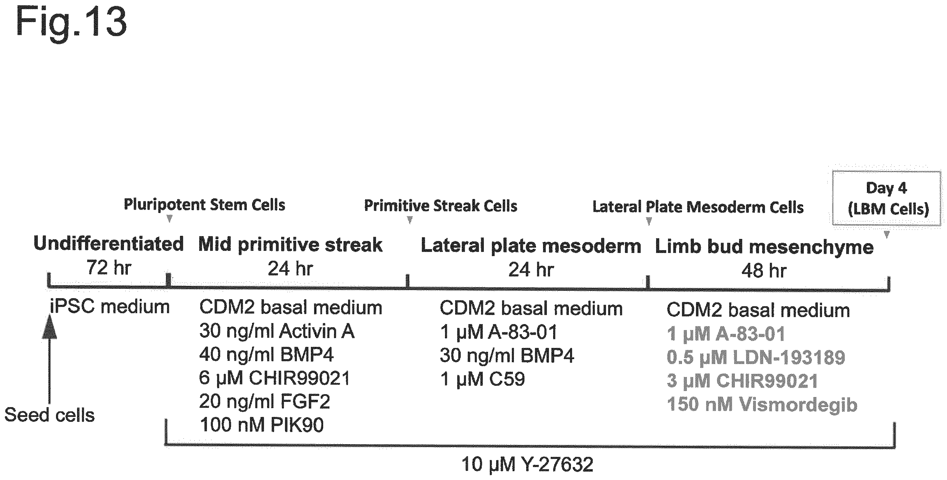

On the basis of the above-mentioned results, Combination 8 was selected as a particularly effective condition for inducing PRRX1-positive limb bud mesenchymal cells from lateral plate mesoderm cells (day 2) (Fig. 13).

### (Evaluation of Differentiation Induction Efficiency)

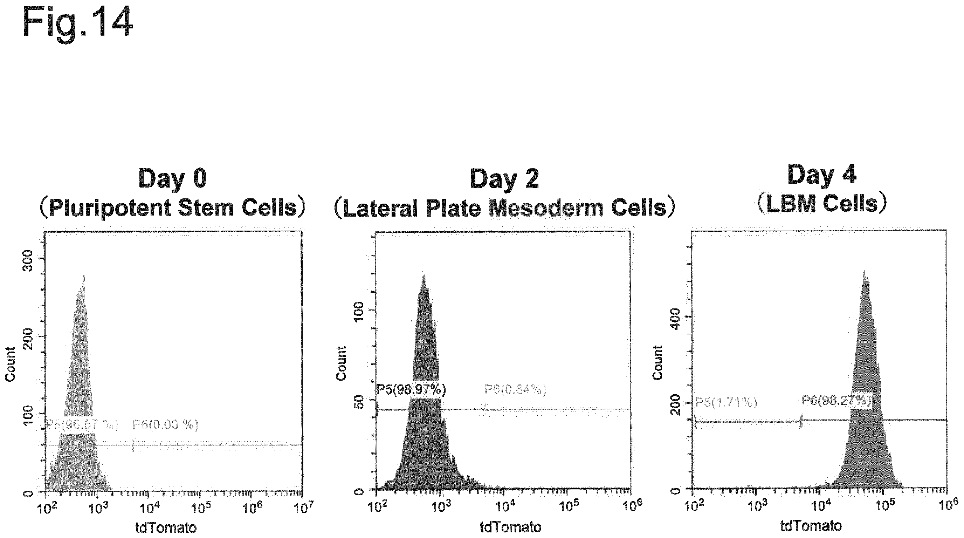

The efficiency of induction into tdTomato-positive cells in the above-mentioned differentiation induction process was evaluated by flow cytometry. The results are shown in Fig. 14. The efficiency of induction into tdTomato-positive cells on day 4 was 98% or more.

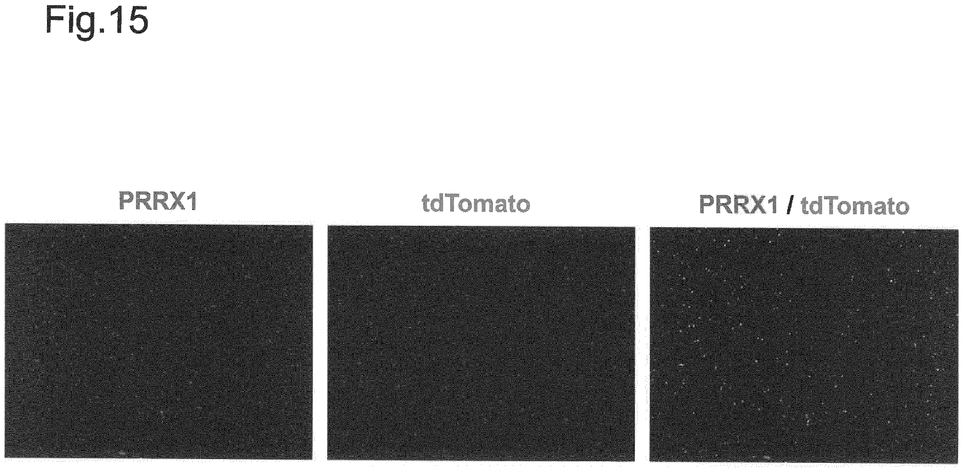

In addition, the cells on day 4 were subjected to immunostaining using a PRRX1 antibody. The results are shown in Fig. 15. All the cells that were PRRX1-positive were tdTomato-positive.

### (CD Antigen Expression Profile)

The expression of CD antigens in the tdTomato-positive cells in the above-mentioned differentiation induction process was evaluated using a CD antibody array (product name: BD Lyoplate Screening Panels, manufactured by BD).

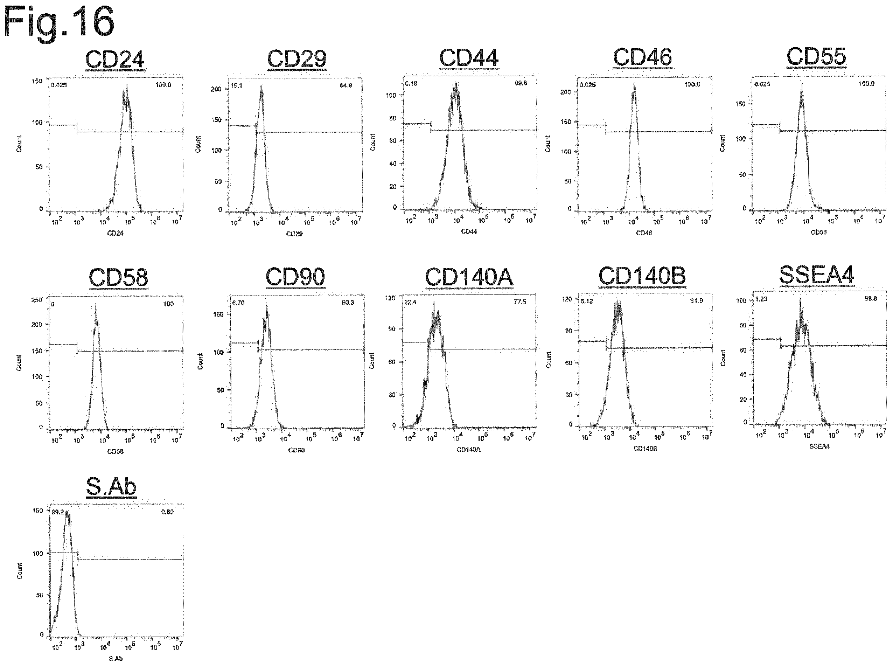

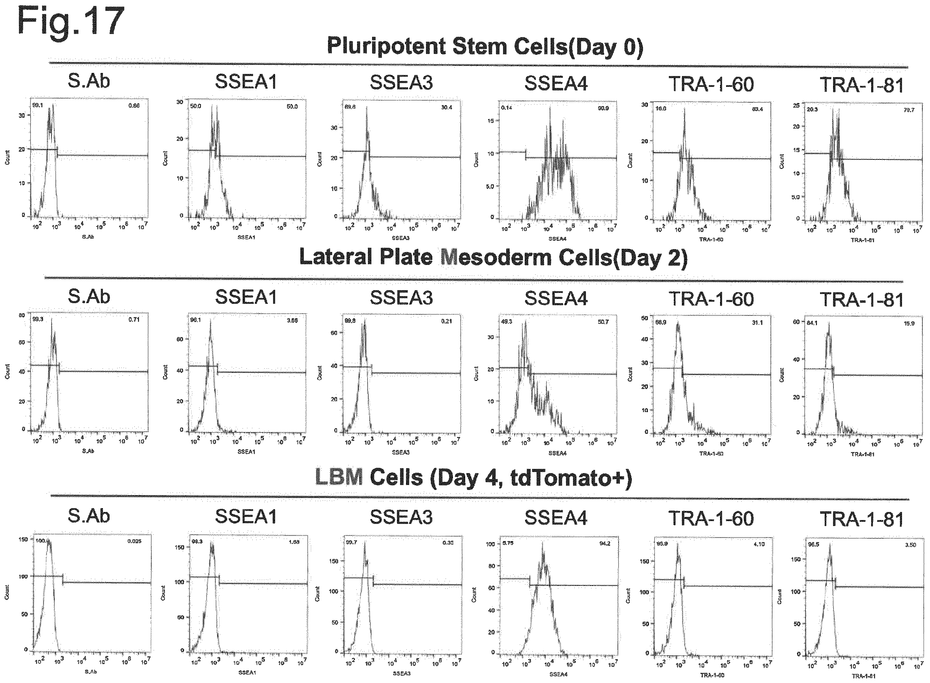

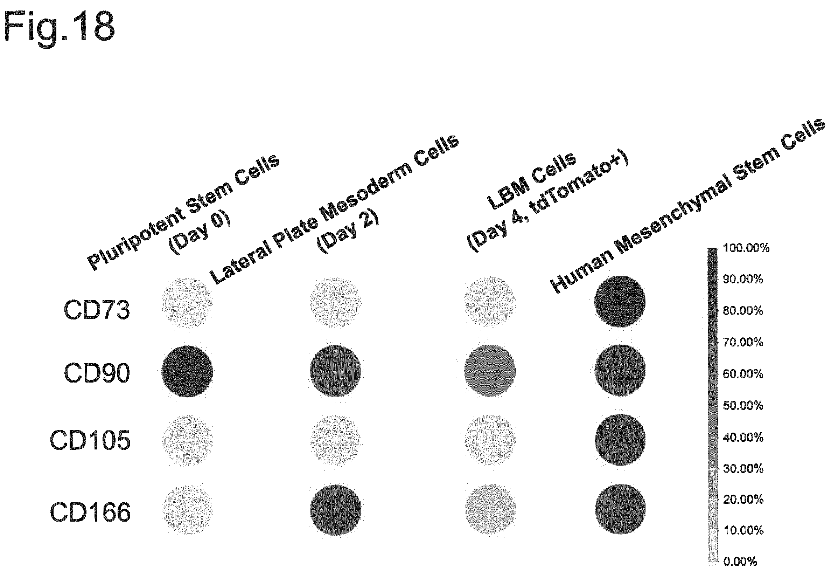

The results are shown in Fig. 16 to Fig. 18. The tdTomato-positive cells on day 4 were
1) CD24-positive, CD29-positive, CD44-positive, CD46-positive, CD55-positive, CD58-positive, CD90-positive, CD140A-positive, and CD140B-positive (Fig. 16),
2) SSEA1-negative, SSEA3-negative, SSEA4-positive, TRA-1-60-negative, and TRA-1-81-negative (Fig. 17), and
3) CD73-negative, CD90-positive, CD105-negative, and CD166-negative (Fig. 18).

That is, the tdTomato-positive cells on day 4 were as follows: 1) the cells were substantially uniform; 2) the expressions of most ES cell markers were decreased in the course of differentiation induction up to day 4; and 3) and the expressions of human mesenchymal stem cell (MSC) markers were also low.

### (Investigation with Human iPS Cells (1383D2 Line))

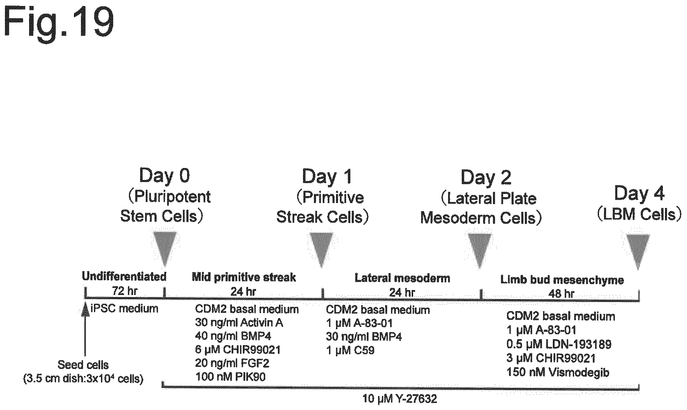

A human iPS cell line (1383D2 line) was treated by the differentiation induction protocol established with the PRRX1 reporter cell line. In Fig. 19, the protocol is illustrated.

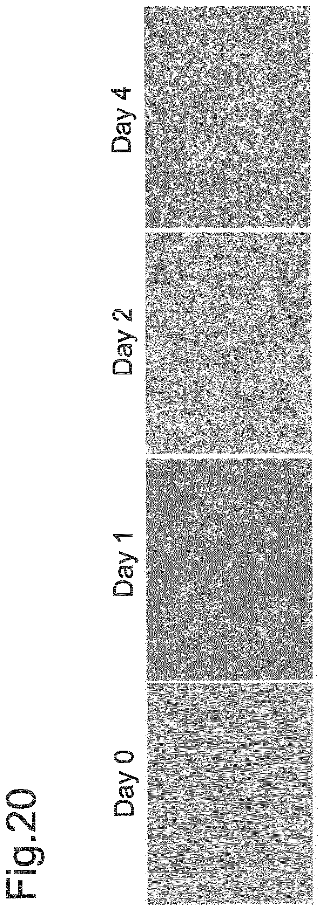

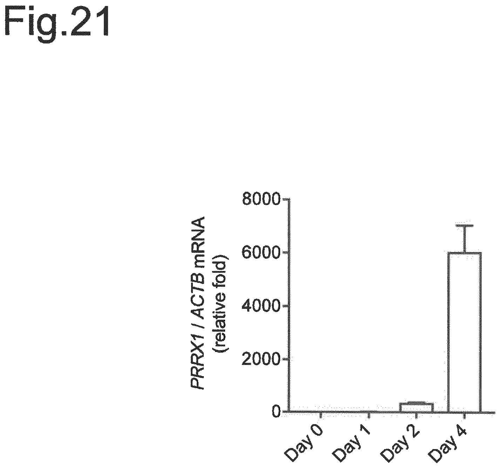

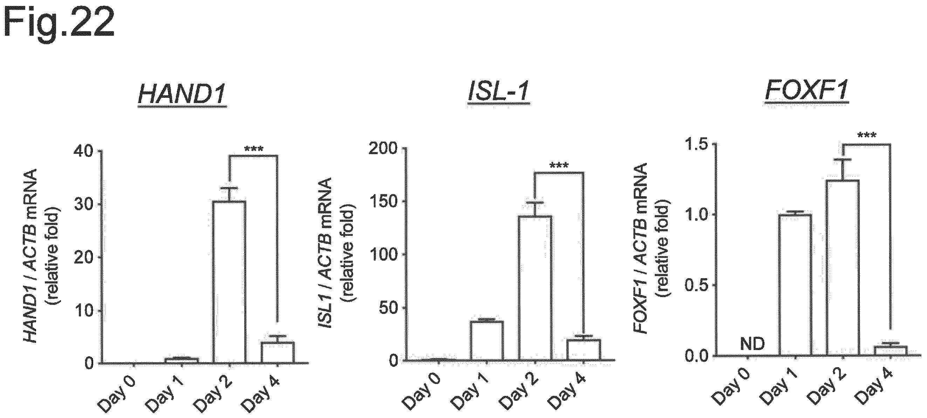

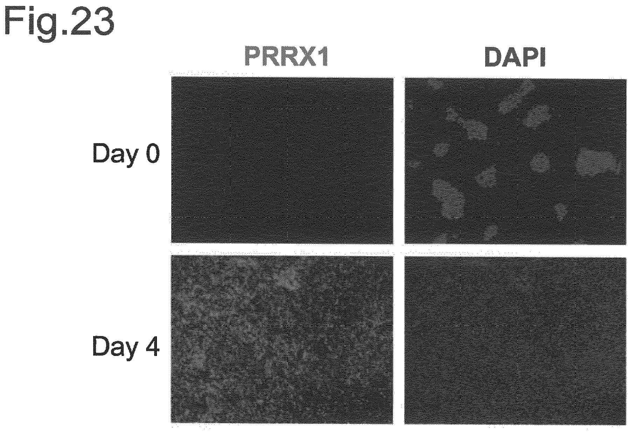

Fig. 20 shows phase-contrast micrographs at different time points, and Fig. 21 shows PRRX1 mRNA expression amounts at different time points. Also in the differentiation induction using the 1383D2 line, an increase in PRRX1 expression was found at the time point of day 4. In addition, Fig. 22 shows the mRNA expression amounts of lateral plate mesoderm markers (HAND1, ISL1, and FOXF1) at different time points. Also in the differentiation induction using the 1383D2 line, the mRNA of each lateral plate mesoderm marker was significantly decreased. Fig. 23 shows the results of immunostaining using a PRRX1 antibody on day 0 and day 4. Most of the cells induced at the time point of day 4 were PRRX1-positive.

### (Investigation with Human ES Cells (SEES4 Line, SEES5 Line, SEES6 Line, and SEES7 Line))

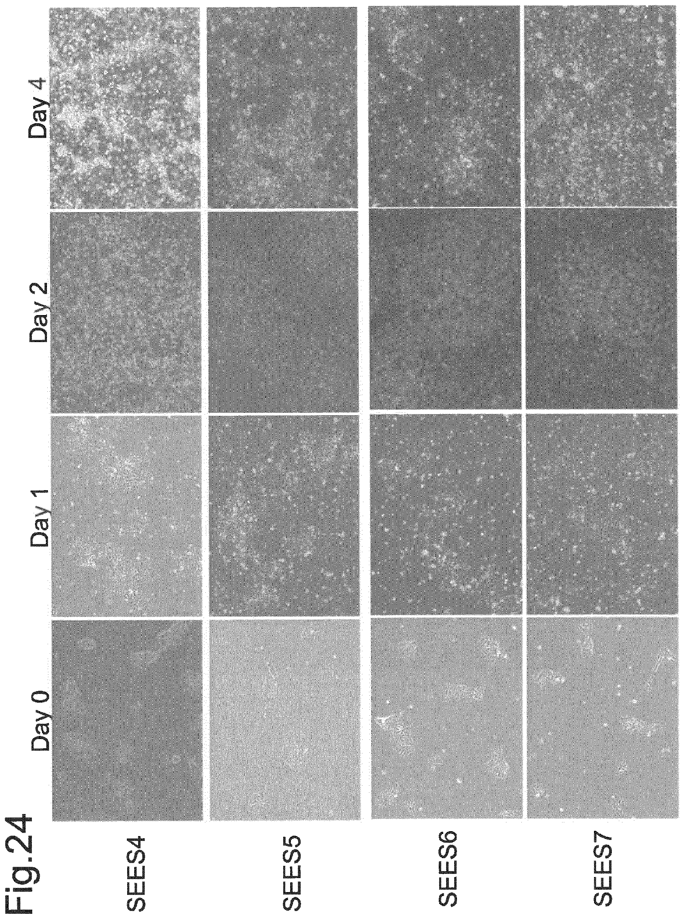

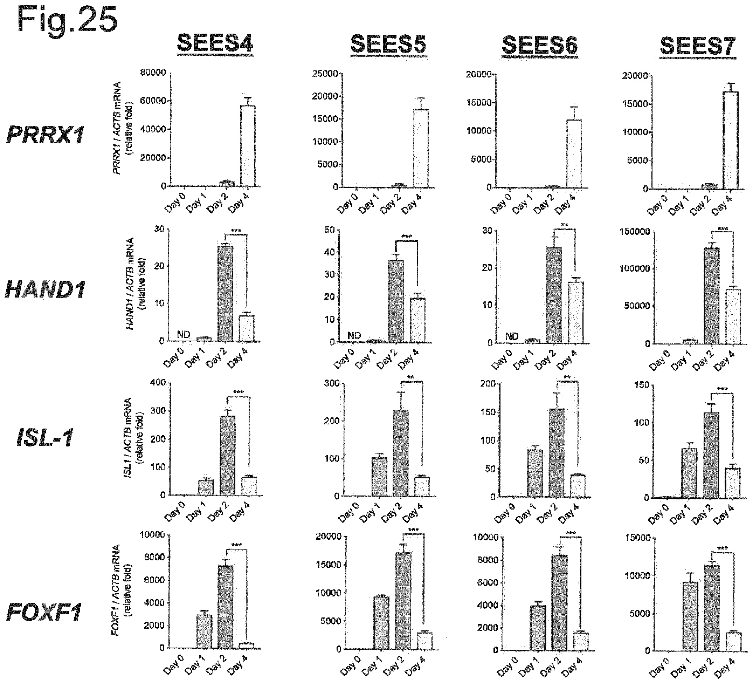

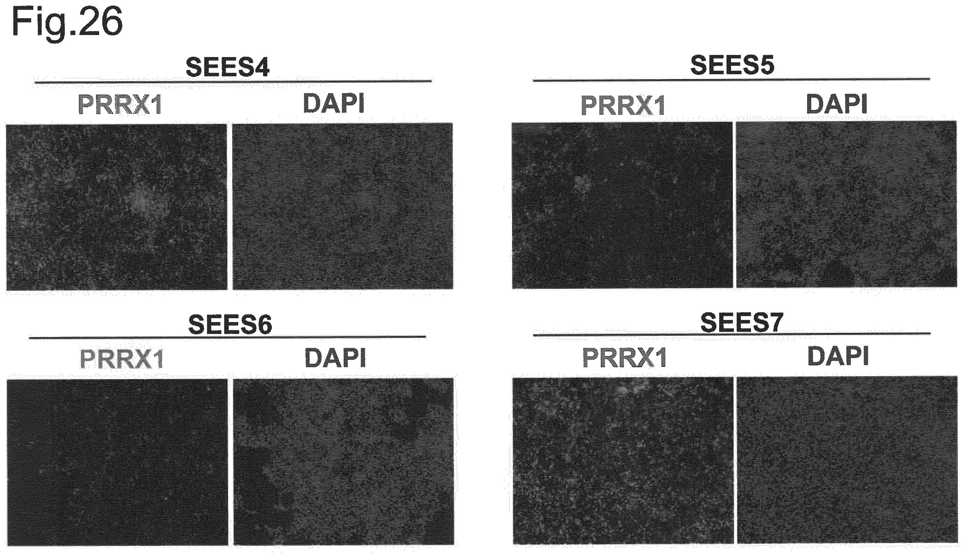

Phase-contrast micrographs at different time points during treatment of human ES cell lines (SEES4 line, SEES5 line, SEES6 line, and SEES7 line) by the differentiation induction protocol established with the PRRX1 reporter cell line are shown in Fig. 24. The mRNA expression amounts of PRRX1 and mesoderm markers (HAND1, ISL1, and FOXF1) at different time points are shown in Fig. 25. Through differentiation induction from day 2 to day 4, the expression of PRRX1 was increased, and the mRNA of each lateral plate mesoderm marker was significantly decreased. The results of immunostaining using a PRRX1 antibody on day 4 are shown in Fig. 26. Most of the cells induced at the time point of day 4 were PRRX1-positive limb bud mesenchymal cells.

### Example 2: Differentiation Induction into PRRX1-positive Cells via Paraxial Mesoderm Lineage

### (Induction of Lateral Plate Mesoderm from iPS Cells via Primitive Streak)

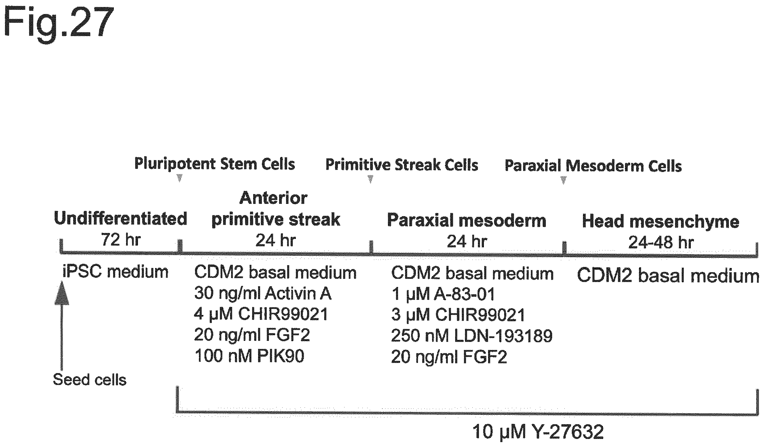

In accordance with a protocol illustrated in Fig. 27, iPS cell lines (414C2 and the PRRX1 reporter cell line) were each induced to differentiate into a primitive streak (anterior-primitive streak, day 1), and then into a paraxial mesoderm (day 2) .

First, the undifferentiated PRRX1 reporter iPS cells obtained in Reference Example 1 were seeded and cultured in a general medium for iPS cells (iPSC medium) for 72 hours (hr) (day 0) .

Then, after being washed with PBS once, the cells were cultured in a medium having the following composition for 24 hours to be induced to differentiate into a paraxial mesoderm.

### • Medium for Primitive Streak Differentiation Induction CDM2 basal medium

30 ng/ml Activin A
4 µM CHIR99021
20 ng/ml FGF2
100 nM PIK90

Then, after being washed with PBS once, the cells were cultured in a medium having the following composition for 24 hours to be induced to differentiate into a paraxial mesoderm.

### • Medium for Paraxial Mesoderm Differentiation Induction CDM2 basal medium

1 µM A-83-01
3 µM CHIR99021
250 nM LDN-193189
20 ng/ml FGF2

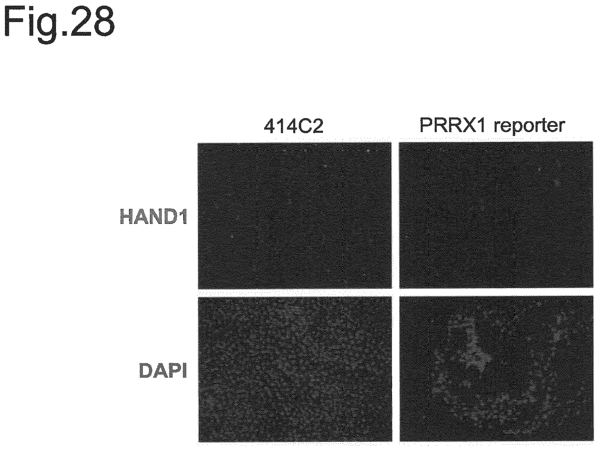

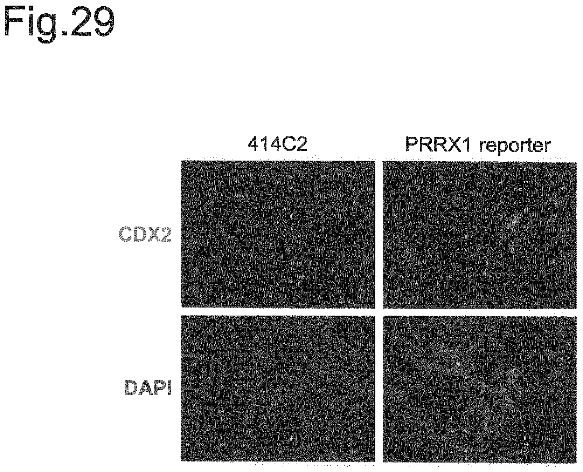

The induced cells were immunostained for HAND1 (lateral plate mesoderm marker) and CDX2 (paraxial mesoderm marker). The results are shown in Fig. 28 and Fig. 29. It was able to be recognized that the induced paraxial mesoderm cells were HAND1-negative and CDX2-positive.

### (Investigation of Differentiation Induction Conditions for PRRX1-positive Cells)

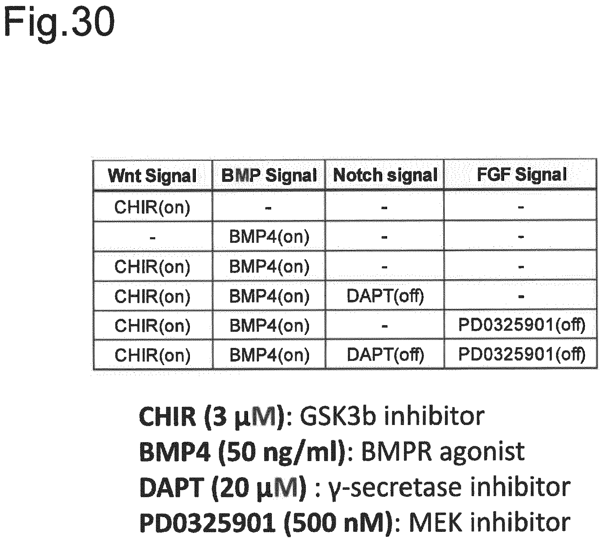

The above-mentioned induced paraxial mesoderm cells (day 2) having the PRRX1 reporter were washed with PBS once, and were then cultured in CDM2 basal media containing activators and/or suppressors for various signals (6 combinations) shown in Fig. 30 for 48 hours.

Reagents used are as described below.
CHIR (CHIR99021, GSK3β inhibitor)
BMP4 (Bone morphogenetic Protein 4)
DAPT (y-Secretase inhibitor)
PD0325901 (MEK inhibitor)

The expression of PRRX1 in the cells after the 48 hours of culture (day 4) was evaluated through observation based on the expression of tdTomato serving as a reporter under a fluorescence microscope.

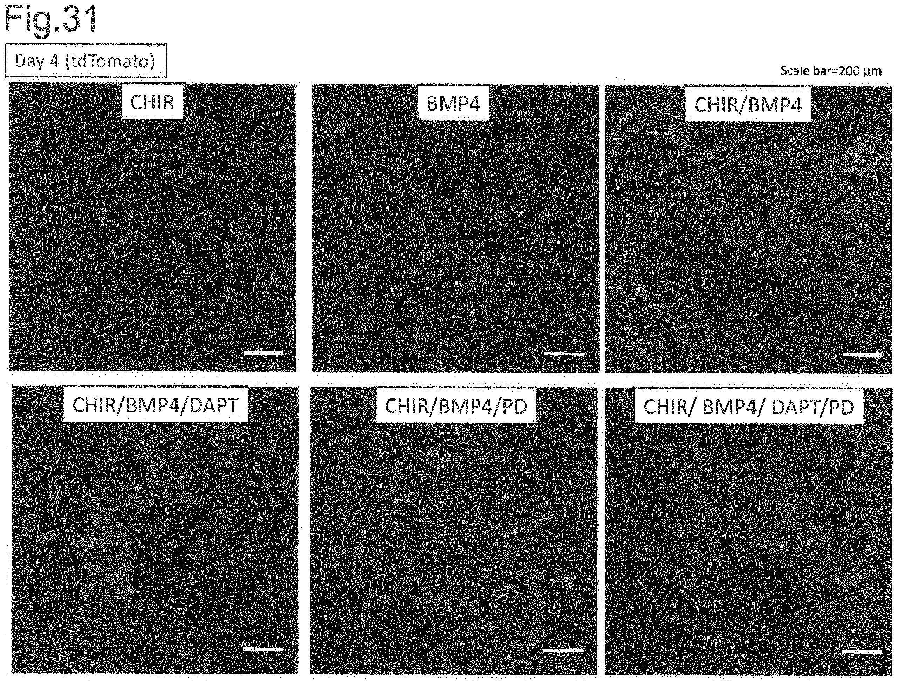

The results are shown in Fig. 31. Particularly high expressions of tdTomato were found in four kinds of combinations, i.e., the combinations CHIR/BMP4, CHIR/BMP4/DAPT, CHIR/BMP4/PD, CHIR/BMP4/DAPT/PD.

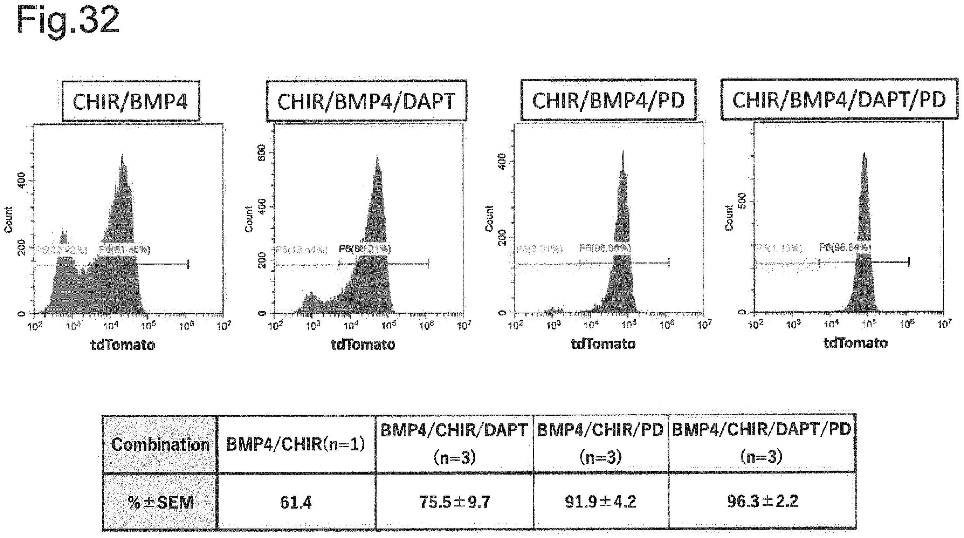

Treatment was performed with each of the four kinds, i.e., CHIR/BMP4, CHIR/BMP4/DAPT, CHIR/BMP4/PD, and CHIR/BMP4/DAPT/PD, and 48 hours later (day 4), the efficiency of induction into tdTomato-positive cells was investigated by flow cytometry. The results are shown in Fig. 32. The efficiency of induction into tdTomato-positive cells was found to be as follows: CHIR/BMP4 (61%), CHIR/BMP4/DAPT (75%), CHIR/BMP4/PD (91%), CHIR/BMP4/DAPT/PD (96%).

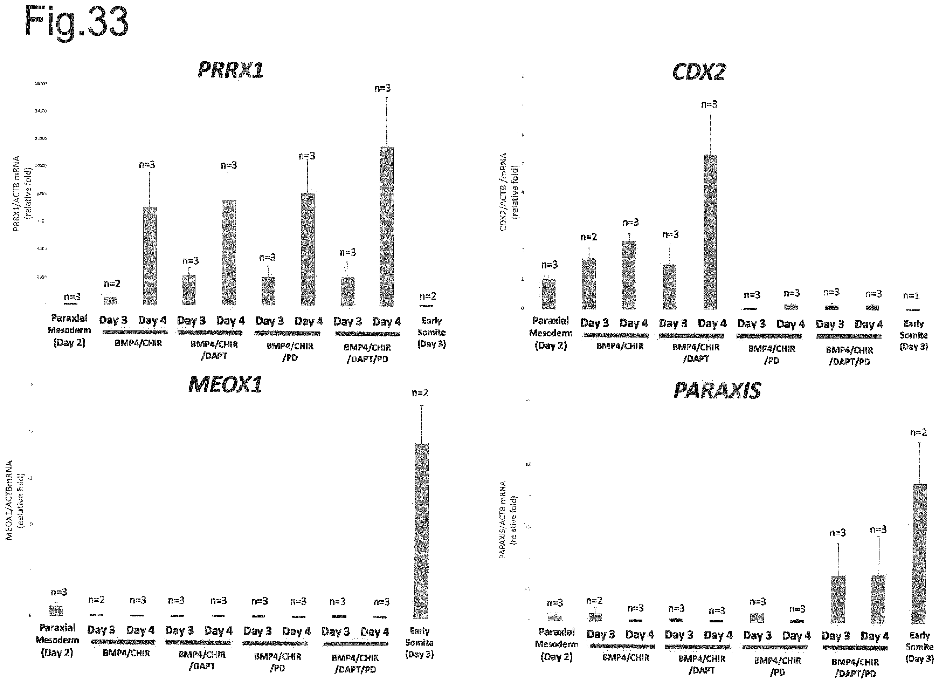

Treatment was performed with each of the four kinds, i.e., CHIR/BMP4, CHIR/BMP4/DAPT, CHIR/BMP4/PD, and CHIR/BMP4/DAPT/PD, and 24 hours later (day 3) or 48 hours later (day 4), RNA was collected in each case and subjected to qPCR for the measurement of the expressions of PRRX1, a paraxial mesoderm marker (CDX2), and somite markers (MEOX1 and PARAXIS). The results are shown in Fig. 33. It was found that the expression of PRRX1 was increased in each of the combinations, but the combination of CHIR/BMP4/PD had low expression amounts of both the paraxial mesoderm marker and the somite markers.

### Example 3: Differentiation Induction from Limb Bud Mesenchymal Cells into RUNX2-positive Osteoblast Progenitor Cells

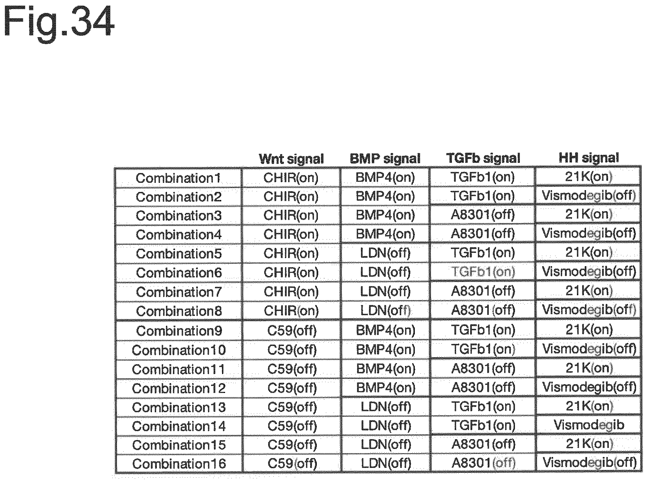

Limb bud mesenchymal cells were obtained in conformity with the method described in Example 1. Then, the obtained cells were treated with activators or suppressors for various signals shown in Fig. 34 to investigate conditions for induction into RUNX2-positive osteoblast progenitor cells (16 combinations).
CHIR (CHIR99021, GSK3b inhibitor)
C59 (Wnt-C59, PORCN inhibitor)
BMP4 (Bone morphogenetic Protein 4)
LDN (LDN193189, ALK2/ALK3 inhibitor)
TGFβ1 (Tumor growth factor β1)
A8301 (A83-01, ALK5 inhibitor)
21K (Hedgehog agonist)
Vismodegib (Hedgehog inhibitor)

The limb bud mesenchymal cells (day 4, limb bud mesenchyme) were treated with the 16 combinations, and 48 hours later (day 6), RNA was collected in each case and subjected to qPCR for the measurement of the expression of RUNX2 (essential transcriptional regulator in osteoblast differentiation).

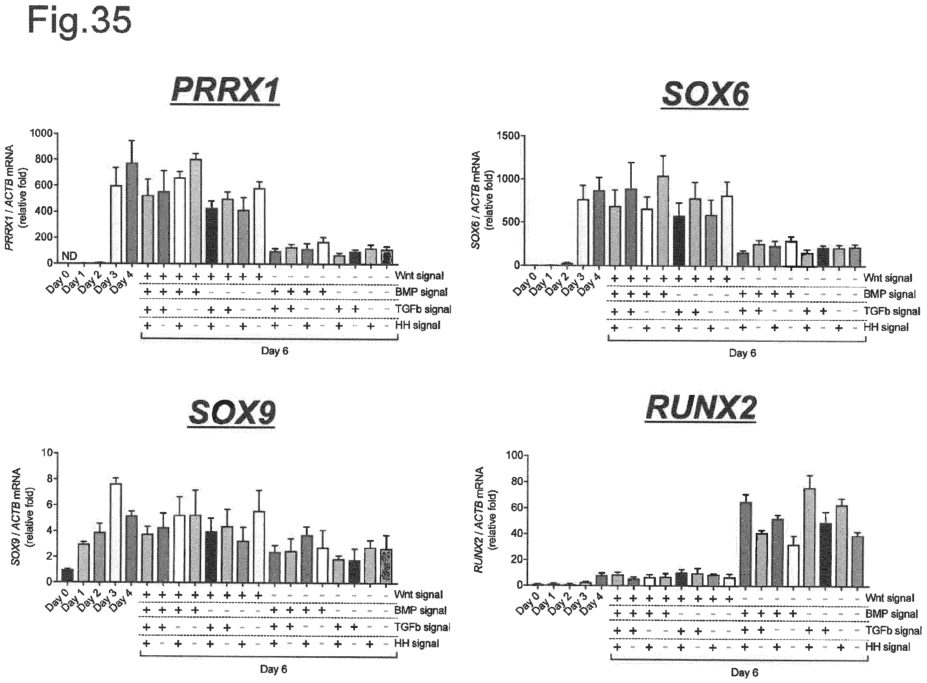

The results are shown in Fig. 35. In each of Combinations 9, 10, 11, 12, 13, 14, 15, and 16, a high expression of RUNX2 was found. Of those, Combination 13 showed a particularly high RUNX2 expression as compared to the other groups.

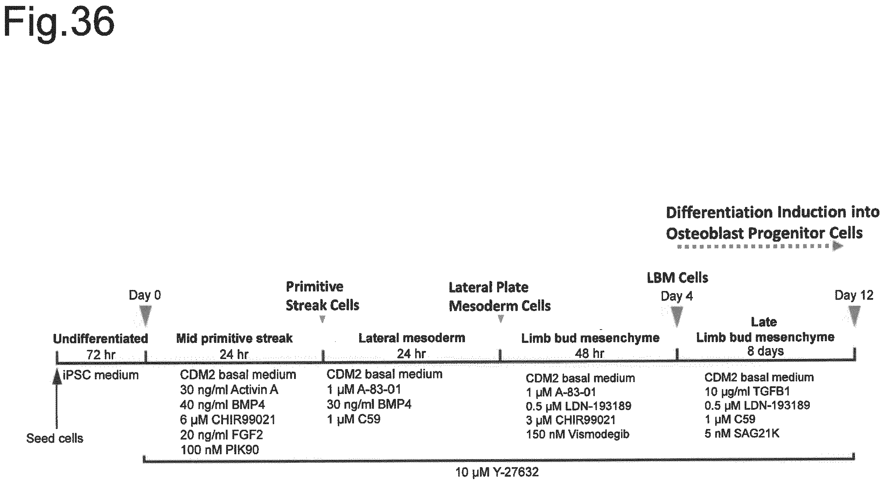

On the basis of the above-mentioned results, Combination 13 was selected as a particularly effective condition for inducing RUNX2-positive osteoblast progenitor cells from limb bud mesenchymal cells (Fig. 36).

RNA was collected with time during the stage starting with the limb bud mesenchymal cells (day 4) up to day 12, and was subjected to qPCR for the measurement of the expressions of various genes (PRRX1, RUNX2, SP7, COL1A1, and bone sialoprotein (BSP)).

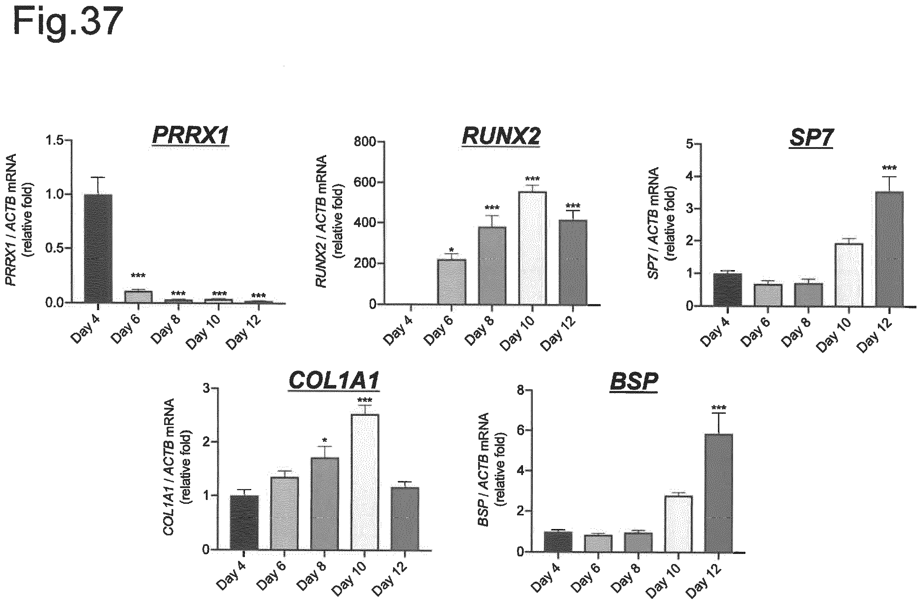

The results are shown in Fig. 37. It was revealed that, when the limb bud mesenchymal cells were induced into RUNX2-positive osteoblast progenitor cells, the expression of PRRX1 was decreased with time, and besides, the RUNX2 expression and the expressions of other osteoblast differentiation markers (SP7, COL1A1, and BSP) were increased.

The 414C2 line, the PRRX1 reporter cell line, the 1383D2 line, the SEES4 line, and the SEES7 line were each induced into limb bud mesenchymal cells (day 4, limb bud mesenchyme), and then treated with Combination 13, and 8 days later (day 12), RNA was collected and subjected to qPCR for the measurement of the expression of RUNX2.

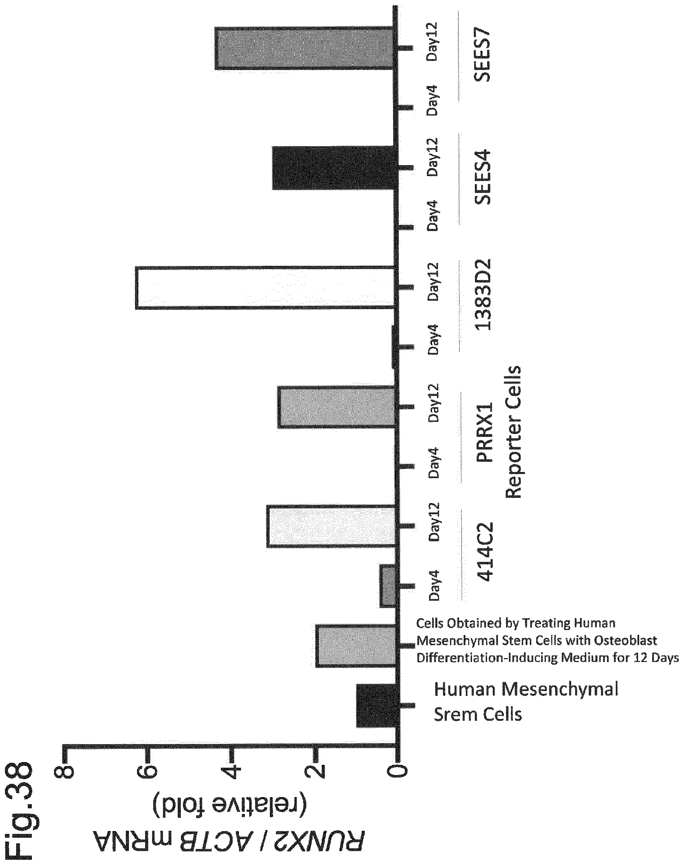

The results are shown in Fig. 38. It was revealed that the limb bud mesenchymal cells derived from each of the cell lines were markedly increased in RUNX2 expression through the treatment with Combination 13.

The PRRX1 reporter cell line, the 1383D2 line, and the SEES4 line were each subjected to cell immunostaining using a RUNX2 antibody at the time points of day 4 and day 12.

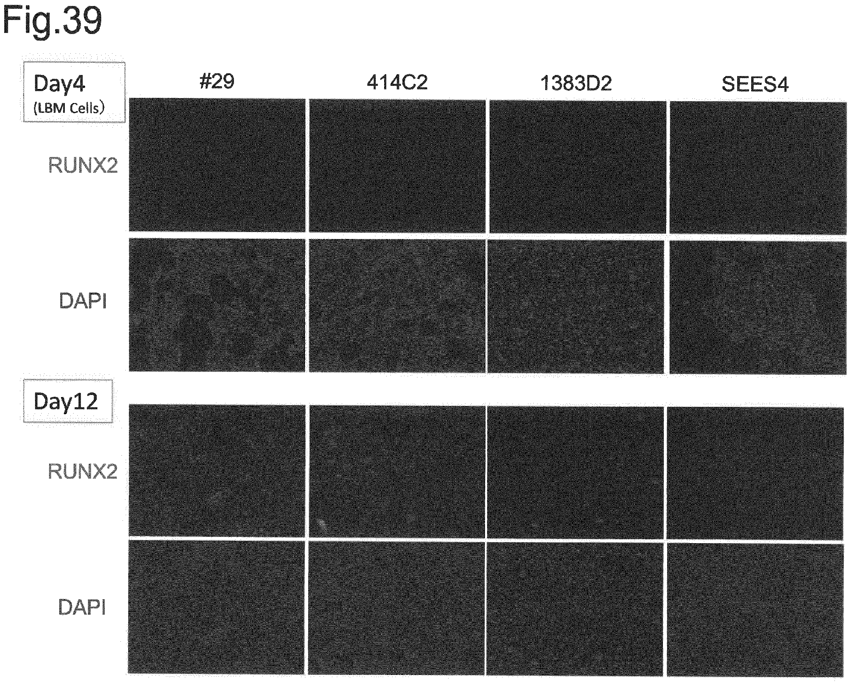

The results are shown in Fig. 39. The expression of RUNX2 was not observed on day 4, but most cells were RUNX2-positive on day 12.

Further, the 414C2 line, the PRRX1 reporter cell line, the SEES4 line, and the SEES7 line were each subjected to flow cytometry analysis using a RUNX2 antibody at the time point of day 12.

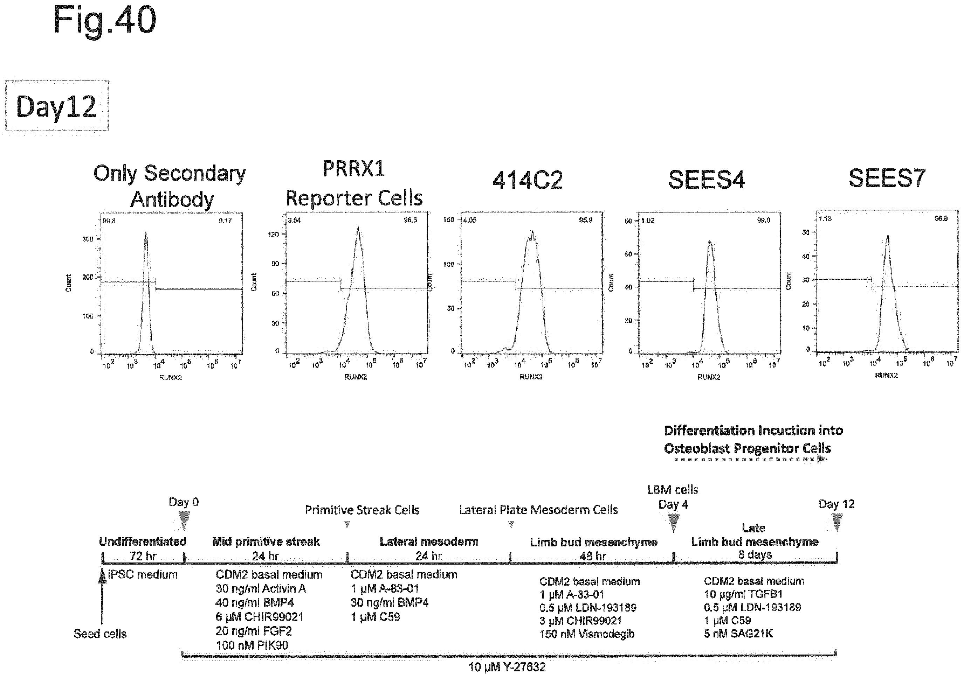

The results are shown in Fig. 40. 95% or more of the cells induced in each line were RUNX2-positive.

The PRRX1 reporter cell line was induced into limb bud mesenchymal cells (day 4, limb bud mesenchyme), and then the cells were treated with Combination 13 and cultured for 8 days. In order to investigate whether the cells at the time point of day 12 had an osteoblast differentiation ability, culture was performed in an osteoblast-inducing medium for 12 days, followed by alizarin red staining.

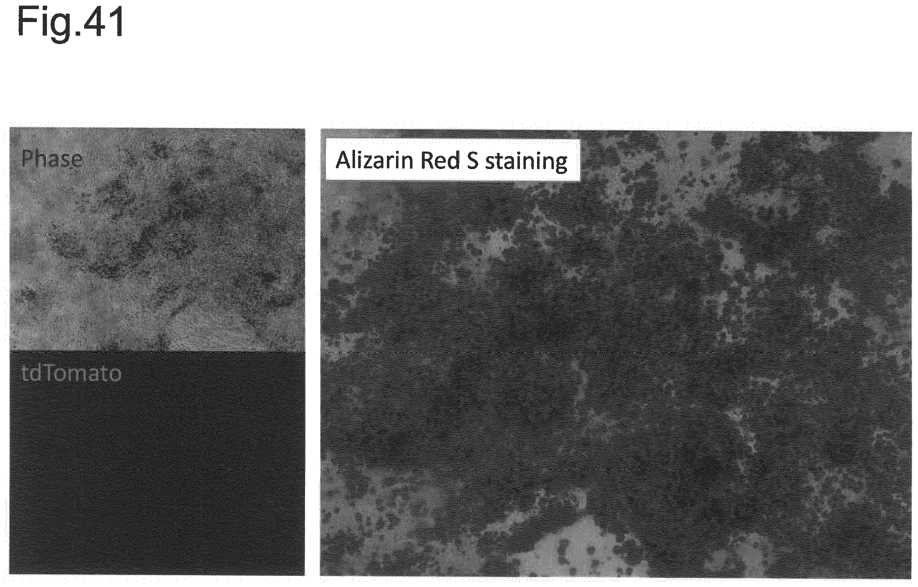

The results are shown in Fig. 41. The induced cells had significantly increased stainability with alizarin red.

### Example 4: Method of Inducing LBMs into hCPCs through Passage Using Stem Medium 3 (hCPC Medium) and Passage Culture Method for hCPCs Using Stem Medium 3

The PRRX1 reporter cell line obtained in Reference Example 1 was used to prepare lateral plate mesoderm-derived PRRX1-positive cells (day 4, limb bud mesenchyme) in accordance with the differentiation induction conditions of Example 1. The conditions of a method for the expansion culture of the cells were investigated.

Through use of Accutase, the lateral plate mesoderm-derived PRRX1-positive cells on day 4 were collected, and the collected cells were subjected to passage culture in culture media having various compositions (stem media 1, 2, and 3). As a result, a satisfactory result was obtained when the stem medium 3 (CDM2 basal medium+CHIR+A8301+EGF+FGF) was supplemented with Y-27632.

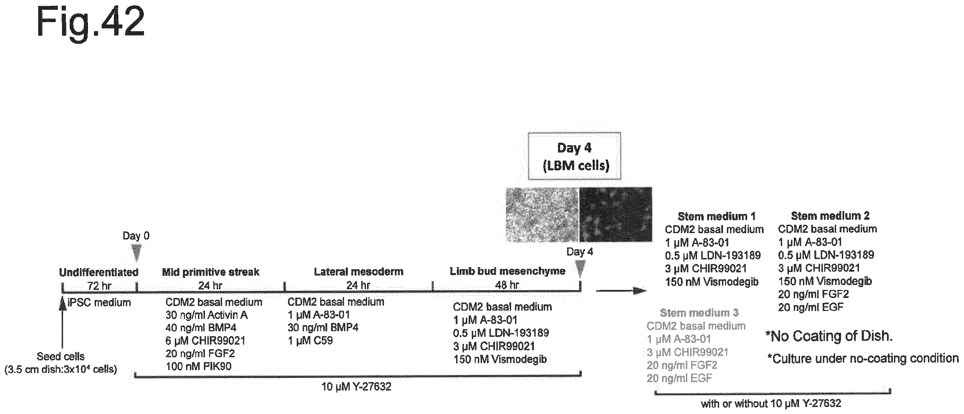

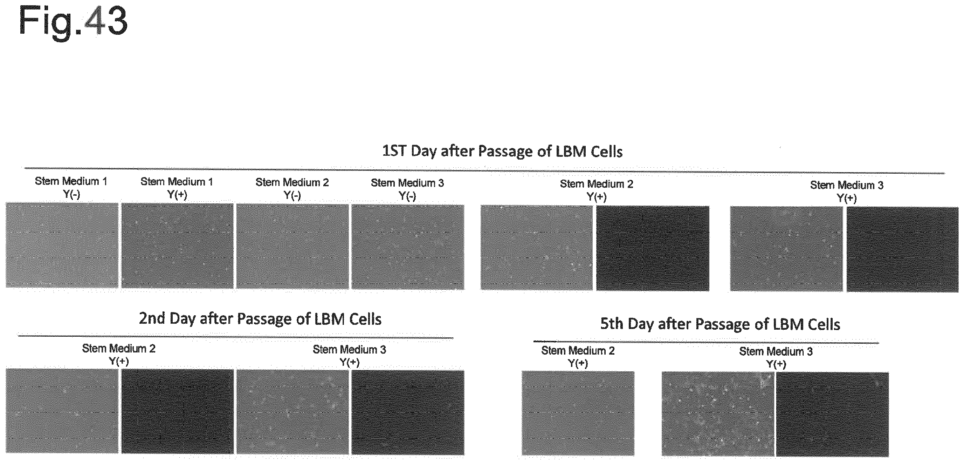

The protocol is illustrated in Fig. 42, and phase-contrast images under different conditions are shown in Fig. 43.

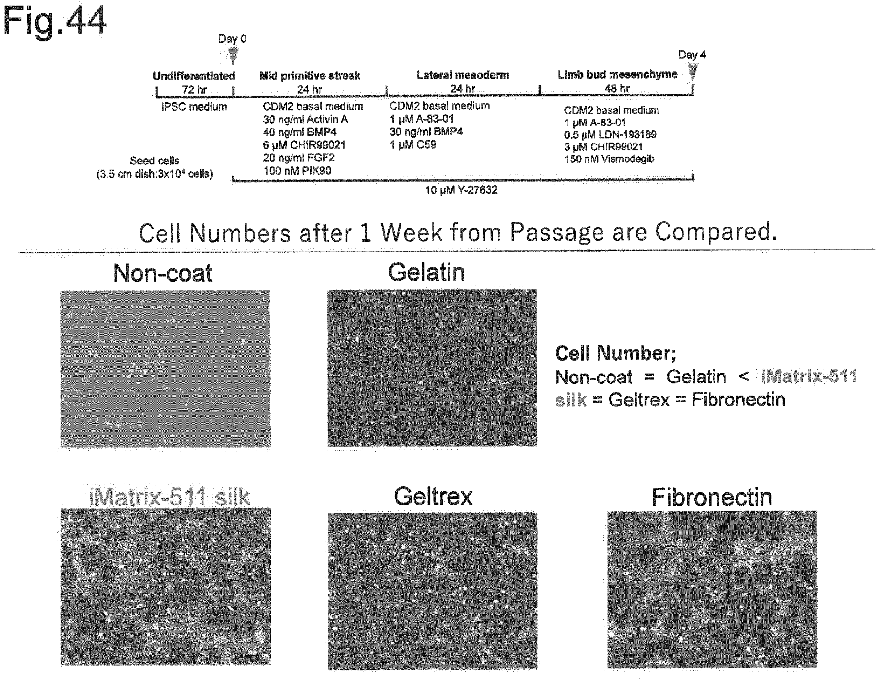

In addition, the kind of a coating agent for a dish was investigated. Cell numbers after 1 week from passage were compared and found to be as follows: Non-coat = Gelatin < iMatrix-511 silk = Geltrex = Fibronectin. Coating with iMatrix-511, which was the same coating agent as that used at the time of differentiation induction and had a clinical grade, was selected, and used in the following experiment (Fig. 44).

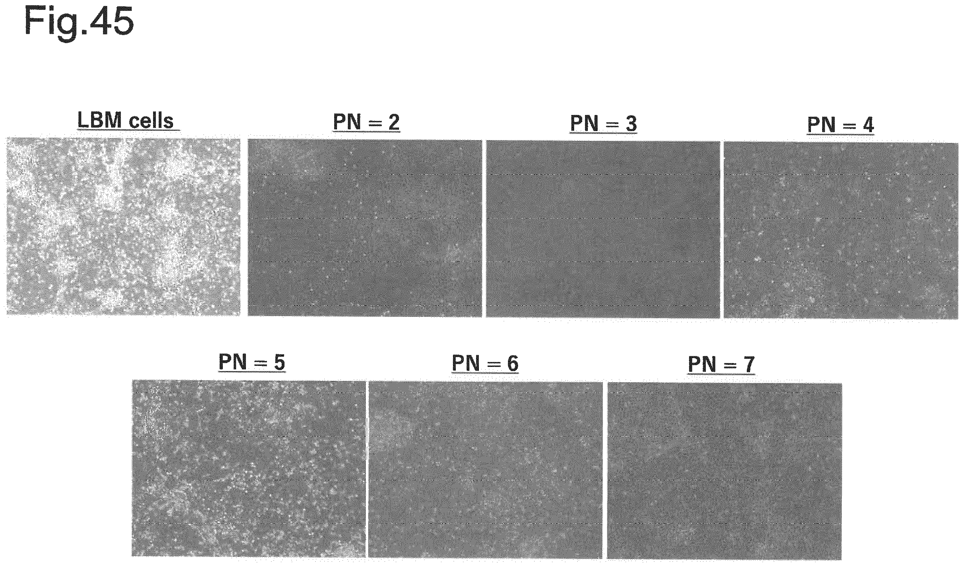

Fig. 45 shows phase-contrast micrographs at the time points of different passage numbers during passage culture under the above-mentioned conditions. Passage was able to be performed under a state of maintaining the morphology of limb bud mesenchymal cells.

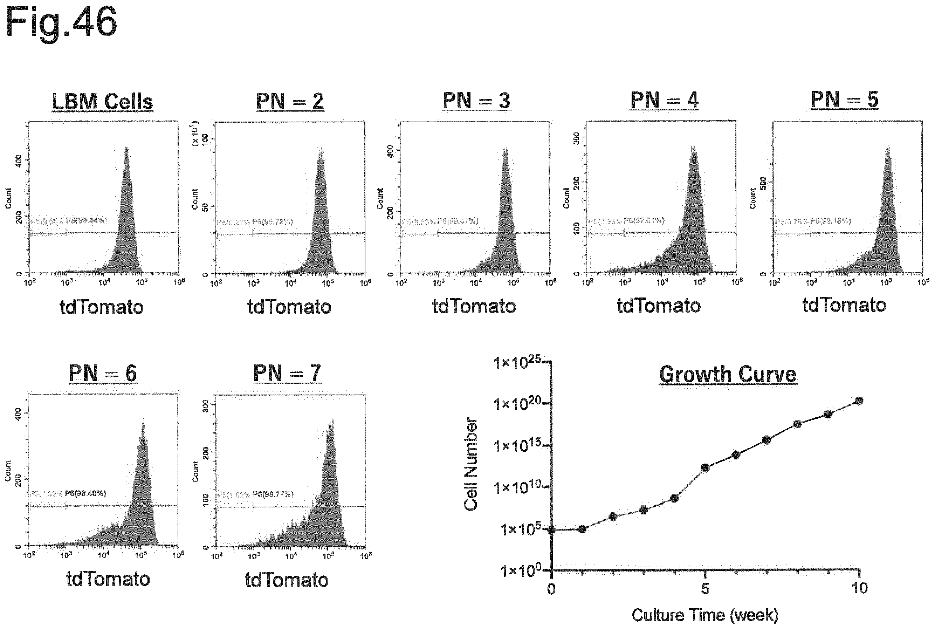

Fig. 46 shows flow cytometry analysis results of tdTomato at different passage numbers during the passage culture, and a growth curve. It was revealed that passage was able to be performed under a state of retaining a tdTomato(PRRX1)-positive state.

### Example 5: Properties (Growth Properties, Karyotype, and Preservation in Liquid Nitrogen) of hCPCs Induced from Limb Bud Mesenchymal Cells

Human chondrocyte progenitor cells (CPCs) were generated from a healthy individual-derived iPSC line (414C2, 1383D2, PRRX1 reporter, HPS1042, or HPS1043) or a human ES cell line (SEES4, SEES6, or SEES7).

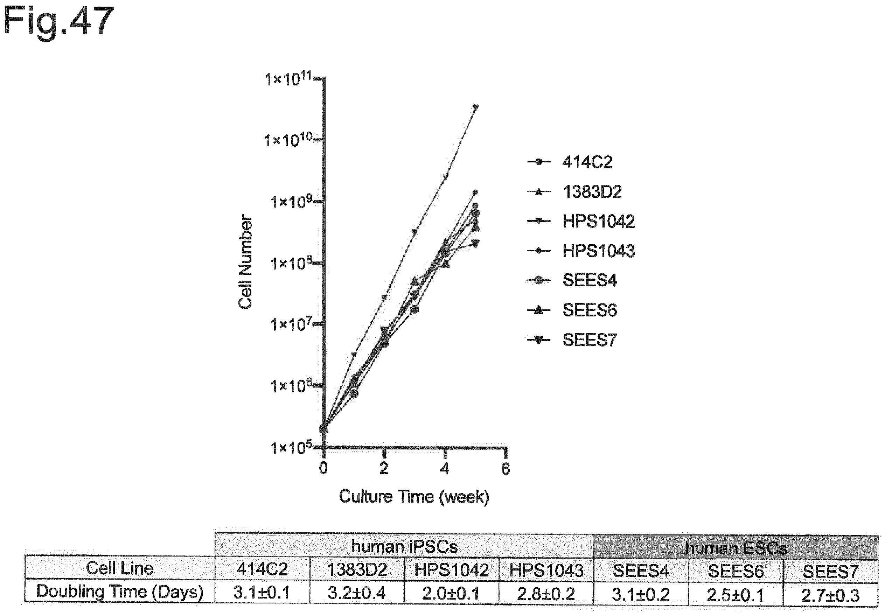

Human LBMs were generated from a healthy individual-derived iPSC line (414C2, 1383D2, HPS1042, or HPS1043), the PRRX1 reporter iPSC line (Reporter), or a human ES cell line (SEES6), and further, human chondrocyte progenitor cells (hCPCs) were generated from the human LBMs. Cell numbers at the time of the generation of the hCPCs from the human LBMs are shown in Fig. 47. It was revealed that the hCPCs had a doubling time of from about 2 days to about 3 days.

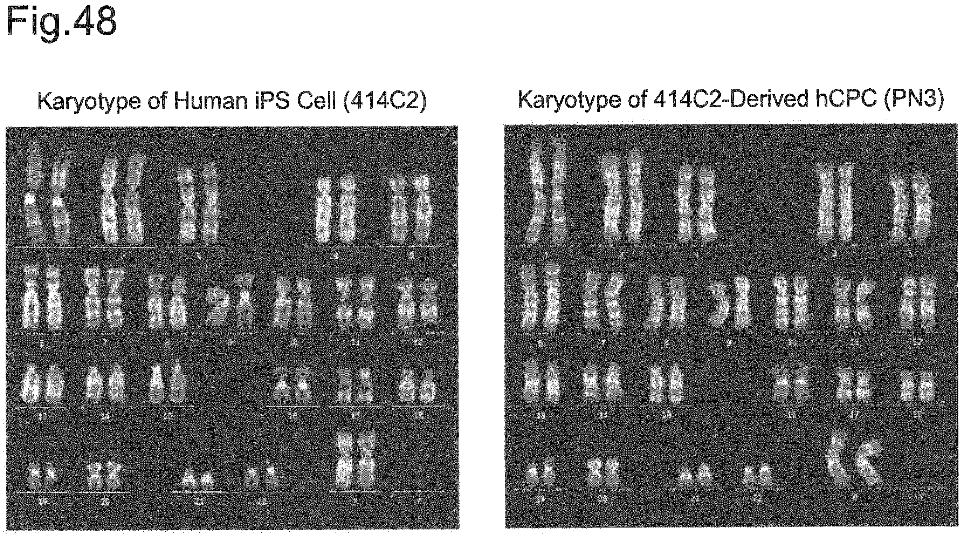

In addition, hCPCs (PN3) was generated from human iPS cells (414C2), and the karyotypes of the human iPS cells (414C2) (Fig. 48(a)) and the hCPCs (PN3) (Fig. 48(b)) were investigated. Fig. 48(a) and Fig. 48(b) revealed that the karyotype was unchanged in the course of induction from the iPS cells to the hCPCs.

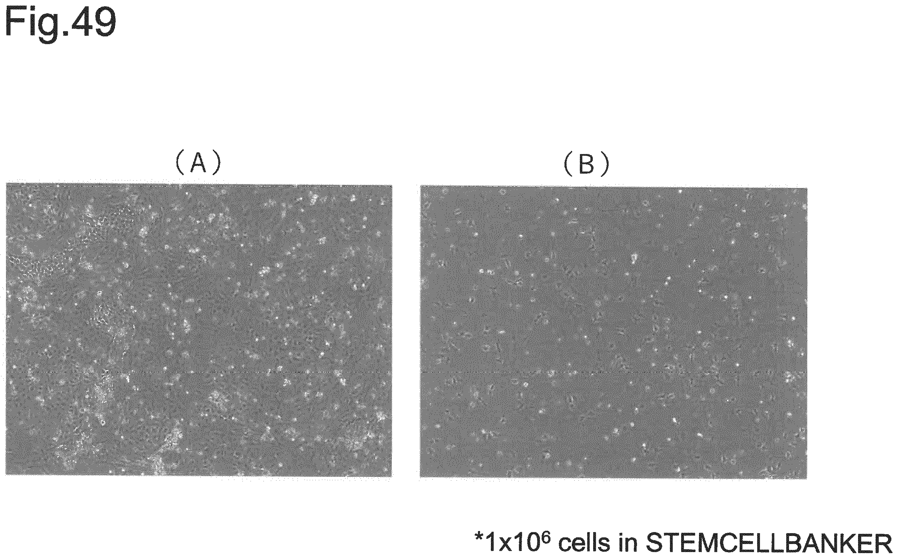

Further, it was revealed that the limb bud mesenchymal cells subjected to passage culture were capable of being preserved in liquid nitrogen with STEM CELL BANKER, and the preserved cells were capable of passage culture by being reseeded after thawing (Fig. 49(a) and Fig. 49(b)).
Example 6: Comparison of expression amounts of RUNX2 (marker gene for osteoblast progenitor cells) when LBMs (day 4) or hCPCs are cultured in RUNX2-positive osteoblast progenitor cell differentiation-inducing medium.

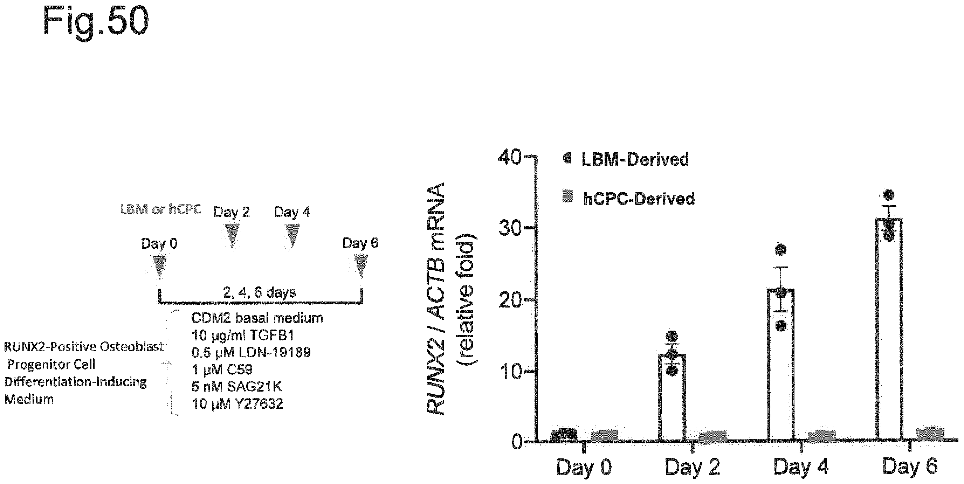

Limb bud mesenchymal cells (LBMs) (day 4) and hCPCs were treated with Combination 13 (RUNX2-positive osteoblast progenitor cell differentiation-inducing medium: The composition is described in Fig. 50), and RNA was collected with time. As a result, the expression of RUNX2 was increased with time in the LBMs (day 4), but such change was not found in the hCPCs (Fig. 50). The hCPCs, which were obtained by subjecting the LBMs (day 4) to expansion culture, were different in differentiation induction capacity from RUNX2-positive osteoblast progenitor cells. The hCPCs had no ability to differentiate into RUNX2-positive osteoblast progenitor cells, and were oriented toward cartilage differentiation, revealing that the hCPCs and the LBMs (day 4) were different from each other.

### • RUNX2 Measurement Conditions

RNA was collected from cells cultured for each number of days and converted into cDNA, and then the mRNA expressions of RUNX2 and ACTB (internal standard) were measured by a real-time PCR method (primers used in this case are shown in Table 1.). After the measurement, the RUNX2 expression amount was corrected with the expression amount of ACTB.

**Table 1**

| Gene | Forward primer sequence | Reverse primer sequence |
|---|---|---|
| RUNX2 | TCAACGATCTGAGATTTGTGGG | GGGGAGGATTTGTGAAGACGG |
| ACTB | AGAAAATCTGGCACCACACC | AGAGGCGTACAGGGATAGCA |

### Example 7

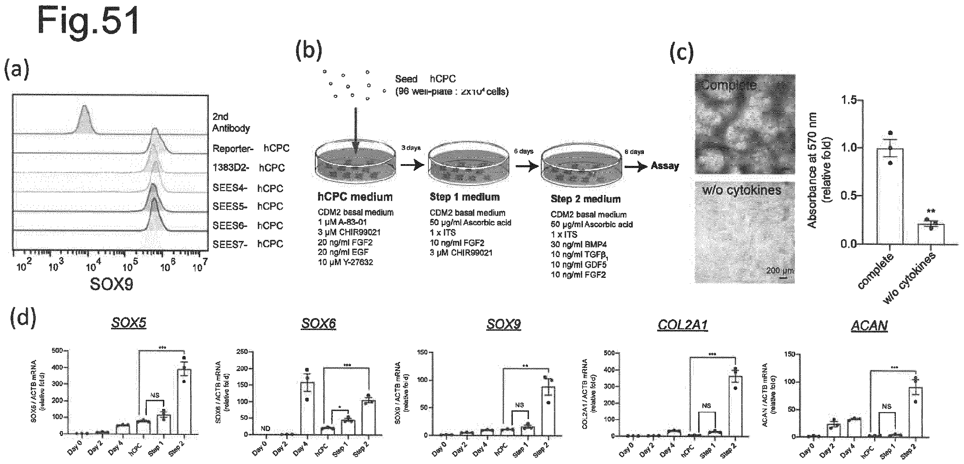

iPS cells (day 0), lateral plate mesoderm cells (day 2), LBMs (day 4), and hCPCs were treated with Step 1 medium and Step 2 medium illustrated in Fig. 51(b). For each type of the treated cells, RNA was collected, and the expressions of chondrocyte markers (SOX5, SOX6, SOX9, COL2A1, and ACAN) were investigated. As a result, it was revealed that the chondrocyte markers were markedly increased in the cells treated with Step 2 medium (Fig. 51(d)).

### Example 8: Staining of Hyaline Cartilage-like Tissue Body

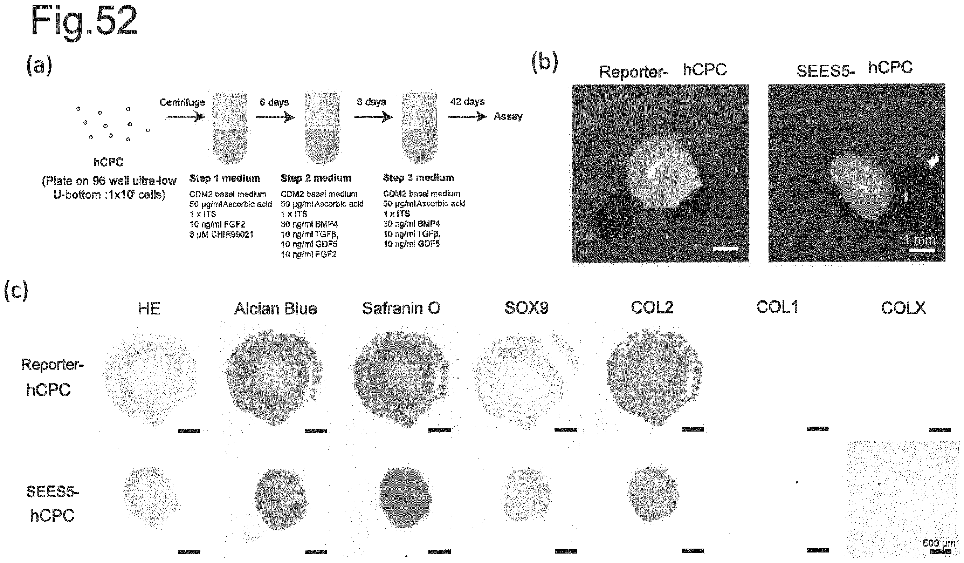

Human chondrocyte progenitor cells (hCPC) were generated from the PRRX1 reporter iPSC line (Reporter) or the human ES cell line (SEES5). The cells were collected from their culture dish, and seeded at 100,000 cells/well into an ultra-low U-bottom 96-well plate, followed by a centrifugal operation to generate aggregates on the wells. After that, the cells were treated by a protocol illustrated in Fig. 52(a), and sections were prepared from hyaline cartilage tissue bodies (Fig. 52(b)) on the 42nd day of the treatment of step 3, and were subjected to HE staining, Alcian Blue staining, Safranin O staining, and immunostaining of chondrocyte markers (SOX9 and COL2), a hypertrophic chondrocyte marker (COLX), and a fibrocartilage marker (COL1) (Fig. 52(c)). The results of Fig. 52(b) and Fig. 52(c) revealed that hyaline cartilage tissues were obtained.

### Example 9: Preparation of Cartilage Tissue Using LBMs or hCPCs

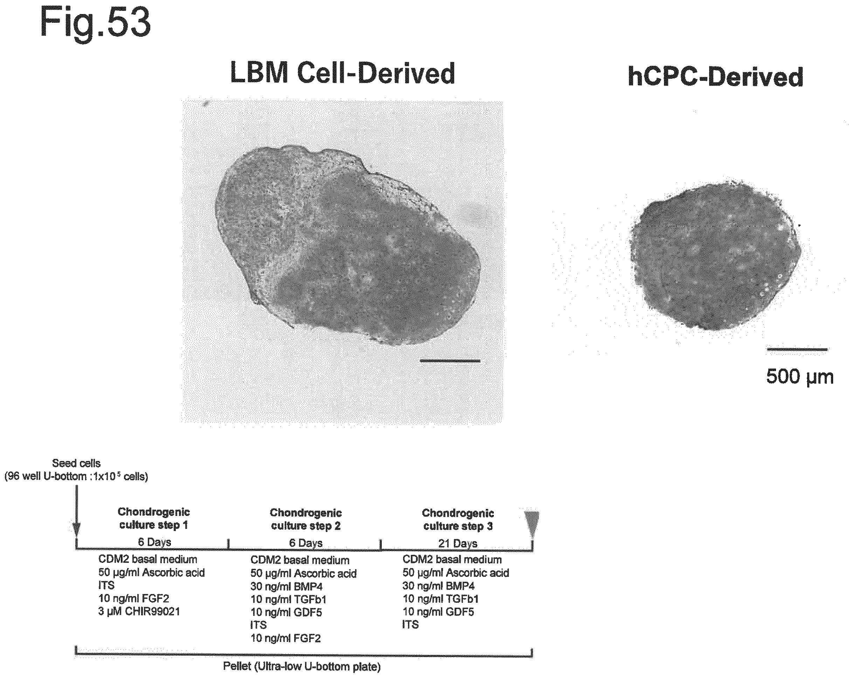

A hyaline cartilage tissue was induced from LBMs or hCPCs under conditions illustrated in Fig. 53, and was subjected to Safranin O staining. The results are shown in Fig. 53. It was revealed that the efficiencies of induction into LBM-derived and hCPC-derived hyaline cartilage bodies differed from each other.

### Example 10: Subcutaneous Transplantation of Hyaline Cartilage-like Tissue Body into NOD-SCID Mouse

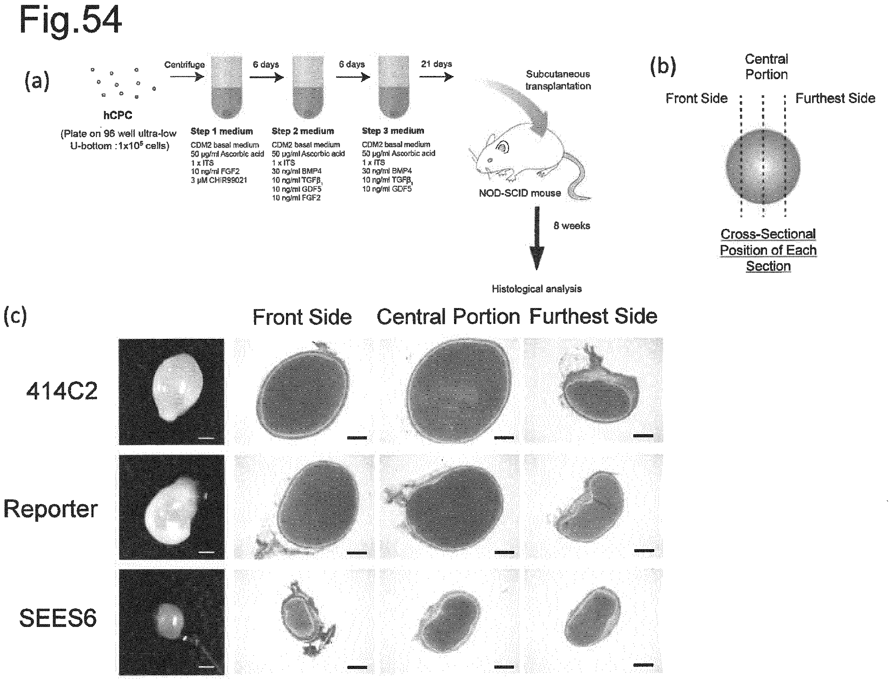

Human chondrocyte progenitor cells (hCPC) were generated from the healthy individual-derived iPSC line (414C2), the PRRX1 reporter iPSC line (Reporter), or the human ES cell line (SEES6). The cells were collected from their culture dish, and seeded at 100,000 cells/well into an ultra-low U-bottom 96-well plate, followed by a centrifugal operation to generate aggregates on the wells. After that, the cells were treated by a protocol illustrated in Fig. 54(a), and an aggregate on the 21st day of the treatment of step 3 was subcutaneously transplanted into a NOD-SCID mouse. Sections were prepared from a hyaline cartilage tissue body obtained in the 8th week after the transplantation (front side, central portion, furthest side, Fig. 54(b)), and were subjected to Safranin O (SafO) staining by which a site in which a cartilage matrix was present was stained red. The results found that a uniform hyaline cartilage tissue body was formed without forming a tumor (Fig. 54(c)).

Example 11: Experiment of Transplantation of Human-derived Cartilage Tissue Mass into Cartilage Defective Site of SCID Rat

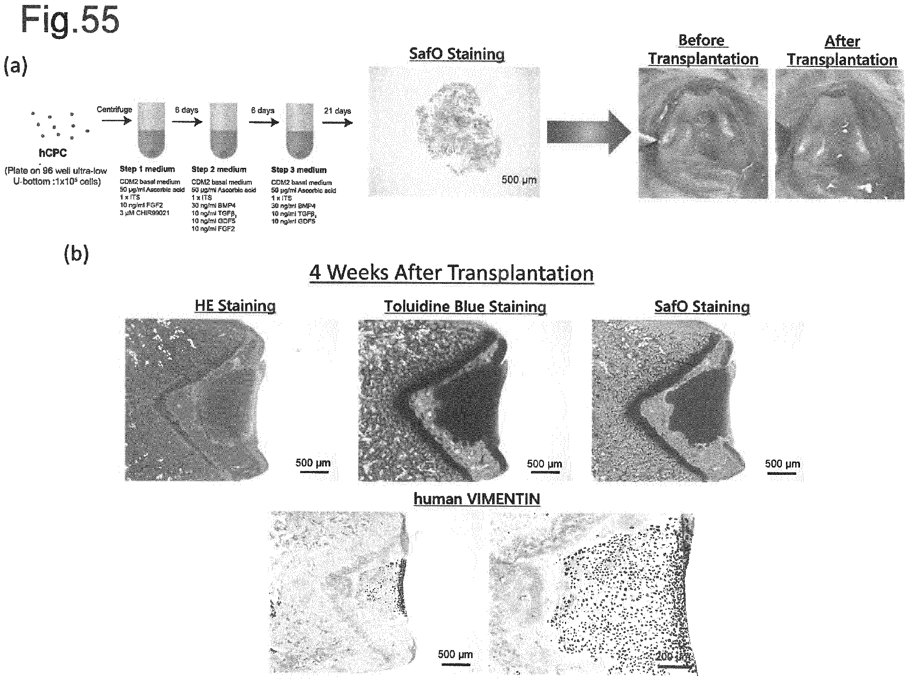

Human chondrocyte progenitor cells (hCPC) were generated from the PRRX1 reporter iPSC line (Reporter). The cells were collected from their culture dish, and seeded at 100,000 cells/well into an ultra-low U-bottom 96-well plate, followed by a centrifugal operation to generate aggregates on the wells. After that, the cells were treated by a protocol illustrated in Fig. 55(a), and an aggregate on the 21st day of the treatment of step 3 was subjected to SafO staining to be recognized as Safranin-positive. The knee joint cartilage of an SCID rat was bored with a biopsy trephine (1 mm), and the cartilage tissue mass (the above-mentioned aggregate) was transplanted. After 4 weeks from the transplantation, SafO staining, toluidine blue staining, and immunostaining using a human vimentin-specific antibody were performed. Engraftment of the human-derived cartilage tissue mass into the cartilage defective site of the SCID mouse was recognized (Fig. 55(b)).

### Example 12: Subrenal Capsule Transplantation of hCPC Cells into NOD-SCID Mouse

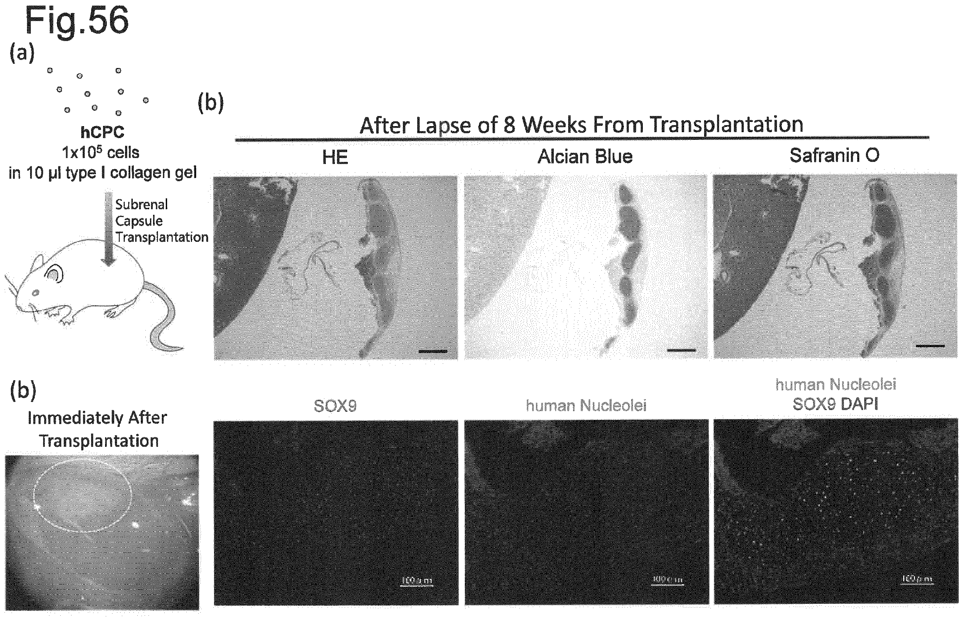

Human chondrocyte progenitor cells (hCPCs) were generated from the PRRX1 reporter iPSC line. 1×10⁵ hCPC cells were suspended in 10 µl of type I collagen gel, and the suspension was transplanted into the subrenal capsule of a NOD-SCID mouse (Fig. 56(a)). The results of staining (HE, Alcian Blue, Safranin O), SOX9 expression, and human nuclear staining (human Nucleolei) after 8 weeks are shown in Fig. 56(b). It was recognized that mere transplantation of the cell suspension of the hCPCs into an adult resulted in the formation of a cartilage tissue at the transplantation site.

### Example 13: Generation of Cartilage Plate

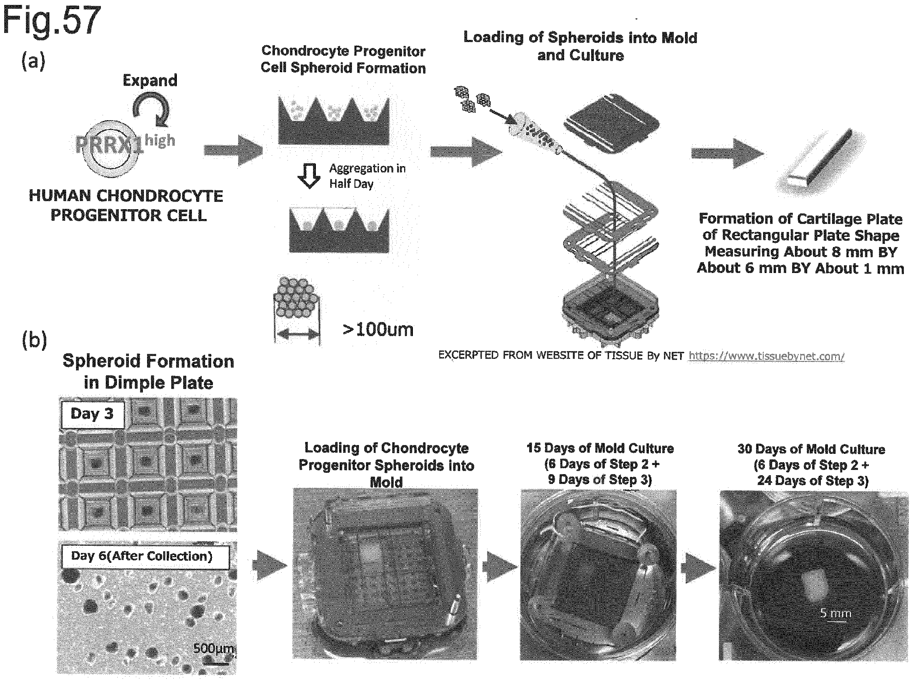

Spheroids of chondrocyte progenitor cells were formed using a dimple plate from human chondrocyte progenitor cells subjected to quality control (Fig. 57(a)), and the spheroids were loaded into a mold and cultured. Thus, a plate-like cartilage tissue larger than a related-art cartilage tissue ball was successfully formed (Fig. 57(b)).

The resultant plate-like cartilage had such a size as to be usable for human transplantation.

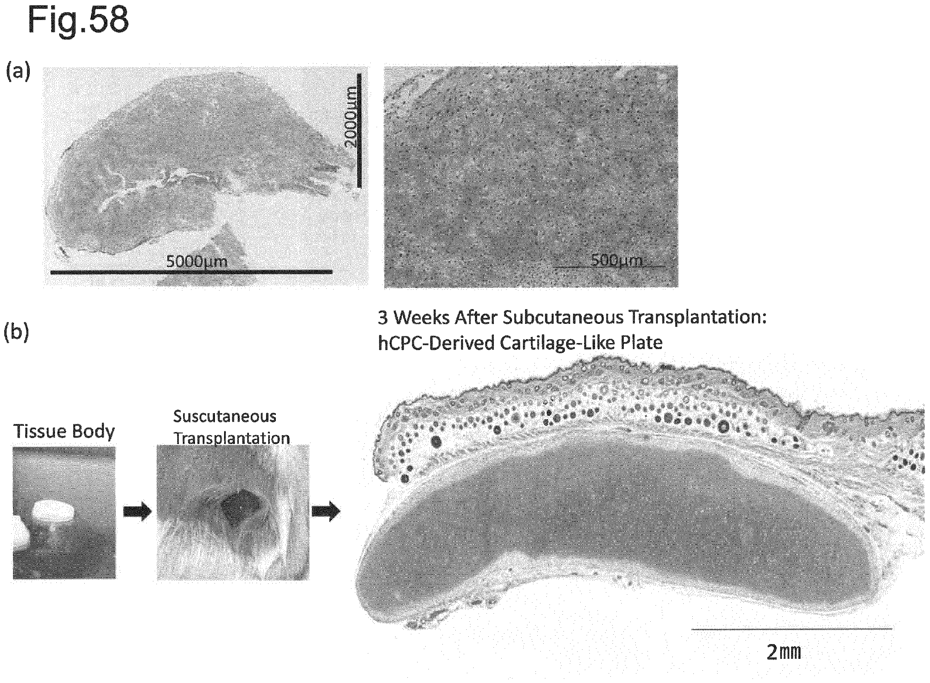

Further, the tissue formed after cartilage differentiation induction in the mold was fixed and subjected to Safranin O staining, and as a result, it was able to be recognized that the tissue body was mostly like hyaline cartilage in terms of morphology, and Safranin O-positive (Fig. 58(a)). Further, the plate-like cartilage tissue was subcutaneously transplanted into a NOD-SCID mouse. After 4 weeks from the transplantation, the tissue body was removed and subjected to Safranin O staining. The result is shown in Fig. 58(b). It was recognized that the generated cartilage plate was subcutaneously engrafted, and the hyaline cartilage tissue body was maintained in the living body without forming a tumor.

### Example 14: Differentiation Induction from Human LBM Cells to hCPCs and Quality Control Method for hCPCs

The human LBM (day 4) cell population obtained in Example 1 was cultured under the following conditions to provide hCPCs.

### • Culture Medium and Conditions for Obtaining hCPCs

### (i) PRRX1-positive Single-peak hCPCs (Fig. 61(a) and Fig. 61(b))

iMatrix511 is used as a coating agent, and human LBM cells are suspended in the following medium and seeded into a culture dish. When the cells are passaged before becoming subconfluent, the hCPCs can be maintained in a PRRX1-positive state.

### • hCPC Medium

**CDM2 basal medium**

| | |
|---|---|
| 1 | µM A-83-01 |
| 3 | µM CHIR99021 |
| 20 | ng/ml FGF2 |
| 20 | ng/ml EGF |
| 10 | µM Y-27632 |

### (ii)PRRX1-positive and PRRX1-negative Double Peak for hCPCs (Fig. 61(a)and Fig. 61(b))

When hCPC cells are cultured in a confluent state for 1 week and passaged, a double-peak cell population of hCPCs is obtained. The double-peak cell population of hCPCs eventually becomes a PRRX1-negative cell population of hCPCs to be described later, and hence the culture thereof cannot be maintained.

### (iii) PRRX1- negative Single-peak hCPCs (Fig. 61(a) and Fig. 61(b))

When the above-mentioned double-peak hCPCs are continuously passaged, a PRRX1-negative hCPC single-peak cell population is obtained.

### (iv) Quality Control of Chondrocyte Progenitor Cells (CPCs) and Limb Bud Mesenchymal Cells (LBMs)

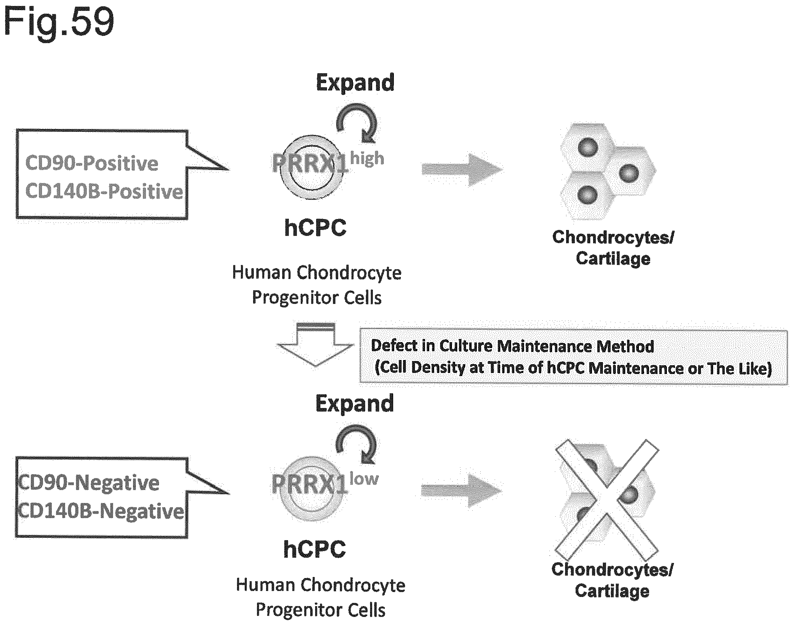

An overview of a quality control technology for CPCs is illustrated in Fig. 59. It was revealed that, in order to stably supply chondrocytes/cartilage tissues from CPCs, it was important to subject hCPCs to expansion culture in a state of satisfying at least one kind of condition selected from the group consisting of CD90 positivity and CD140B positivity.

An overview of a quality control technology for LBMs is illustrated in Fig. 60. It was revealed that, in order to supply CPCs having a high cartilage tissue-forming ability from LBMs, it was important to induce CPCs from "LBMs in a state of satisfying at least one kind of condition selected from the group consisting of CD44 positivity, CD99 positivity, CD140B positivity, CD9 negativity, CD49f negativity, and CD57 negativity, " and in particular, it was important to induce CPCs from "LBMs in a state of satisfying at least one kind of condition selected from the group consisting of CD44 positivity, CD140B positivity, and CD49f negativity. "

### Example 15: Induction from hCPCs Subjected to One-stage Quality Control into Cartilage Tissue

Further, a cartilage tissue was induced from each type of hCPCs under the following conditions and subjected to HE staining, Alcian Blue staining, and Safranin O staining. The results are shown in Fig. 61(c).

### • HE Staining Conditions

After deparaffinization, staining was performed with Eosin for 1 minute, and then the specimen was treated with hematoxylin for 20 minutes. Thus, nuclear staining was performed.

### • Alcian Blue Staining Conditions

After deparaffinization, the specimen was treated with 1% Alcian Blue/3% acetic acid for 20 minutes, and then washed with 3% acetic acid, followed by treatment of the specimen with hematoxylin for 20 minutes. Thus, nuclear staining was performed.

### • Safranin O Staining Conditions

After deparaffinization, staining was performed with Weigert's iron hematoxylin solution for 10 minutes. Next, staining was performed with a fast green solution for 5 minutes, followed by washing with 1% acetic acid. Next, staining was performed with a 0.1% Safranin O solution for 5 minutes.

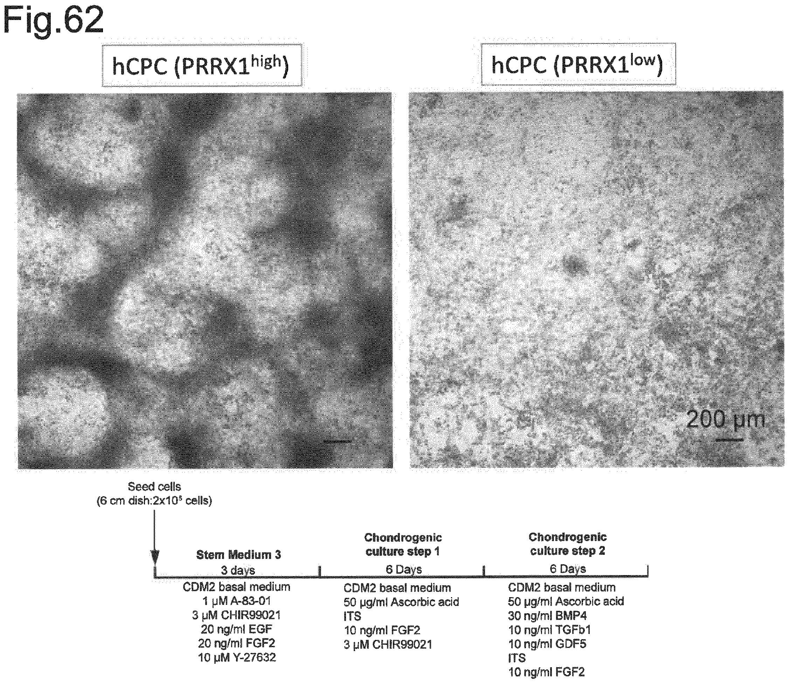

Further, for the PRRX1-positive single-peak hCPCs and the PRRX1-negative single-peak hCPCs, nodule formation through cartilage differentiation induction was promoted, and Alcian Blue staining was performed. The results are shown in Fig. 62.

Further, the obtained hCPCs (PRRX1-positive single peak, PRRX1-negative single peak, and PRRX1-positive and PRRX1-negative double peak) were analyzed using RNA sequences, and mRNAs highly expressed in PRRX1-positive and PRRX1-negative cells were selected. The results are shown in Fig. 63 to Fig. 66.

Further, for human iPS cells (414C2), hCPCs (PRRX1-negative), and hCPCs (PRRX1-positive), the expressions of CD antigens were evaluated using antibodies against the respective CD antigens. The results are shown in Fig. 67.

### Example 16: Quality Control Method for Human LBMs

Human iPS cells are seeded at a density of 3×10⁴ cells/3.5 cm dish, and after 3 days (Pluripotent 3 days) or after 7 days (Pluripotent 7 days) from the seeding, the treatment for obtaining LBM cells (described in Example 1) is performed.

When the treatment is performed from 3 days after the seeding, LBMs having a high expression of PRRX1 (PRRX1-positive, pluripotent 3 days) are obtained, whereas when the treatment is performed from 7 days after the seeding, LBMs having a low expression of PRRX1 (PRRX1-negative, pluripotent 7 days) are obtained (Fig. 68(a) to Fig. 68(c)). That is, it is suggested that the state of human pluripotent stem cells at the start of induction is important in obtaining LBMs (PRRX1-positive).

In the first passage [hCPC (pluripotent 7 days, PN1)] for inducing hCPCs from LBMs (PRRX1-negative, pluripotent 7 days), a population having a low expression of PRRX1 is obtained, and when the passage number is increased (PN1→PN2→PN3→PN4→PN5), at the stage of PN2, a nonuniform cell population including a population having a low expression of PRRX1 and a population having a high expression thereof is obtained [hCPCs, (pluripotent 7 days, PN2)], and at the final stage of PN5, a uniform population having a high expression of PRRX1 is obtained [hCPCs, (pluripotent 7 days, PN5)] (Fig. 68(b)).

When the nonuniform cell population including a population having a low expression of PRRX1 and a population having a high expression thereof [hCPCs (pluripotent 7 days, PN2)], and the uniform cell population having a high expression of PRRX1 [hCPCs (pluripotent 7 days, PN5)] were each subjected to cartilage differentiation induction, a cartilage tissue that was uniformly stained with Alcian blue/Safranin O was formed from the hCPCs (pluripotent 7 days, PN5), but a nonuniform cartilage tissue that was sparsely stained with Alcian blue/Safranin O was formed from the hCPCs (pluripotent 7 days, PN2) (Fig. 68(c)).

Further, for human iPS cells (Negative control), LBMs (PRRX1-negative, 7 days), and hCPCs (PRRX1-positive, 3 days), the expressions of CD antigens were evaluated using antibodies against the respective CD antigens. The results are shown in Fig. 69.

### Example 17: Type II Collagenopathy Disease Model 1

hCPCs were generated from each of type II collagenopathy-related disease patient-derived iPSC lines (ACGII and HCG) (Fig. 70(a)). For the hCPCs from each line, immunostaining for the expression of PRRX1 (Fig. 70(b)) and growth curves (Fig. 70(c)) were examined. The hCPCs from each line were subjected to cartilage differentiation induction "under plane culture" through the above-mentioned treatment process, followed by cartilage matrix staining by Alcian blue staining, or the measurement (qPCR) of the expressions of various cartilage differentiation marker genes. The results found that, as compared to the healthy individual-derived hCPCs (414C2), the PRRX1 reporter iPSC line-derived hCPCs (Reporter), and the ES cell-derived hCPCs (SEES4, SEES5, and SEES7), the type II collagenopathy-related disease patient-derived hCPCs (ACG-II-1 and HCG-1) were reduced in ability to form an Alcian blue staining-positive cartilage matrix (Fig. 70(d)), and were reduced in expressions of various cartilage differentiation marker genes (Fig. 70(e)). The type II collagenopathy-related disease patient-derived cells are reduced in chondrocyte differentiation ability as compared to the healthy individual-derived cells. Through use of hCPCs, patient pathology can be reproduced. Through use of this pathology model, drugs effective for a wide range of cartilage-related diseases not limited to type II collagenopathy can be searched for.

### Example 18: Type II Collagenopathy Disease Model 2

hCPCs were generated from the healthy individual-derived iPSC line (414C2) or the type II collagenopathy-related disease patient-derived iPSC line (ACGII-1 or HCG-1). The cells were collected from their culture dish, and seeded at 100,000 cells/well into an ultra-low U-bottom 96-well plate, followed by a centrifugal operation to generate aggregates on the wells. After that, the cells were treated by the protocol described above, and the finally obtained aggregates were subjected to electron microscopy. As a result, in the type II collagenopathy-related disease patient-derived cells, reductions in glycogen amount in the chondrocytes were observed as compared to the healthy individual-derived cells. In addition, it was found that, in the patient-derived cells, normal aligned arrangement of ER found in normal cells was not found (Fig. 71). In the type II collagenopathy-related disease patient-derived cells, reductions in glycogen amount in the chondrocytes are observed as compared to the healthy individual-derived cells. In addition, in the patient-derived cells, normal aligned arrangement of ER found in normal cells is not found. Through use of the hCPCs induced from the patient-derived iPS cells, patient pathology can be reproduced.

### Example 19: Osteogenesis Imperfecta Disease Model

Osteogenesis imperfecta patient-derived iPSC lines (OIO2-1 and OIO8-12) or iPSC lines obtained by returning their COL1A1 mutation to the normal type through genome editing (respective kinds of rescue lines; OIO2-1(res1) and OIO8-12(res1)) were induced into RUNX2-positive osteoblast progenitor cells under plane culture, and then differentiation induction into osteoblasts was performed (Fig. 72), followed by bone matrix staining by alizarin red staining, the measurement of a Ca²⁺ accumulation amount and the ratio thereof to total protein, and the measurement of ALP activity. As a result, it was found that alizarin red stainability and the various measured values were significantly higher in the rescue lines (Fig. 72). The osteogenesis imperfecta patient-derived cells are reduced in osteoblast differentiation ability as compared to the cells obtained by returning the mutation to the normal type. Through use of the protocol for inducing differentiation from PRRX1-positive limb bud mesenchymal cells into RUNX2-positive osteoblast progenitor cells, osteogenesis imperfecta patient pathology can be reproduced in vitro.

### Industrial Applicability

The LBMs, the CPCs, or the osteoblast progenitor cells subjected to quality control according to the present invention, and the chondrocytes or cartilage tissue and osteoblasts or bone tissue induced therefrom can be preferably used for transplantation for treating a bone- or cartilage-related disease (e.g., knee osteoarthritis, meniscus injury, rheumatoid arthritis, or osteoporosis).

## Claims

1. A limb bud mesenchymal cell population, which is derived from mammalian lateral plate mesoderm cells, and is PRRX1 protein-positive.

2. The limb bud mesenchymal cell population according to claim 1, wherein the limb bud mesenchymal cell population satisfies at least one kind of condition selected from the group consisting of CD44 positivity, CD140B positivity, and CD49f negativity.

3. A method of preparing the limb bud mesenchymal cell population of claim 1 or 2, comprising the steps of:
inducing pluripotent stem cells to differentiate into lateral plate mesoderm cells; and
culturing the lateral plate mesoderm cells obtained in the differentiation induction step under Wnt signal-activating and non-FGF signal-activating conditions.

4. A method of preparing an osteoblast progenitor cell population, comprising a step of culturing the limb bud mesenchymal cell population of claim 1 or 2 under a Wnt signal activator-free environment.

5. A method of preparing a mammalian chondrocyte progenitor cell population, comprising a step of culturing the limb bud mesenchymal cell population of claim 1 or 2 under a Wnt signal-activating environment.

6. The method of preparing a mammalian chondrocyte progenitor cell population according to claim 5, wherein the method comprises a step of culturing the limb bud mesenchymal cell population of claim 1 or 2 under a Wnt signal-activating environment and under an FGF signal-activating condition.

7. A quality control method for a mammalian limb bud mesenchymal cell population, comprising a step of determining whether mammalian limb bud mesenchymal cells satisfy at least one kind of condition selected from the group consisting of CD44 positivity, CD140B positivity, and CD49f negativity.

8. A quality control method for a mammalian chondrocyte progenitor cell population, comprising a step of determining whether mammalian chondrocyte progenitor cells satisfy at least one kind of condition selected from the group consisting of CD90 positivity and CD140B positivity.

9. A mammalian chondrocyte progenitor cell population, which satisfies at least one kind of condition selected from the group consisting of CD90 positivity and CD140B positivity.

10. The mammalian chondrocyte progenitor cell population according to claim 9, wherein the mammalian chondrocyte progenitor cell population is cryopreserved.

11. A mammalian osteoblast progenitor cell population, which has a RUNX2-positive rate of 95% or more.

12. The mammalian osteoblast progenitor cell population according to claim 11, wherein the mammalian osteoblast progenitor cell population is cryopreserved.

13. A kit for inducing mammalian pluripotent stem cells into limb bud mesenchymal cells (LBMs), comprising the following items (i) to (iii):
(i) a medium for inducing pluripotent stem cells into primitive streak cells;
(ii) a medium for inducing primitive streak cells into lateral plate mesoderm cells; and
(iii) a medium for inducing lateral plate mesoderm cells into limb bud mesenchymal cells.

14. A kit for inducing mammalian pluripotent stem cells into chondrocyte progenitor cells, comprising the following items (i) to (iv) :
(i) a medium for inducing pluripotent stem cells into primitive streak cells;
(ii) a medium for inducing primitive streak cells into lateral plate mesoderm cells;
(iii) a medium for inducing lateral plate mesoderm cells into limb bud mesenchymal cells; and
(iv) a medium for inducing limb bud mesenchymal cells into chondrocyte progenitor cells.

15. A kit for inducing mammalian pluripotent stem cells into chondrocytes, comprising the following items (i) to (v):
(i) a medium for inducing pluripotent stem cells into primitive streak cells;
(ii) a medium for inducing primitive streak cells into lateral plate mesoderm cells;
(iii) a medium for inducing lateral plate mesoderm cells into limb bud mesenchymal cells;
(iv) a medium for inducing limb bud mesenchymal cells into chondrocyte progenitor cells; and
(v) a medium for inducing chondrocyte progenitor cells into chondrocytes.

16. A kit for inducing mammalian pluripotent stem cells into RUNX2-positive osteoblast progenitor cells, comprising the following items (i) to (iv):
(i) a medium for inducing pluripotent stem cells into primitive streak cells;
(ii) a medium for inducing primitive streak cells into lateral plate mesoderm cells;
(iii) a medium for inducing lateral plate mesoderm cells into limb bud mesenchymal cells; and
(iv) a medium for inducing limb bud mesenchymal cells into RUNX2-positive osteoblast progenitor cells.

17. A transplantation material, comprising:
the mammalian chondrocyte progenitor cell population of claim 9 or 10; or
chondrocytes or a cartilage tissue obtained by differentiation induction from the chondrocyte progenitor cell population.

18. A transplantation material, comprising:
the mammalian osteoblast progenitor cell population of claim 11 or 12; or
osteoblasts or a bone tissue obtained by differentiation induction from the osteoblast progenitor cell population.

19. A cartilage-related disease model, comprising:
a chondrocyte progenitor cell population, which is induced from cartilage-related disease patient-derived iPS cells, and satisfies at least one kind of condition selected from the group consisting of CD90 positivity and CD140B positivity; or
chondrocytes induced from the chondrocyte progenitor cell population.

20. A bone-related disease model, comprising:
an osteoblast progenitor cell population, which is induced from bone-related disease patient-derived iPS cells, and has a RUNX2-positive rate of 95% or more; or
osteoblasts induced from the osteoblast progenitor cell population.
